# EUROPEAN PATENT SPECIFICATION

(11) **EP 1 663 974 B1**
(45) Date of publication and mention of the grant of the patent: **14.01.2009**
(21) Application number: 04775439.5
(22) Date of filing: 15.09.2004
(51) Int. Cl.: C07D 211/86, C07D 241/08, A61K 31/4412, A61P 29/00

(54) **2-PYRIDONE DERIVATIVES AS NETROPHIL ELASTASE INHIBITORS AND THEIR USE**
2-PYRIDONDERIVATE ALS INHIBITOREN VON NEUTROPHILELASTASE UND DEREN VERWENDUNG
DERIVES 2-PYRIDONE UTILISES COMME INHIBITEURS D'ELASTASE NEUTROPHILE ET UTILISATION DE CEUX-CI

(30) Priority: 18.09.2003 SE 0302487
(43) Date of publication of application: 07.06.2006
(73) Proprietor: AstraZeneca AB, 151 85 Södertälje (SE)
(72) Inventor: HANSEN, Peter, S-221 87 Lund (SE); LAWITZ, Karolina, S-221 87 Lund (SE); LÖNN, Hans, S-221 87 Lund (SE); NIKITIDIS, Antonios, S-221 87 Lund (SE)
(86) International application number: PCT/SE2004/001336
(87) International publication number: WO 2005/026124

(56) References cited:
- WO-A1-02/053543
- GB-A- 2 383 326
- US-A- 5 521 179

## Description

### Field of the Invention

This invention relates to novel 2-pyridone derivatives, processes for their preparation, pharmaceutical compositions comprising them, and their use in therapy.

### Background of the Invention

Elastases are possibly the most destructive enzymes in the body, having the ability to degrade virtually all connective tissue components. The uncontrolled proteolytic degradation by elastases has been implicated in a number of pathological conditions. Human neutrophil elastase (hNE), a member of the chymotrypsin superfamily of serine proteases is a 33-KDa enzyme stored in the azurophilic granules of the neutrophils. In neutrophils the concentration of NE exceeded 5 mM and its total cellular amount has been estimated to be up to 3 pg. Upon activation, NE is rapidly released from the granules into the extracellular space with some portion remaining bound to neutrophil plasma membrane (See Kawabat et al. 2002, Eur. J. Pharmacol. 451, 1-10). The main intracellular physiological function of NE is degradation of foreign organic molecules phagocytosed by neutrophils, whereas the main target for extracellular elastase is elastin (Janoff and Scherer, 1968, J. Exp. Med. 128, 1137-1155). NE is unique, as compared to other proteases (for example, proteinase 3) in that it has the ability to degrade almost all extracellular matrix and key plasma proteins (See Kawabat et al., 2002, Eur. J. Pharmacol. 451, 1-10). It degrades a wide range of extracellular matrix proteins such as elastin, Type 3 and type 4 collagens, laminin, fibronectin, cytokines, etc. (Ohbayashi, H., 2002, Expert Opin. Investig. Drugs, 11, 965-980). NE is a major common mediator of many pathological changes seen in chronic lung disease including epithelial damage (Stockley, R.A. 1994, Am. J. Resp. Crit. Care Med. 150, 109-113).

The destructive role of NE was solidified almost 40 years ago when Laurell and Eriksson reported an association of chronic airflow obstruction and emphysema with deficiency of serum α₁-antitrypsin (Laurell and Eriksson, 1963, Scand. J. Clin. Invest. 15, 132-140). Subsequently it was determined that α₁-antitrypsin is the most important endogenous inhibitor of human NE. The imbalance between human NE and endogenous antiprotease is believed to cause excess human NE in pulmonary tissues which is considered as a major pathogenic factor in chronic obstructive pulmonary disease (COPD). The excessive human NE shows a prominent destructive profile and actively takes part in destroying the normal pulmonary structures, followed by the irreversible enlargement of the respiratory airspaces, as seen mainly in emphysema. There is an increase in neutrophil recruitment into the lungs which is associated with increased lung elastase burden and emphysema in α₁-proteinase inhibitor-deficient mice (Cavarra et al., 1996, Lab. Invest. 75, 273-280). Individuals with higher levels of the NE-α₁ protease inhibitor complex in bronchoalveolar lavage fluid show significantly accelerated decline in lung functions compared to those with lower levels (Betsuyaku et al. 2000, Respiration, 67, 261-267). Instillation of human NE via the trachea in rats causes lung haemorrhage, neutrophil accumulation during acute phase and emphysematous changes during chronic phase (Karaki et al., 2002, Am. J. Resp. Crit. Care Med., 166, 496-500). Studies have shown that the acute phase of pulmonary emphysema and pulmonary haemorrhage caused by NE in hamsters can be inhibited by pre-treatment with inhibitors of NE ( Fujie et al.,1999, Inflamm. Res. 48, 160-167).

Neutrophil-predominant airway inflammation and mucus obstruction of the airways are major pathologic features of COPD, including cystic fibrosis and chronic bronchitis. NE impairs mucin production, leading to mucus obstruction of the airways. NE is reported to increase the expression of major respiratory mucin gene, MUC5AC (Fischer, B.M & Voynow, 2002, Am. J. Respir. Cell Biol., 26, 447-452). Aerosol administration of NE to guinea pigs produces extensive epithelial damage within 20 minutes of contact (Suzuki et al., 1996, Am. J. Resp. Crit. Care Med., 153, 1405-1411). Furthermore NE reduces the ciliary beat frequency of human respiratory epithelium *in vitro* (Smallman et al., 1984, Thorax, 39, 663-667) which is consistent with the reduced mucociliary clearance that is seen in COPD patients (Currie et al., 1984, Thorax, 42, 126-130). The instillation of NE into the airways leads to mucus gland hyperplasia in hamsters (Lucey et al., 1985, Am. Resp. Crit. Care Med., 132, 362-366). A role for NE is also implicated in mucus hypersecretion in asthma. In an allergen sensitised guinea pig acute asthma model an inhibitor of NE prevented goblet cell degranulation and mucus hypersecretion (Nadel et al., 1999, Eur. Resp. J., 13, 190-196).

NE has been also shown to play a role in the pathogenesis of pulmonary fibrosis. NE: α₁-protenase inhibitor complex is increased in serum of patients with pulmonary fibrosis, which correlates with the clinical parameters in these patients (Yamanouchi et al., 1998, Eur. Resp. J. 11, 120-125). In a murine model of human pulmonary fibrosis, a NE inhibitor reduced bleomycin-induced pulmonary fibrosis (Taooka et al., 1997, Am. J. Resp. Crit. Care Med., 156, 260-265). Furthermore investigators have shown that NE deficient mice are resistant to bleomycin-induced pulmonary fibrosis (Dunsmore et al., 2001, Chest, 120, 35S-36S). Plasma NE level was found to be elevated in patients who progressed to ARDS implicating the importance of NE in early ARDS disease pathogenesis. (Donnelly et al., 1995, Am. J. Res. Crit. Care Med., 151, 428-1433). The antiproteases and NE complexed with antiprotease are increased in lung cancer area (Marchandise et al., 1989, Eur. Resp. J. 2, 623-629). Recent studies have shown that polymorphism in the promoter region of the NE gene are associated with lung cancer development (Taniguchi et al., 2002, Clin. Cancer Res., 8, 1115-1120.

Acute lung injury caused by endotoxin in experimental animals is associated with elevated levels of NE (Kawabata, et al., 1999, Am. J. Resp. Crit. Care, 161, 2013-2018). Acute lung inflammation caused by intratracheal injection of lipopolysaccharide in mice has been shown to elevate the NE activity in bronchoalveolar lavage fluid which is significantly inhibited by a NE inhibitor (Fujie et al., 1999, Eur. J. Pharmacol., 374, 117-125; Yasui, et al., 1995, Eur. Resp. J., 8, 1293-1299). NE also plays an important role in the neutrophil-induced increase of pulmonary microvascular permeability observed in a model of acute lung injury caused by tumour necrosis factor α (TNFα) and phorbol myristate acetate (PMA) in isolated perfused rabbit lungs (Miyazaki et al., 1998, Am. J. Respir. Crit. Care Med., 157, 89-94).

A role for NE has also been suggested in monocrotoline-induced pulmonary vascular wall thickening and cardiac hypertrophy (Molteni et al., 1989, Biochemical Pharmacol. 38, 2411-2419). Serine elastase inhibitor reverses the monocrotaline-induced pulmonary hypertension and remodelling in rat pulmonary arteries (Cowan et al., 2000, Nature Medicine, 6, 698-702). Recent studies have shown that serine elastase, that is, NE or vascular elastase are important in cigarette smoke-induced muscularisation of small pulmonary arteries in guinea pigs (Wright et al., 2002, Am. J. Respir. Crit. Care Med., 166, 954-960).

NE plays a key role in experimental cerebral ischemic damage (Shimakura et al., 2000, Brain Research, 858, 55-60), ischemia-reperfusion lung injury (Kishima et al., 1998, Ann. Thorac. Surg. 65, 913-918) and myocardial ischemia in rat heart (Tiefenbacher et al., 1997, Eur. J. Physiol., 433, 563-570). Human NE levels in plasma are significantly increased above normal in inflammatory bowel diseases, for example, Crohn's disease and ulcerative colitis (Adeyemi et al., 1985, Gut, 26, 1306-1311). In addition NE has also been assumed to be involved in the pathogenesis of rheumatoid arthritis (Adeyemi et al., 1986, Rheumatol. Int., 6, 57). The development of collagen induced arthritis in mice is suppressed by a NE inhibitor (Kakimoto et al., 1995, Cellular Immunol. 165, 26-32).

Thus, human NE is known as one of the most destructive serine proteases and has been implicated in a variety of inflammatory diseases. The important endogenous inhibitor of human NE is α₁-antitrypsin. The imbalance between human NE and antiprotease is believed to give rise to an excess of human NE resulting in uncontrolled tissue destruction. The protease/ antiprotease balance may be upset by a decreased availability of α₁-antitrypsin either through inactivation by oxidants such as cigarette smoke, or as a result of genetic inability to produce sufficient serum levels. Human NE has been implicated in the promotion or exacerbation of a number of diseases such as pulmonary emphysema, pulmonary fibrosis, adult respiratory distress syndrome (ARDS), ischemia reperfusion injury, rheumatoid arthritis and pulmonary hypertension.

WO 02/053543 discloses pyridone derivatives having affinity for cannabinoid 2-type receptor. US 5,521,179 discloses 1-pyridylacetamide derivatives which are inhibitors of human neutrophil elastase. GB 2 383 326 discloses novel 1,4-dihydro-1,4-diphenylpyridine derivatives useful for the treatment of acute and chronic inflammatory processes.

The present invention discloses novel 2-pyridione derivatives that are inhibitors of human neutrophil elastase and homologous serine proteases such as proteinase 3 and pancreatic elastase, and are thereby useful in therapy.

### Disclosure of the Invention

The present invention provides a compound of formula (I) 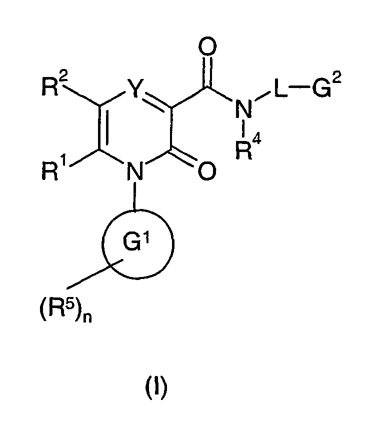 wherein:
**Y** represents CR³or N;
**R¹** represents H or C1 to 6 alkyl;
**R²** represents:
   i) CN, NO₂, OH, OSO₂R⁴⁷, O-C2 to 6 alkanoyl, CO₂R⁴⁷, CHO or C2 to 6 alkanoyl; or
   ii) C1 to 6 alkoxy optionally substituted by OH, C1 to 6 alkoxy, CN, NR⁵⁴R⁵⁵, CONR⁵⁴R⁵⁵, OCOR⁴⁷ or one or more F atoms; or
   iii) C3 to 6 saturated or partially unsaturated cycloalkyl optionally further substituted by C1 to 6 alkyl; or
   iv) C4 to 7 saturated or partially unsaturated heterocyclic ring containing 1 to 3 heteroatoms independently selected from O, S(O)ₘ and NR⁶² optionally further substituted by C1 to 6 alkyl; or
   v) CONR⁴⁸R⁴⁹, CONR⁵⁰NR⁴⁸R⁴⁹, C(=NOR⁵²)R⁵³, C(=NH)NHOR⁵² or NR⁴⁸R⁴⁹; or
   vi) C2 to 6 alkenyl or C2 to 6 alkynyl; said alkenyl or alkynyl group being optionally further substituted by C1 to 6 alkoxy or phenyl or a five- or six-membered heteroaromatic ring containing 1 to 3 heteroatoms independently selected from O, S and N; said phenyl or heteroaromatic ring being optionally further substituted by halogen, CN, C1 to 6 alkyl or C1 to 6 alkoxy; or
   vii) C1 to 6 alkyl substituted by one or more F atoms; or
   viii) C1 to 6 alkyl substituted by one or more groups selected from halogen, OH, oxo, azido, NR⁴⁸R⁴⁹, C1 to 6 alkoxy and C1 to 6 alkoxy substituted by one or more F atoms; or
   ix) C1 to 6 alkyl substituted by phenyl or a five- or six-membered heteroaromatic ring containing 1 to 3 heteroatoms independently selected from O, S and N; said phenyl or heteroaromatic ring being optionally further substituted by halogen, CN, C1 to 6 alkyl or C1 to 6 alkoxy;
**R⁴⁸** and **R⁴⁹** independently represent H, OH, C1 to 6 alkoxy, C3 to 6 cycloalkyl, CHO, C2 to 6 alkanoyl, CO₂R⁵⁰, C(X)NR⁶³R⁶⁴ or C1 to 6 alkyl; said alkyl being optionally further substituted by OH, C1 to 4 alkoxy, C3 to 6 cycloalkyl, CN or phenyl or a five- or six-membered heteroaromatic ring containing 1 to 3 heteroatoms independently selected from O, S and N; said alkanoyl being optionally further substituted by CN;
**X** represent O or S;
or the group **NR⁴⁸R⁴⁹** together represents a saturated or partially unsaturated 5 to 7 membered azacyclic ring optionally incorporating one further heteroatom selected from O, S and NR⁵⁶; said azacyclic ring being optionally further substituted by one or more substituents selected from OR⁵⁷ and C1 to 4 alkyl; said alkyl being optionally further substituted by OR⁵⁷;
**R³** represents H or F;
**G¹** represents phenyl or a five- or six-membered heteroaromatic ring containing 1 to 3 heteroatoms independently selected from O, S and N;
**R⁵** represents H, halogen, C1 to 6 alkyl, CN, C1 to 6 alkoxy, NO₂, NR¹⁴R¹⁵, C1 to 3 alkyl substituted by one or more F atoms or C1 to 3 alkoxy substituted by one or more F atoms;
**R¹⁴** and **R¹⁵** independently represent H or C 1 to 3 alkyl; said alkyl being optionally further substituted by one or more F atoms;
**n** represents an integer 1, 2 or 3 and when n represents 2 or 3, each R⁵ group is selected independently;
**R⁴** represents H or C1 to 6 alkyl; said alkyl being optionally further substituted by OH or C1 to 6 alkoxy;
or **R⁴** and **L** are joined together such that the group **-NR⁴L** represents a 5 to 7 membered azacyclic ring optionally incorporating one further heteroatom selected from O, S and NR¹⁶; said ring being optionally further substituted by C1 to 6 alkyl or NR⁶⁰R⁶¹; said alkyl being optionally further substituted by OH;
**L** represents a bond, O, NR²⁹ or C1 to 6 alkyl; said alkyl optionally incorporating a heteroatom selected from O, S and NR¹⁶; and said alkyl being optionally further substituted by OH or OMe;
**G²** represents a monocyclic ring system selected from:
   i) phenyl or phenoxy,
   ii) a 5 or 6 membered heteroaromatic ring containing one to three heteroatoms independently selected from O, S and N,
   iii) a C3 to 6 saturated or partially unsaturated cycloalkyl, or
   iv) a C4 to 7 saturated or partially unsaturated heterocyclic ring containing one or two heteroatoms independently selected from O, S(O)p and NR¹⁷ and optionally further incorporating a carbonyl group; or
**G²** represents a bicyclic ring system in which each of the two rings is independently selected from:
   i) phenyl,
   ii) a 5 or 6 membered heteroaromatic ring containing one to three heteroatoms independently selected from O, S and N,
   iii) a C3 to 6 saturated or partially unsaturated cycloalkyl, or
   iv) a C4 to 7 saturated or partially unsaturated heterocyclic ring containing one or two heteroatoms independently selected from O, S(O)p and NR¹⁷ and optionally further incorporating a carbonyl group;
   and the two rings are either fused together, or are bonded directly together or are separated by a linker group selected from O, S(O)_{q} or CH₂,
   said monocyclic or bicyclic ring system being optionally further substituted by one to three substituents independently selected from CN, OH, C1 to 6 alkyl, C1 to 6 alkoxy, halogen, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C(=NH)NH₂, S(O)ₛR²⁵, SO₂NR²⁶R²⁷, C1 to 3 alkoxy substituted by one or more F atoms and C1 to 3 alkyl substituted by SO₂R³⁹ or by one or more F atoms; or
   when L does not represent an bond, **G²** may also represent H;
   at each occurrence, **m, p, q, s** and **t** independently represent an integer 0, 1 or 2;
**R¹⁸** and **R¹⁹** independently represent **H**, C1 to 6 alkyl, formyl, C2 to 6 alkanoyl, S(O)ₜR³² or SO₂NR³³R³⁴; said alkyl group being optionally further substituted by halogen, CN, C1 to 4 alkoxy or CONR⁴¹R⁴²;
**R²⁵** represents H, C1 to 6 alkyl or C3 to 6 cycloalkyl; said alkyl group being optionally further substituted by one or more substituents selected independently from OH, CN, CONR³⁵R³⁶, CO₂R³⁷, OCOR⁴⁰, C3 to 6 cycloalkyl, a C4 to 7 saturated heterocyclic ring containing one or two heteroatoms independently selected from O, S(O)p and NR⁴³ and phenyl or a 5 or 6 membered heteroaromatic ring containing one to three heteroatoms independently selected from O, S and N; said aromatic ring being optionally further substituted by one or more substituents selected independently from halogen, CN, C1 to 4 alkyl, C1 to 4 alkoxy, OH, CONR⁴⁴R⁴⁵, CO₂R⁴⁶, S(O)ₛR⁶⁵ and NHCOCH₃;
**R³²** represents H, C1 to 6 alkyl or C3 to 6 cycloalkyl;
**R¹⁶, R¹⁷, R²⁰, R²¹, R²², R²³, R²⁴, R²⁶, ,R²⁷, R²⁹, R³¹, R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁵⁰, R⁵², R⁵³, R⁵⁴, R⁵⁵, R⁵⁶, R⁵⁷, R⁶⁰, R⁶¹, R⁶², R⁶³, R⁶⁴ and R⁶⁵** independently represent H or C1 to 6 alkyl;
and pharmaceutically acceptable salts thereof.

The compounds of formula (I) may exist in enantiomeric and/or tautomeric forms. It is to be understood that all enantiomers, diastereomers, racemates, tautomers and mixtures thereof are included within the scope of the invention.

Unless otherwise indicated, the term "C1 to 6 alkyl" referred to herein denotes a straight or branched chain alkyl group having from 1 to 6 carbon atoms. Examples of such groups include methyl, ethyl, n-propyl, i-propyl, n-butyl, i-butyl, t-butyl, pentyl and hexyl. The terms "C1 to 3 alkyl" and "C1 to 4 alkyl" are to be interpreted analogously.

Examples of "C1 to 3 alkyl substituted by one or more F atoms" include fluoromethyl, difluoromethyl, trifluoromethyl, 2,2,2-trifluoroethyl, 1,1-difluoroethyl, pentafluoroethyl and 3,3,3-trifluoropropyl.

Unless otherwise indicated, the term "C1 to 6 alkoxy " referred to herein denotes an oxygen substituent bonded to a straight or branched chain alkyl group having from 1 to 6 carbon atoms. Examples of such groups include methoxy, ethoxy, n-propoxy, i-propoxy, n-butoxy, i-butoxy and s-butoxy. The terms "C1 to 3 alkoxy" and "C1 to 4 alkoxy" are to be interpreted analogously.

Examples of "C1 to 6 alkoxy substituted by one or more F atoms" include fluoromethoxy, trifluoromethoxy, 2,2,2-trifluoroethoxy and 3,3,3-trifluoropropoxy.

Unless otherwise indicated, the term "C2 to 6 alkanoyl" referred to herein denotes a straight or branched chain alkyl group having from 1 to 5 carbon atoms bonded to the molecule via a carbonyl group. Examples of such groups include acetyl, propionyl and pivaloyl.

Unless otherwise indicated, the term "halogen" referred to herein denotes fluorine, chlorine, bromine and iodine.

Examples of a five or six membered heteroaromatic ring containing 1 to 3 heteroatoms independently selected from O, S and N include furan, thiophene, pyrrole, oxazole, oxadiazole, isoxazole, imidazole, thiazole, triazole, thiadiazole, pyridine, pyrimidine and pyrazine.

Unless otherwise indicated, the term "C3 to 6 saturated or partially unsaturated cycloalkyl" referred to herein denotes a 3 to 6 membered non-aromatic carbocyclic ring optionally incorporating one or more double bonds. Examples include cyclopropyl, cyclopentyl, cyclopentenyl, cyclohexyl and cyclohexenyl. The term "five- or six-membered saturated or partially unsaturated cycloalkyl ring" is to be interpreted analogously.

Unless otherwise indicated, the term "C4 to 7 saturated or partially unsaturated heterocyclic ring containing one or two heteroatoms independently selected from O, S(O)ₚ and NR¹⁷ and optionally further incorporating a carbonyl group" referred to herein denotes a 4 to 7 membered non-aromatic heterocyclic ring optionally incorporating one or more double bonds and optionally incorporating a carbonyl group. Examples include tetrahydrofuran, thiolane 1,1-dioxide, tetrahydropyran, 4-oxo-4H-pyran, pyrrolidine, pyrroline, imidazolidine, 1,3-dioxolane, piperidine, piperazine, morpholine, perhydroazepine, pyrrolidone and piperidone. The terms "five- or six-membered saturated or partially unsaturated heterocyclic ring containing one heteroatom selected from O, S and NR¹³" and "C4 to 7 saturated or partially unsaturated heterocyclic ring containing 1 to 3 heteroatoms independently selected from O, S(O)ₘ and NR⁶²" are to be interpreted analogously.

Examples of a "5 to 7 membered azacyclic ring optionally incorporating one further heteroatom selected from O, S and NR¹⁶" include pyrrolidine, piperidine, morpholine, thiomorpholine and piperazine.

In the definition of L, "C1 to 6 alkyl; said alkyl optionally incorporating a heteroatom selected from O, S and NR¹⁶" embraces a straight or branched chain arrangement of 1 to 6 carbon atoms in which any two carbon atoms are optionally separated by O, S or NR¹⁶. The definition thus includes, for example, methylene, ethylene, propylene, hexamethylene, ethylethylene, -CH₂CH₂O-CH₂-, -CH₂CH₂O-CH₂-CH₂-, -CH₂CH₂S- and -CH₂CH₂NR¹⁶-.

Examples of bicyclic ring systems in which the two rings are either fused together, or are bonded directly together or are separated by a linker group selected from O, S(O)_{q} or CH₂ include biphenyl, thienylphenyl, pyrazolylphenyl, phenoxyphenyl, phenylcyclopropyl, naphthyl, indanyl, quinolyl, tetrahydroquinolyl, benzofuranyl, indolyl, isoindolyl, indolinyl, benzofuranyl, benzothienyl, indazolyl, benzimidazolyl, benzthiazolyl, purinyl, isoquinolyl, chromanyl, indenyl, quinazolyl, quinoxalyl, chromanyl, isocromanyl, 3H-indolyl, 1H-indazolyl, quinuclidyl, tetrahydronaphthyl, dihydrobenzofuranyl, morpholine-4-ylphenyl, 1,3-benzodioxolyl, 1,1-dioxido-2,3-dihydro-1-benzothienyl, 2,3-dihydro-1,4-benzodioxinyl, 1,3-benzodioxinyl and 3,4-dihydro-isochromenyl.

In one embodiment, Y in formula (I) represents CR³. In another embodiment, Y represents N.

In one embodiment, R¹ in formula (I) represents C1 to 6 alkyl. In another embodiment, R¹ represents CH₃.

In one embodiment R² in formula (I) represents CONR⁴⁸R⁴⁹. In another embodiment, R² represents C1 to 6 alkoxy or C1 to 6 alkoxy substituted by OH, C1 to 6 alkoxy, or by one or more F atoms. In yet another embodiment, R² represents C1 to 6 alkyl substituted by one or more F atoms or C1 to 6 alkyl substituted by OH, NR⁴⁸R⁴⁹, C1 to 6 alkoxy or C1 to 6 alkoxy substituted by one or more F atoms.

In one embodiment, R³ in formula (I) represents H.

In one embodiment, G¹ in formula (I) represents phenyl or pyridyl. In another embodiment, G¹ in formula (I) represents phenyl.

In one embodiment, R⁵ in formula (I) represents halogen, C1 to 6 alkyl, CN or C1 to 3 alkyl substituted by one or more F atoms. In another embodiment, R⁵ in formula (I) represents Cl, CH₃, CN or CF₃.

In one embodiment, n represents the integer 1.

In another embodiment, G¹ in formula (I) represents phenyl, R⁵ represents CF₃ and n represents the integer 1.

In one embodiment, R⁴ represents H.

In one embodiment, L represents C1 to 6 alkyl (C1 to 6 alkylidene). In another embodiment, L represents -CH₂-. In another embodiment, L represents NR²⁹ and R²⁹ represents H.

In one embodiment, G² represents an optionally substituted monocyclic ring system selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)p and NR¹⁷ and optionally further incorporating a carbonyl group.

In another embodiment, G² represents optionally substituted phenyl. In another embodiment, G² represents phenyl substituted by OSO₂R³⁸, S(O)ₛR²⁵, SO₂NR²⁶R²⁷, NR¹⁸R¹⁹ (wherein at least one of R¹⁸ and R¹⁹ represents S(O)ₜR³² or SO2NR³³R³⁴) or C1 to 3 alkyl substituted by SO₂R³⁹. In another embodiment, G² represents phenyl substituted by S(O)ₛR²⁵ and R²⁵ represents C1 to 6 alkyl or C3 to 6 cycloalkyl and s represents the integer 2.

In another embodiment, G² represents an optionally substituted bicyclic ring system in which each of the two rings is independently selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)p and NR¹⁷ and optionally further incorporating a carbonyl group;
and the two rings are either fused together, or are bonded directly together or are separated by a linker group selected from O, S(O)_{q} or CH₂.

In one embodiment, Y in formula (I) represents CR³ and R³ represents H; R¹ represents C1 to 6 alkyl; R² represents CONR⁴⁸R⁴⁹ or optionally substituted C1 to 6 alkoxy or substituted C1 to 6 alkyl; G¹ represents phenyl; R⁵ represents halogen, C1 to 6 alkyl, CN or C1 to 3 alkyl substituted by one or more F atoms; R⁴ represents H; L represents C1 to 6 alkyl; and G² represents an optionally substituted monocyclic ring system selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)p and NR¹⁷ and optionally further incorporating a carbonyl group.

In one embodiment, Y in formula (I) represents CR³ and R³ represents H; R¹ represents C1 to 6 alkyl; R² represents CONR⁴⁸R⁴⁹ or optionally substituted C1 to 6 alkoxy or substituted C1 to 6 alkyl; G¹ represents phenyl; R⁵ represents halogen, C1 to 6 alkyl, CN or C1 to 3 alkyl substituted by one or more F atoms; R⁴ represents H; L represents C1 to 6 alkyl; and G² represents phenyl substituted by OSO₂R³⁸, S(O)ₛR²⁵, SO₂NR²⁶R²⁷, NR¹⁸R¹⁹ (wherein at least one of R¹⁸ and R¹⁹ represents S(O)ₜR³² or SO2NR³³R³⁴) or C1 to 3 alkyl substituted by SO₂R³⁹.

In one embodiment, Y in formula (I) represents CR³ and R³ represents H; R¹ represents methyl; R² represents CONR⁴⁸R⁴⁹ or optionally substituted C1 to 6 alkoxy or substituted C1 to 6 alkyl; G¹ represents phenyl; R⁵ represents Cl, CH₃, CN or CF₃; R⁴ represents H; L represents C1 to 6 alkyl; and G² represents phenyl substituted by OSO₂R³⁸, S(O)ₛR²⁵, SO₂NR²⁶R²⁷, NR¹⁸R¹⁹ (wherein at least one of R¹⁸ and R¹⁹ represents S(O)ₜR³² or SO2NR³³R³⁴) or C1 to 3 alkyl substituted by SO₂R³⁹.

In one embodiment, Y in formula (I) represents CR³ and R³ represents H; R¹ represents methyl; R² represents CONR⁴⁸R⁴⁹; G¹ represents phenyl; R⁵ represents Cl, CH₃, CN or CF₃; R⁴ represents H; L represents C1 to 3 alkyl; and G² represents phenyl substituted by S(O)ₛR²⁵ and R²⁵ represents C1 to 6 alkyl or C3 to 6 cycloalkyl and s represents the integer 2.

In one embodiment, Y in formula (I) represents CR³ and R³ represents H; R¹ represents methyl; R² represents optionally substituted C1 to 6 alkoxy; G¹ represents phenyl; R⁵ represents Cl, CH₃, CN or CF₃; R⁴ represents H; L represents C1 to 3 alkyl; and G² represents phenyl substituted by S(O)ₛR²⁵ and R²⁵ represents C1 to 6 alkyl or C3 to 6 cycloalkyl and s represents the integer 2.

In one embodiment, Y in formula (I) represents CR³ and R³ represents H; R¹ represents methyl; R² represents substituted C1 to 6 alkyl; G¹ represents phenyl; R⁵ represents Cl, CH₃, CN or CF₃; R⁴ represents H; L represents C1 to 3 alkyl; and G² represents phenyl substituted by S(O)ₛR²⁵ and R²⁵ represents C1 to 6 alkyl or C3 to 6 cycloalkyl and s represents the integer 2.

In one embodiment, Y in formula (I) represents CR³ or N; R¹ represents H or C1 to 6 alkyl; R² represents CN, NO₂, OH, CO₂R⁴⁷, CHO, C2 to 6 alkanoyl, C1 to 6 alkoxy, C1 to 6 alkoxy substituted by one or more F atoms, C3 to 6 saturated or partially unsaturated cycloalkyl, C4 to 7 saturated or partially unsaturated heterocyclic ring containing 1 to 3 heteroatoms independently selected from O, S(O)ₘ and NR⁶², CONR⁴⁸R⁴⁹, CONR⁵⁰NHR⁵¹, C(=NOR⁵²)R⁵³, NR⁵⁸R⁵⁹, C2 to 6 alkenyl or C2 to 6 alkynyl; said alkenyl or alkynyl group being optionally further substituted by C1 to 6 alkoxy or phenyl or a five- or six-membered heteroaromatic ring containing 1 to 3 heteroatoms independently selected from O, S and N; said phenyl or heteroaromatic ring being optionally further substituted by halogen, CN, C1 to 6 alkyl or C1 to 6 alkoxy; or R² represents C1 to 6 alkyl substituted by one or more F atoms; or C1 to 6 alkyl substituted by OH, NR⁵⁸R⁵⁹, C1 to 6 alkoxy or C1 to 6 alkoxy substituted by one or more F atoms; or C1 to 6 alkyl substituted by phenyl or a five- or six-membered heteroaromatic ring containing 1 to 3 heteroatoms independently selected from O, S and N; said phenyl or heteroaromatic ring being optionally further substituted by halogen, CN, C1 to 6 alkyl or C1 to 6 alkoxy; R⁴⁸ represents H, OH, C1 to 6 alkoxy or C1 to 6 alkyl; said alkyl being optionally further substituted by OH, C1 to 4 alkoxy or NR⁵⁴R⁵⁵; R⁴⁹ represents H or C1 to 6 alkyl; said alkyl being optionally further substituted by OH, C1 to 4 alkoxy or NR⁵⁴R⁵⁵; or the group NR⁴⁸R⁴⁹ together represents a 5 to 7 membered azacyclic ring optionally incorporating one further heteroatom selected from O, S and NR⁵⁶; said azacyclic ring being optionally further substituted by OR⁵⁷ or C1 to 4 alkyl; said alkyl being optionally further substituted by OR⁵⁷; R⁵⁸ and R⁵⁹ independently represent H, C1 to 6 alkyl, NR⁶⁰R⁶¹ or CONR⁶³R⁶⁴; or the group NR⁵⁸R⁵⁹ together represents a 5 to 7 membered azacyclic ring optionally incorporating one further heteroatom selected from O, S and NR⁵⁶; said azacyclic ring being optionally further substituted by OR⁵⁷ or C1 to 4 alkyl; said alkyl being optionally further substituted by OR⁵⁷; R⁵¹ represents C2 to 4 alkanoyl optionally further substituted by CN, or R⁵¹ represents C(X)NH₂ wherein X represents O or S; R³ represents H or F; G¹ represents phenyl or a five- or six-membered heteroaromatic ring containing 1 to 3 heteroatoms independently selected from O, S and N; R⁵ represents H, halogen, C1 to 6 alkyl, CN, C1 to 6 alkoxy, NO₂, NR¹⁴R¹⁵, C1 to 3 alkyl substituted by one or more F atoms or C1 to 3 alkoxy substituted by one or more F atoms; R¹⁴ and R¹⁵ independently represent H or C1 to 3 alkyl; said alkyl being optionally further substituted by one or more F atoms; n represents an integer 1, 2 or 3 and when n represents 2 or 3, each R⁵ group is selected independently; R⁴ represents H or C1 to 6 alkyl; said alkyl being optionally further substituted by OH or C1 to 6 alkoxy; or R⁴ and L are joined together such that the group -NR⁴L represents a 5 to 7 membered azacyclic ring optionally incorporating one further heteroatom selected from O, S and NR¹⁶; L represents a bond, O, NR²⁹ or C1 to 6 alkyl; said alkyl optionally incorporating a heteroatom selected from O, S and NR¹⁶; and said alkyl being optionally further substituted by OH or OMe; G² represents a monocyclic ring system selected from:
i) phenyl or phenoxy,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)p and NR¹⁷ and optionally further incorporating a carbonyl group; or
G² represents a bicyclic ring system in which each of the two rings is independently selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)p and NR¹⁷ and optionally further incorporating a carbonyl group;
and the two rings are either fused together, or are bonded directly together or are separated by a linker group selected from O, S(O)_{q} or CH₂; said monocyclic or bicyclic ring system being optionally further substituted by one to three substituents independently selected from CN, OH, C1 to 6 alkyl, C1 to 6 alkoxy, halogen, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C(=NH)NH₂, S(O)ₛR²⁵, SO₂NR²⁶R²⁷, C1 to 3 alkoxy substituted by one or more F atoms and C1 to 3 alkyl substituted by SO₂R³⁹ or by one or more F atoms; or when L does not represent an bond, G² may also represent H; m, p, q, s and t independently represent an integer 0, 1 or 2; R¹⁸ and R¹⁹ independently represent H, C1 to 6 alkyl, formyl, C2 to 6 alkanoyl, S(O)ₜR³² or SO₂NR³³R³⁴; said alkyl group being optionally further substituted by halogen, CN, C1 to 4 alkoxy or CONR⁴¹R⁴²; R²⁵ represents H, C1 to 6 alkyl or C3 to 6 cycloalkyl; said alkyl group being optionally further substituted by one or more substituents selected independently from OH, CN, CONR³⁵R³⁶, CO₂R³⁷, OCOR⁴⁰, C3 to 6 cycloalkyl, a C4 to 7 saturated heterocyclic ring containing one or two heteroatoms independently selected from O, S(O)p and NR⁴³ and phenyl or a 5 or 6 membered heteroaromatic ring containing one to three heteroatoms independently selected from O, S and N; said aromatic ring being optionally further substituted by one or more substituents selected independently from halogen, CN, C1 to 4 alkyl, C1 to 4 alkoxy, OH, CONR⁴⁴R⁴⁵, CO₂R⁴⁶, S(O)ₛR⁶⁵ and NHCOCH₃; R³² represents H, C1 to 6 alkyl or C3 to 6 cycloalkyl; and R¹⁶, R¹⁷, R²⁰, R²¹, R²², R²³, R²⁴, R²⁶, R²⁷, R²⁹, R³¹, R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁵⁰, R⁵², R⁵³, R⁵⁴, R⁵⁵, R⁵⁶, R⁵⁷, R⁶⁰, R⁶¹, R⁶², R⁶³, R⁶⁴ and R⁶⁵ independently represent H or C1 to 6 alkyl;

In another aspect, the invention specifically provides any compound as described in the Examples herein, or the free base thereof or a pharmaceutically acceptable salt thereof. Particular compounds include:
5-cyano-6-methyl-N-[4-(methylsulfonyl)benzyl]-2-oxo-1-[3-(trifluoromethyl)phenyl]-1,2-dihydropyridine-3-carboxamide;
6-methyl-N-[4-(methylsulfonyl)benzyl]-5-nitro-2-oxo-1-[3-(trifluoromethyl)phenyl]-1,2-dihydropyridine-3-carboxamide;
5-(1-butoxyvinyl)-6-methyl-N-[4-(methylsulfonyl)benzyl]-2-oxo-1-[3-(trifluoromethyl)phenyl]-1,2-dihydropyridine-3-carboxamide;
5-acetyl-6-methyl-N-[4-(methylsulfonyl)benzyl]-2-oxo-1-[3-(trifluoromethyl)phenyl]-1,2-dihydropyridine-3-carboxamide;
5-[(1E)-N-methoxyethanimidoyl]-6-methyl-N-[4-(methylsulfonyl)benzyl]-2-oxo-1-[3-(trifluoromethyl)phenyl]-1,2-dihydropyridine-3-carboxamide;
5-[(1E)-N-hydroxyethanimidoyl]-6-methyl-N-[4-(methylsulfonyl)benzyl]-2-oxo-1-[3-(trifluoromethyl)phenyl]-1,2-dihydropyridine-3-carboxamide;
6-methyl-N-[4-(methylsulfonyl)benzyl]-2-oxo-5-(pyridin-3-ylethynyl)-1-[3-(trifluoromethyl)phenyl]-1,2-dihydropyridine-3-carboxamide;
6-methyl-N-[4-(methylsulfonyl)benzyl]-2-oxo-5-(2-pyridin-3-ylethyl)-1-[3-(trifluoromethyl)phenyl]-1,2-dihydropyridine-3-carboxamide;
6-methyl-N-[4-(methylsulfonyl)benzyl]-2-oxo-1-[3-(trifluoromethyl)phenyl]-5-vinyl-1,2-dihydropyridine-3-carboxamide;
ethyl 2-methyl-5-({[4-(methylsulfonyl)benzyl]amino}carbonyl)-6-oxo-1-[3-(trifluoromethyl)phenyl]-1,6-dihydropyridine-3-carboxylate;
5-(4-methanesulfonyl-benzylcarbamoyl)-2-methyl-6-oxo-1-(3-trifluoromethyl-phenyl)-1,6-dihydro-pyridine-3-carboxylic acid;
6-methyl-2-oxo-1-(3-trifluoromethyl-phenyl)-1,2-dihydro-pyridine-3,5-dicarboxylic acid 5-dimethylamide 3-(4-methanesulfonyl-benzylamide);
6-methyl-2-oxo-1-(3-trifluoromethyl-phenyl)-1,2-dihydro-pyridine-3,5-dicarboxylic acid 5-amide 3-(4-methanesulfonyl-benzylamide);
6-methyl-2-oxo-1-(3-trifluoromethyl-phenyl)-1,2-dihydro-pyridine-3,5-dicarboxylic acid 3-(4-methanesulfonyl-benzylamide) 5-methylamide;
6-methyl-2-oxo-1-(3-trifluoromethyl-phenyl)-1,2-dihydro-pyridine-3,5-dicarboxylic acid 5-[(2-hydroxy-ethyl)-methyl-amide]3-(4-methanesulfonyl-benzylamide);
6-methyl-2-oxo-1-(3-trifluoromethyl-phenyl)-1,2-dihydro-pyridine-3,5-dicarboxylic acid 3-(4-methanesulfonyl-benzylamide) 5-(methyl-propyl-amide);
6-methyl-2-oxo-5-(pyrrolidine-1-carbonyl)-1-(3-trifluoromethyl-phenyl)-1,2-dihydropyridine-3,5-dicarboxylic acid 3-(4-methanesulfonyl-benzylamide);
6-methyl-2-oxo-1-(3-trifluoromethyl-phenyl)-1,2-dihydro-pyridine-3,5-dicarboxylic acid 5-[(2-dimethylamino-ethyl)-methyl-amide] 3-(4-methanesulfonyl-benzylamide);
5-((2*R*)-2-hydroxymethyl-pyrrolidine-1-carbonyl)-6-methyl-2-oxo-1-(3-trifluoromethyl - phenyl)-1,2-dihydro-pyridine-3-carboxylic acid 3-(4-methanesulfonyl -benzylamide);
5-(3-hydroxy-pyrrolidine-1-carbonyl)-6-methyl-2-oxo-1-(3-trifluoromethyl-phenyl)-1,2-dihydro-pyridine-3,5-dicarboxylic acid 3-(4-methanesulfonyl-benzylamide);
N³-[(1,1-dioxido-2,3-dihydro-1-benzothien-5-yl)methyl]-N⁵,N⁵,6-trimethyl-2-oxo-1-[3-(trifluoromethyl)phenyl]-1,2-dihydropyridine-3,5-dicarboxamide;
5-(N¹-acetyl-hydrazinocarbonyl)-6-methyl-2-oxo-1-(3-trifluoromethyl-phenyl)-1,2-dihydro-pyridine-3-carboxylic acid 4-methanesulfonyl-benzylamide;
5-[N¹-(2-cyano-acetyl)-hydrazinocarbonyl]-6-methyl-2-oxo-1-(3-trifluoromethyl-phenyl)-1,2-dihydro-pyridine-3-carboxylic acid 4-methanesulfonyl-benzylamide;
5-{[2-(aminocarbonothioyl)hydrazino]carbonyl}-6-methyl-*N*-[4-(methylsulfonyl) benzyl]-2-oxo-1-[3-(trifluoromethyl)phenyl]-1,2-dihydropyridine-3-carboxamide;
5-hydrazinocarbonyl-6-methyl-2-oxo-1-(3-trifluoromethyl-phenyl)-1,2-dihydro-pyridine-3-carboxylic acid 4-methanesulfonyl-benzylamide;
5-({2-[(ethylamino)carbonyl]hydrazino}carbonyl)-6-methyl-*N*-[4-(methylsulfonyl) benzyl]-2-oxo-1-[3-(trifluoromethyl)phenyl]-1,2-dihydropyridine-3-carboxamide;
5-({2-[(N,N-dimethylamino)carbonyl]hydrazino}carbonyl)-6-methyl-*N*-[4-(methylsulfonyl) benzyl]-2-oxo-1-[3-(trifluoromethyl)phenyl]-1,2-dihydropyridine-3-carboxamide;
5-(3,3-dimethyl-ureido)-6-methyl-2-oxo-1-(3-trifluoromethyl-phenyl)-1,2-dihydropyridine-3-carboxylic acid 4-methanesulfonyl-benzylamide;
6-methyl-5-(3-methyl-ureido)-2-oxo-1-(3-trifluoromethyl-phenyl)-1,2-dihydro-pyridine-3-carboxylic acid 4-methanesulfonyl-benzylamide;
6-methyl-2-oxo-1-(3-trifluoromethyl-phenyl)-5-ureido-1,2-dihydro-pyridine-3-carboxylic acid 4-methanesulfonyl-benzylamide;
5-amino-6-methyl-2-oxo-1-(3-trifluoromethyl-phenyl)-1,2-dihydro-pyridine-3-carboxylic acid 4-methanesulfonyl-benzylamide;
6-methyl-N-[4-(methylsulfonyl)benzyl]-2-oxo-5-propionyl-1-[3-(trifluoromethyl)phenyl]-1,2-dihydropyridine-3-carboxamide;
5-formyl-6-methyl-N-[4-(methylsulfonyl)benzyl]-2-oxo-1-[3-(trifluoromethyl)phenyl]-1,2-dihydropyridine-3-carboxamide;
6-methyl-N-[4-(methylsulfonyl)benzyl]-2-oxo-5-(3-oxobutyl)-1-[3-(trifluoromethyl)phenyl]-1,2-dihydropyridine-3-carboxamide;
5-acetyl-N-[4-(isopropylsulfonyl)benzyl]-6-methyl-2-oxo-1-[3-(trifluoromethyl)phenyl]-1,2-dihydropyridine-3-carboxamide;
5-acetyl-1-(3-cyano-phenyl)-6-methyl-2-oxo-1,2-dihydro-pyridine-3-carboxylic acid 4-methanesulfonyl-benzylamide;
5-acetyl-1-(3-chloro-phenyl)-6-methyl-2-oxo-1,2-dihydro-pyridine-3-carboxylic acid 4-methanesulfonyl-benzylamide;
5-acetyl-6-methyl-2-oxo-1-m-tolyl-1,2-dihydro-pyridine-3-carboxylic acid 4-methanesulfonyl-benzylamide;
5-(1-hydroxyethyl)-6-methyl-N-[4-(methylsulfonyl)benzyl]-2-oxo-1-[3-(trifluoromethyl)phenyl]-1,2-dihydropyridine-3-carboxamide;
5-(1-azidoethyl)-6-methyl-N-[4-(methylsulfonyl)benzyl]-2-oxo-1-[3-(trifluoromethyl)phenyl]-1,2-dihydropyridine-3-carboxamide;
6-methyl-N-[4-(methylsulfonyl)benzyl]-5-(1-morpholin-4-ylethyl)-2-oxo-1-[3-(trifluoromethyl)phenyl]-1,2-dihydropyridine-3-carboxamide;
5-(1-hydroxypropyl)-6-methyl-N-[4-(methylsulfonyl)benzyl]-2-oxo-1-[3-(trifluoromethyl)phenyl]-1,2-dihydropyridine-3-carboxamide;
5-(1-hydroxyethyl)-N-[4-(isopropylsulfonyl)benzyl]-6-methyl-2-oxo-1-[3-(trifluoromethyl)phenyl]-1,2-dihydropyridine-3-carboxamide;
N-[4-(cyclopropylsulfonyl)benzyl]-5-formyl-6-methyl-2-oxo-1-[3-(trifluoromethyl)phenyl]-1,2-dihydropyridine-3-carboxamide;
5-[(E)-(methoxyimino)methyl]-6-methyl-N-[4-(methylsulfonyl)benzyl]-2-oxo-1-[3-(trifluoromethyl)phenyl]-1,2-dihydropyridine-3-carboxamide;
5-(hydroxymethyl)-6-methyl-N-[4-(methylsulfonyl)benzyl]-2-oxo-1-[3-(trifluoromethyl)phenyl]-1,2-dihydropyridine-3-carboxamide;
5-[(dimethylamino)methyl]-6-methyl-*N*-[4-(methylsulfonyl)benzyl]-2-oxo-1-[3-(trifluoromethyl)phenyl]-1,2-dihydropyridine-3-carboxamide;
6-methyl-5-[(methylamino)methyl]-*N*-[4-(methylsulfonyl)benzyl]-2-oxo-1-[3-(trifluoromethyl)phenyl]-1,2-dihydropyridine-3-carboxamide;
6-methyl-*N*-[4-(methylsulfonyl)benzyl]-5-(morpholin-4-ylmethyl)-2-oxo-1-[3-(trifluoromethyl)phenyl]-1,2-dihydropyridine-3-carboxamide;
5-{[(2-furylmethyl)amino]methyl}-6-methyl-*N*-[4-(methylsulfonyl)benzyl]-2-oxo-1-[3-(trifluoromethyl)phenyl]-1,2-dihydropyridine-3-carboxamide;
5-[(cyclopropylamino)methyl]-6-methyl-*N*-[4-(methylsulfonyl)benzyl]-2-oxo-1-[3-(trifluoromethyl)phenyl]-1,2-dihydropyridine-3-carboxamide;
5-{[(2-hydroxypropyl)amino]methyl}-6-methyl-*N*-[4-(methylsulfonyl)benzyl]-2-oxo-1-[3-(trifluoromethyl)phenyl]-1,2-dihydropyridine-3-carboxamide;
5-[(cyclopentylamino)methyl]-6-methyl-*N*-[4-(methylsulfonyl)benzyl]-2-oxo-1-[3-(trifluoromethyl)phenyl]-1,2-dihydropyridine-3-carboxamide;
5-{[(2-hydroxyethyl)(methyl)amino]methyl}-6-methyl-*N*-[4-(methylsulfonyl)benzyl]-2-oxo-1-[3-(trifluoromethyl)phenyl]-1,2-dihydropyridine-3-carboxamide;
6-methyl-*N*-[4-(methylsulfonyl)benzyl]-2-oxo-5-(pyrrolidin-1-ylmethyl)-1-[3-(trifluoromethyl)phenyl]-1,2-dihydropyridine-3-carboxamide;
5-{[methoxy(methyl)amino]methyl}-6-methyl-*N*-[4-(methylsulfonyl)benzyl]-2-oxo-1-[3-(trifluoromethyl)phenyl]-1,2-dihydropyridine-3-carboxamide;
5-{[(cyanomethyl)amino]methyl}-6-methyl-N-[4-(methylsulfonyl)benzyl]-2-oxo-1-[3-(trifluoromethyl)phenyl]-1,2-dihydropyridine-3-carboxamide;
5-{[(cyclopropylmethyl)amino]methyl}-6-methyl-*N*-[4-(methylsulfonyl)benzyl]-2-oxo-1-[3-(trifluoromethyl)phenyl]-1,2-dihydropyridine-3-carboxamide;
5-[(3-hydroxypyrrolidin-1-yl)methyl]-6-methyl-*N*-[4-(methylsulfonyl)benzyl]-2-oxo-1-[3-(trifluoromethyl)phenyl]-1,2-dihydropyridine-3-carboxamide;
5-(2-hydroxyethoxy)-N-[4-(isopropylsulfonyl)benzyl]-6-methyl-2-oxo-1-[3-(trifluoromethyl)phenyl]-1,2-dihydropyridine-3-carboxamide;
2-methyl-5-({[4-(methylsulfonyl)benzyl]amino}carbonyl)-6-oxo-1-[3-(trifluoromethyl)phenyl]-1,6-dihydropyridin-3-yl acetate;
5-methoxy-6-methyl-*N*-[4-(methylsulfonyl)benzyl]-2-oxo-1-[3-(trifluoromethyl)phenyl]-1,2-dihydropyridine-3-carboxamide;
5-(3-methoxypropoxy)-6-methyl-*N*-[4-(methylsulfonyl)benzyl]-2-oxo-1-[3-(trifluoromethyl)phenyl]-1,2-dihydropyridine-3-carboxamide;
2-methyl-5-({[4-(methylsulfonyl)benzyl]amino}carbonyl)-6-oxo-1-[3-(trifluoromethyl)phenyl]-1,6-dihydropyridin-3-yl methanesulfonate;
5-ethoxy-6-methyl-N-[4-(methylsulfonyl)benzyl]-2-oxo-1-[3-(trifluoromethyl)phenyl]-1,2-dihydropyridine-3-carboxamide;
5-(2-hydroxyethoxy)-6-methyl-N-[4-(methylsulfonyl)benzyl]-2-oxo-1-[3-(trifluoromethyl)phenyl]-1,2-dihydropyridine-3-carboxamide;
5-(cyanomethoxy)-6-methyl-N-[4-(methylsulfonyl)benzyl]-2-oxo-1-[3-(trifluoromethyl)phenyl]-1,2-dihydropyridine-3-carboxamide;
2-({2-methyl-5-({[4-(methylsulfonyl)benzyl]amino}carbonyl)-6-oxo-1-[3-(trifluoromethyl)phenyl]-1,6-dihydropyridin-3-yl}oxy)ethyl acetate;
5-[2-(dimethylamino)-2-oxoethoxy]-6-methyl-N-[4-(methylsulfonyl)benzyl]-2-oxo-1-[3-(trifluoromethyl)phenyl]-1,2-dihydropyridine-3-carboxamide;
5-(2-aminoethoxy)-*N*-[4-(isopropylsulfonyl)benzyl]-6-methyl-2-oxo-1-[3-(trifluoromethyl)phenyl]-1,2-dihydropyridine-3-carboxamide;
5-(acetylamino)-6-methyl-N-[4-(methylsulfonyl)benzyl]-2-oxo-1-[3-(trifluoromethyl)phenyl]-1,2-dihydropyridine-3-carboxamide;
*N*-[4-(isopropylsulfonyl)benzyl]-6-methyl-5-[3-(methylamino)propoxy]-2-oxo-1-[3-(trifluoromethyl)phenyl]-1,2-dihydropyridine-3-carboxamide;
5-(1-methoxyethyl)-6-methyl-*N*-[4-(methylsulfonyl)benzyl]-2-oxo-1-[3-(trifluoromethyl)phenyl]-1,2-dihydropyridine-3-carboxamide;
5-(2-bromo-1-methoxyethyl)-6-methyl-N-[4-(methylsulfonyl)benzyl]-2-oxo-1-[3-(trifluoromethyl)phenyl]-1,2-dihydropyridine-3-carboxamide;
5-(1-isopropoxyethyl)-6-methyl-N-[4-(methylsulfonyl)benzyl]-2-oxo-1-[3-(trifluoromethyl)phenyl]-1,2-dihydropyridine-3-carboxamide;
5-(N¹-isobutyryl-hydrazinocarbonyl)-6-methyl-2-oxo-1-(3-trifluoromethyl-phenyl)-1,2-dihydro-pyridine-3-carboxylic acid 4-methanesulfonyl-benzylamide;
*N⁵*-methoxy-6-methyl-*N³*-[4-(methylsulfonyl)benzyl]-2-oxo-1-[3-(trifluoromethyl)phenyl]-1,2-dihydropyridine-3,5-dicarboxamide;
*N⁵*-methoxy-*N⁵*,6-dimethyl-*N³*-[4-(methylsulfonyl)benzyl]-2-oxo-1-[3-(trifluoromethyl)phenyl]-1,2-dihydropyridine-3,5-dicarboxamide;
5-[(2,5-dimethyl-2,5-dihydro-1H-pyrrol-1-yl)carbonyl]-6-methyl-N-[4-(methylsulfonyl)benzyl]-2-oxo-1-[3-(trifluoromethyl)phenyl]-1,2-dihydropyridine-3-carboxamide;
6-methyl-*N³*-[4-(methylsulfonyl)benzyl]-2-oxo-*N⁵*-pyrrolidin-1-yl-1-[3-(trifluoromethyl)phenyl]-1,2-dihydropyridine-3,5-dicarboxamide;
6-methyl-N-[4-(methylsulfonyl)benzyl]-2-oxo-5-(piperidin-1-ylcarbonyl)-1-[3-(trifluoromethyl)phenyl]-1,2-dihydropyridine-3-carboxamide;
6-methyl-*N³*-[4-(methylsulfonyl)benzyl]-*N⁵*-morpholin-4-yl-2-oxo-1-[3-(trifluoromethyl)phenyl]-1,2-dihydropyridine-3,5-dicarboxamide;
6-methyl-5-[(4-methylpiperidin-1-yl)carbonyl]-N-[4-(methylsulfonyl)benzyl]-2-oxo-1-[3-(trifluoromethyl)phenyl]-1,2-dihydropyridine-3-carboxamide;
6-methyl-*N³*-[4-(methylsulfonyl)benzyl]-2-oxo-*N⁵*-piperidin-1-yl-1-[3-(trifluoromethyl)phenyl]-1,2-dihydropyridine-3,5-dicarboxamide;
*N⁵*-(tert-butyl)-*N⁵*,6-dimethyl-*N³*-[4-(methylsulfonyl)benzyl]-2-oxo-1-[3-(trifluoromethyl)phenyl]-1,2-dihydropyridine-3,5-dicarboxamide;
*N⁵*-butyl-*N⁵*,6-dimethyl-N³-[4-(methylsulfonyl)benzyl]-2-oxo-1-[3-(trifluoromethyl)phenyl]-1,2-dihydropyridine-3,5-dicarboxamide;
*N⁵*-ethyl-*N⁵*-isopropyl-6-methyl-*N³*-[4-(methylsulfonyl)benzyl]-2-oxo-1-[3-(trifluoromethyl)phenyl]-1,2-dihydropyridine-3,5-dicarboxamide;
5-[N¹-(formyl-hydrazinocarbonyl]-6-methyl-2-oxo-1-(3-trifluoromethyl-phenyl)-1,2-dihydro-pyridine-3-carboxylic acid 4-methanesulfonyl-benzylamide;
N¹-[5-(4-methanesulfonyl-benzylcarbamoyl)-2-methyl-6-oxo-1-(3-trifluoromethylphenyl)-1,6-dihydro-pyridine-3-carbonyl]-hydrazinecarboxylic acid ethyl ester;
5-({2-[(ethylamino)carbonothioyl]hydrazino}carbonyl)-6-methyl-*N*-[4-(methylsulfonyl)benzyl]-2-oxo-1-[3-(trifluoromethyl)phenyl]-1,2-dihydropyridine-3-carboxamide;
5-(isoxazolidin-2-ylcarbonyl)-6-methyl-*N*-[4-(methylsulfonyl)benzyl]-2-oxo-1-[3-(trifluoromethyl)phenyl]-1,2-dihydropyridine-3-carboxamide;
6-methyl-2-oxo-1-(3-trifluoromethyl-phenyl)-1,2-dihydro-pyridine-3,5-dicarboxylic acid 5-(methoxy-methyl-amide) 3-[4-(propane-2-sulfonyl)-benzylamide];
6-methyl-2-oxo-1-(3-trifluoromethyl-phenyl)-1,2-dihydro-pyridine-3,5-dicarboxylic acid 3-(4-ethanesulfonyl-benzylamide) 5-(methoxy-methyl-amide);
6-methyl-2-oxo-1-(3-trifluoromethyl-phenyl)-1,2-dihydro-pyridine-3,5-dicarboxylicacid 3-(4-cyclopropanesulfonyl-benzylamide) 5-(methoxy-methyl-amide);
6-methyl-2-oxo-1-(3-trifluoromethyl-phenyl)-1,2-dihydro-pyridine-3,5-dicarboxylic acid 5-[(2-hydroxy-ethyl)-amide] 3-(4-methanesulfonyl-benzylamide;
5-(isoxazolidine-2-carbonyl)-6-methyl-2-oxo-1-(3-trifluoromethyl-phenyl)1,2dihydropyridine-3-carboxylic acid 4-ethanesulfonyl-benzylamide;
5-(isoxazolidine-2-carbonyl)-6-methyl-2-oxo-1-(3-trifluoromethylphenyl) 1,2dihydropyridine-3-carboxylic acid 4-cyclopropanesulfonylbenzylamide;
5-(N-hydroxycarbamimidoyl)-6-methyl-2-oxo-1-(3-trifluoromethyl-phenyl)-1,2-dihydropyridine-3-carboxylic acid 4-methanesulfonyl-benzylamide;
*N³*-(cyclohexylmethyl)-*N⁵,N⁵*,6-trimethyl-2-oxo-1-[3-(trifluoromethyl)phenyl]-1,2-dihydropyridine-3,5-dicarboxamide;
*N*⁵,*N*⁵,6-trimethyl-2-oxo-*N*³-(pyridin-3-ylmethyl)-1-[3-(trifluoromethyl)-phenyl]-1,2-dihydropyridine-3,5-dicarboxamide;
*N*⁵,*N*⁵,6-trimethyl-*N*³-(2-morpholin-4-ylethyl)-2-oxo-1-[3-(trifluoromethyl)-phenyl]-1,2-dihydropyridine-3,5-dicarboxamide;
*N*⁵,*N*⁵,6-trimethyl-*N*³-(3-morpholin-4-ylpropyl)-2-oxo-1-[3-(trifluoromethyl)-phenyl]-1,2-dihydropyridine-3,5-dicarboxamide;
*N*³-benzyl-*N*⁵,*N*⁵,6-trimethyl-2-oxo-1-[3-(trifluoromethyl)phenyl]-1,2-dihydro-pyridine-3,5-dicarboxamide;
N³-[2-(1*H*-indol-3-yl)ethyl]-*N*⁵,*N*⁵,6-trimethyl-2-oxo-1-[3-(trifluoromethyl)-phenyl]-1,2-dihydropyridine-3,5-dicarboxamide;
*N*⁵,*N*⁵,6-trimethyl-2-oxo-*N*³-(1-phenylethyl)-1-[3-(trifluoromethyl)phenyl]-1,2-dihydropyridine-3,5-dicarboxamide;
*N*⁵,*N*⁵,6-trimethyl-2-oxo-*N*³-(2-phenylethyl)-1-[3-(trifluoromethyl)phenyl]-1,2-dihydropyridine-3,5-dicarboxamide;
*N*⁵,*N*⁵,6-trimethyl-2-oxo-*N*³-[(2R)-2-phenylcyclopropyl]-1-[3-(trifluoromethyl)-phenyl]-1,2-dihydropyridine-3,5-dicarboxamide;
*N*³-(2,3-dihydro-1*H*-inden-2-yl)-*N*⁵,*N*⁵,6-trimethyl-2-oxo-1-[3-(trifluoromethyl)-phenyl]-1,2-dihydropyridine-3,5-dicarboxamide;
*N*³-[2-(1,3-benzodioxol-5-yl)ethyl]-*N*⁵,*N*⁵,6-trimethyl-2-oxo-1-[3-(trifluoromethyl)phenyl]-1,2-dihydropyridine-3,5-dicarboxamide;
5-{[4-(2-hydroxyethyl)piperazin-1-yl]carbonyl}-*N*,*N*,2-trimethyl-6-oxo-1-[3-(trifluoromethyl)phenyl]-1,6-dihydropyridine-3-carboxamide;
*N*³-[(1-ethylpyrrolidin-2-yl)methyl]-*N*⁵,*N*⁵,6-trimethyl-2-oxo-1-[3-(trifluoromethyl)phenyl]-1,2-dihydropyridine-3,5-dicarboxamide;
*N*⁵,*N*⁵,6-trimethyl-*N*³-[3-(2-methylpiperidin-1-yl)propyl]-2-oxo-1-[3-(trifluoromethyl)phenyl]-1,2-dihydropyridine-3,5-dicarboxamide;
*N*⁵,*N*⁵,6-trimethyl-*N*³-(1-naphthylmethyl)-2-oxo-1-[3-(trifluoromethyl)phenyl]-1,2-dihydropyridine-3,5-dicarboxamide;
*N*³-(1,3-benzodioxol-5-ylmethyl)-*N*⁵,*N*⁵,6-trimethyl-2-oxo-1-[3-(trifluoro-methyl)phenyl]-1,2-dihydropyridine-3,5-dicarboxamide;
*N*³-(3,4-difluorobenzyl)-*N*⁵,*N*⁵,6-trimethyl-2-oxo-1-[3-(trifluoromethyl)phenyl]-1,2-dihydropyridine-3,5-dicarboxamide;
*N*³-(2-chloro-4-fluorobenzyl)-*N*⁵,*N*⁵,6-trimethyl-2-oxo-1-[3-(trifluoromethyl)-phenyl]-1,2-dihydropyridine-3,5-dicarboxamide;
*N*⁵,*N*⁵,6-trimethyl-2-oxo-*N*³-(2-thienylmethyl)-1-[3-(trifluoromethyl)phenyl]-1,2-dihydropyridine-3,5-dicarboxamide;
*N*³-(3,4-dichlorobenzyl)-*N*⁵,*N*⁵,6-trimethyl-2-oxo-1-[3-(trifluoromethyl)phenyl]-1,2-dihydropyridine-3,5-dicarboxamide;
*N*³-[2-(2,4-dichlorophenyl)ethyl]-*N*⁵,*N*⁵,6-trimethyl-2-oxo-1-[3-(trifluoromethyl)-phenyl]-1,2-dihydropyridine-3,5-dicarboxamide;
*N*³-(2-cyclohex-1-en-1-ylethyl)-*N*⁵,*N*⁵,6-trimethyl-2-oxo-1-[3-(trifluoromethyl)-phenyl]-1,2-dihydropyridine-3,5-dicarboxamide;
*N*³-[1-(4-chlorophenyl)ethyl]-*N*⁵,*N*⁵,6-tiimethyl-2-oxo-1-[3-(trifluoromethyl)-phenyl]-1,2-dihydropyridine-3,5-dicarboxamide;
*N*⁵,*N*⁵,6-trimethyl-2-oxo-*N*³-[3-(2-oxopyrrolidin-1-yl)propyl]-1-[3-(trifluoromethyl)phenyl]-1,2-dihydropyridine-3,5-dicarboxamide;
*N*⁵,*N*⁵,6-trimethyl-2-oxo-*N*³-(pyridin-4-ylmethyl)-1-[3-(trifluoromethyl)phenyl]-1,2-dihydropyridine-3,5-dicarboxamide;
*N*,*N*,2-trimethyl-6-oxo-5-[(4-phenylpiperazin-1-yl)carbonyl]-1-[3-(trifluoromethyl)phenyl]-1,6-dihydropyridine-3-carboxamide;
*N*,*N*,2-trimethyl-6-oxo-5-[(4-pyridin-2-ylpiperazin-1-yl)carbonyl]-1-[3-(trifluoromethyl)phenyl]-1,6-dihydropyridine-3-carboxamide;
*N*³-(2,3-dihydro-1-benzofuran-5-ylmethyl)-*N*⁵,*N*⁵,6-trimethyl-2-oxo-1-[3-(trifluoromethyl)phenyl]-1,2-dihydropyridine-3,5-dicarboxamide;
methyl 4-{[({5-[(dimethylamino)carbonyl]-6-methyl-2-oxo-1-[3-(trifluoromethyl)phenyl]-1,2-dihydropyridin-3-yl}carbonyl)amino]methyl}benzoate;
5-{[3-(dimethylamino)pyrrolidin-1-yl]carbonyl}-*N*,*N*,2-trimethyl-6-oxo-1-[3-(trifluoromethyl)phenyl]-1,6-dihydropyridine-3-carboxamide;
*N*⁵,*N*⁵,6-trimethyl-2-oxo-*N*³-[2-(2-thienyl)ethyl]-1-[3-(trifluoromethyl)phenyl]-1,2-dihydropyridine-3,5-dicarboxamide;
*N*⁵,*N*⁵,6-trimethyl-2-oxo-*N*³-(4-phenoxybenzyl)-1-[3-(trifluoromethyl)phenyl]-1,2-dihydropyridine-3,5-dicarboxamide;
*N*⁵,*N*⁵,6-trimethyl-2-oxo-*N*³-(3-thienylmethyl)-1-[3-(trifluoromethyl)phenyl]-1,2-dihydropyridine-3,5-dicarboxamide;
*N*³-[2-(4-*tert*-butylphenyl)ethyl]-*N*⁵,*N*⁵,6-trimethyl-2-oxo-1-[3-(trifluoromethyl)-phenyl]-1,2-dihydropyridine-3,5-dicarboxamide;
*N*³-{2-[4-(aminosulfonyl)phenyl]ethyl}-*N*⁵,*N*⁵,6-trimethyl-2-oxo-1-[3-(trifluoromethyl)phenyl]-1,2-dihydropyridine-3,5-dicarboxamide;
*N*⁵,*N*⁵,6-trimethyl-2-oxo-*N*³-[4-(1H-pyrazol-1-yl)benzyl]-1-[3-(trifluoromethyl)-phenyl]-1,2-dihydropyridine-3,5-dicarboxamide;
*N*⁵,*N*⁵,6-trimethyl-2-oxo-*N*³-phenoxy-1-[3-(trifluoromethyl)phenyl]-1,2-dihydro-pyridine-3,5-dicarboxamide;
*N*³-(2,3-dihydro-1,4-benzodioxin-2-ylmethyl)-*N*⁵,*N*⁵,6-trimethyl-2-oxo-1-[3-(trifluoromethyl)phenyl]-1,2-dihydropyridine-3,5-dicarboxamide;
N³-[(6-fluoro-4*H*-1,3-benzodioxin-8-yl)methyl]-*N*⁵,*N*⁵,6-trimethyl-2-oxo-1-[3-(trifluoromethyl)phenyl]-1,2-dihydropyridine-3,5-dicarboxamide;
*N*³-(1-benzothien-3-ylmethyl)-*N*⁵,*N*⁵,6-trimethyl-2-oxo-1-[3-(trifluoromethyl)-phenyl]-1,2-dihydropyridine-3,5-dicarboxamide;
*N*⁵,*N*⁵,6-trimethyl-2-oxo-*N*³-[2-(tetrahydro-2*H*-pyran-4-yl)ethyl]-1-[3-(trifluoromethyl)phenyl]-1,2-dihydropyridine-3,5-dicarboxamide;
*N*⁵,*N*⁵,6-trimethyl-*N*³-[(1-methyl-1*H*-pyrazol-4-yl)methyl]-2-oxo-1-[3-(trifluoromethyl)phenyl]-1,2-dihydropyridine-3,5-dicarboxamide;
*N*⁵,*N*⁵,6-trimethyl-2-oxo-*N*³-[(1-phenyl-1*H*-pyrazol-4-yl)methyl]-1-[3-(trifluoromethyl)phenyl]-1,2-dihydropyridine-3,5-dicarboxamide;
*N*³-[(5-methoxy-4-oxo-4*H*-pyran-2-yl)methyl]-*N*⁵,*N*⁵,6-trimethyl-2-oxo-1-[3-(trifluoromethyl)phenyl]-1,2-dihydropyridine-3,5-dicarboxamide;
*N*³-(3-azepan-1-ylpropyl)-*N*⁵,*N*⁵,6-trimethyl-2-oxo-1-[3-(trifluoromethyl)phenyl]-1,2-dihydropyridine-3,5-dicarboxamide;
*N*³-(4-cyanobenzyl)-*N*⁵,*N*⁵,6-trimethyl-2-oxo-1-[3-(trifluoromethyl)phenyl]-1,2-dihydropyridine-3,5-dicarboxamide;
*N*⁵,*N*⁵,6-trimethyl-2-oxo-*N*³-[3-(5-oxo-4,5-dihydro-1*H*-pyrazol-4-yl)propyl]-1-[3-(trifluoromethyl)phenyl]-1,2-dihydropyridine-3,5-dicarboxamide;
N3-{[(2R)-1-ethylpyrrolidin-2-yl]methyl}-*N*⁵,*N*⁵,6-trimethyl-2-oxo-1-[3-(trifluoromethyl)phenyl]-1,2-dihydropyridine-3,5-dicarboxamide;
5-cyclopropyl-6-methyl-N-[4-(methylsulfonyl)benzyl]-2-oxo-1-[3-(trifluoromethyl)phenyl]-1,2-dihydropyridine-3-carboxamide;
6-methyl-5-(2-methyl-1,3-dioxolan-2-yl)-N-[4-(methylsulfonyl)benzyl]-2-oxo-1-[3-(trifluoromethyl)phenyl]-1,2-dihydropyridine-3-carboxamide;
5-(4,5-dihydro-oxazol-2-yl)-6-methyl-2-oxo-1-(3-trifluoromethyl-phenyl)-1,2-dihydropyridine-3-carboxylic acid 4-methanesulfonyl-benzylamide;
5-cyclopropyl-6-methyl-N-{[5-(methylsulfonyl)pyridin-2-yl]methyl}-2-oxo-1-[3-(trifluoromethyl)phenyl]-1,2-dihydropyridine-3-carboxamide;
and pharmaceutically acceptable salts thereof.

The present invention includes compounds of formula (I) in the form of salts, in particular acid addition salts. Suitable salts include those formed with both organic and inorganic acids. Such acid addition salts will normally be pharmaceutically acceptable although salts of non-pharmaceutically acceptable acids may be of utility in the preparation and purification of the compound in question. Thus, preferred salts include those formed from hydrochloric, hydrobromic, sulphuric, phosphoric, citric, tartaric, lactic, pyruvic, acetic, succinic, fumaric, maleic, methanesulphonic and benzenesulphonic acids.

In a further aspect the invention provides a process for the preparation of a compound of formula (I) which comprises:
a) reacting a compound of formula (II) 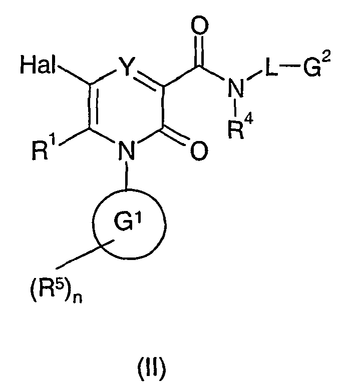 with a nucleophilic equivalent of R², such as Cu(I)CN, an alkyl vinyl ether, an organo-tin compound, an organo boronic acid, a terminal alkyne or an alcohol and carbon monoxide; wherein R¹, R², R⁴, R⁵, Y, G¹, G², L and n are as defined in formula (I) and Hal represents a halogen atom, preferably bromo or iodo; or
b) reacting a compound of formula (XV) 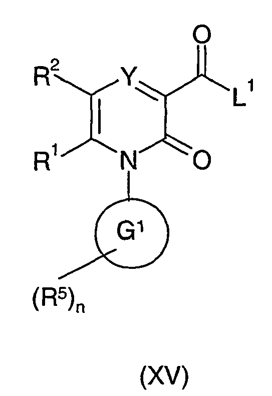
wherein R¹, R², R⁵, n, G¹ and Y are as defined in formula (I) and L¹ represents a leaving group,
with a compound of formula (IX) or a salt thereof 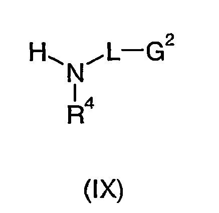 wherein R⁴, G² and L are as defined in formula (I);
and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

In process (a), the reaction is carried out at a suitable temperature, generally between 50 °C and 150 °C, in a suitable solvent such as toluene or N,N-dimethylformamide in the presence of a transition metal catalyst such as palladium or in the presence of a base such as potassium carbonate.

In process (b), the reaction is carried out at a suitable temperature, generally between 0 °C and the boiling point of the solvent, in a suitable solvent such as dichloromethane or N-methylpyrrolidinone. The process is optionally carried out in the presence of a base and/or a coupling reagent such as HATU, HOAT, HOBT or DIEA. Suitable leaving groups L¹ include OH and halogen.

The man skilled in the art will readily appreciate that many compounds of formula (I) may also be prepared by processes in which the final step or steps involve functional group interchanges within the substituent R². Some such processes are detailed below. Other specific examples of such processes are described in the Examples section of this specification. All such processes form another aspect of the present invention.

Compounds of formula (I) wherein R² represents CONR⁴⁸R⁴⁹ or CONR⁵⁰NR⁴⁸R⁴⁹ can be prepared by reacting a compound of formula (III) 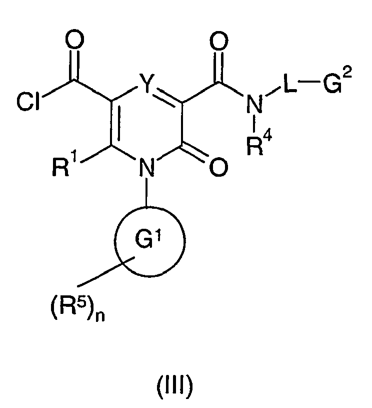 wherein R¹, R⁴, R⁵, Y, G¹, G², L and n are as defined in formula (I);
with an amine of the general formula NHR⁴⁸R⁴⁹ or NHR⁵⁰NR⁴⁸R⁴⁹.

The process is carried out at a suitable temperature, generally between 0 °C and 50 °C in a suitable solvent such as 1,4-dioxane.

Compounds of formula (III) can be prepared by reacting a compound of formula (IV) 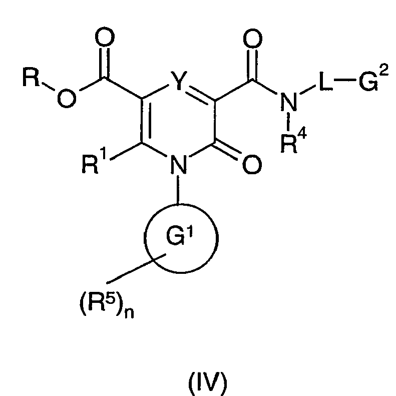 wherein R¹, R⁴, R⁵, Y, G¹, G², L and n are as defined in formula (I) and R represents an alkyl group;
with an aqueous base such as sodium hydroxide followed by subsequent treatment of the product with a chlorinating agent such as thionyl chloride. The process is carried out at a suitable temperature, generally between 10 °C and 50 °C in a suitable solvent such as tetrahydrofuran or dichloromethane.

Compounds of formula (I) wherein R² is defined as NR⁴⁸R⁴⁹ can be prepared by reacting a compound of formula (V): 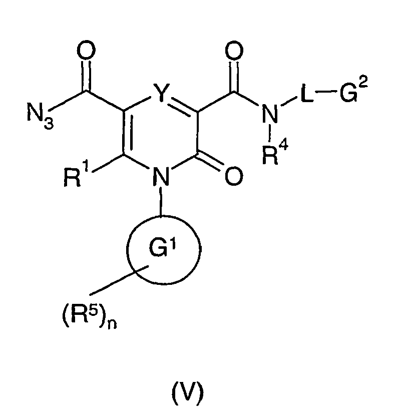 wherein R¹, R⁴, R⁵ Y, G¹, G², L and n are as defined in formula (I);
with an aqueous acid or an alkylamine. The process is carried out at a suitable temperature, generally between 50 °C and 150 °C in a suitable solvent such as toluene. The aqueous acid or alkylamine is added after heating for a time period of, typically between 0.5 and 16 hours.

Compounds of formula (V) can be prepared by reacting a compound of formula (IV) wherein R is hydrogen with diphenylphosphoryl azide. The process is carried out at a suitable temperature, generally between 0 °C and 50 °C in a suitable solvent such as dichloromethane.

Compounds of formula (IV) can be prepared by reacting a compound of formula (II) with carbon monoxide in the presence of an alcohol such as methanol or ethanol. The process is carried out at a suitable temperature, generally between 50 °C and 150 °C in a suitable solvent in a suitable solvent such as methanol or ethanol in a carbon monoxide atmosphere at elevated pressure, generally between 2 and 10 atmospheres. The reaction is performed in the presence of a transition metal catalyst such as palladium.

Compounds of formula (I) wherein R² is C2 to 6 alkanoyl can be prepared by reacting a compound of formula (VI): 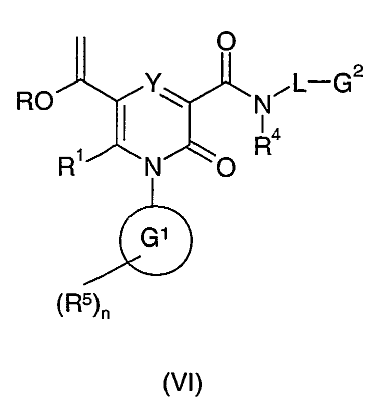 wherein R¹, R⁴, R⁵, Y, G¹, G², L and n are as defined in formula (I) and R is an alkyl group;
with an aqueous base.

The process is carried out at a suitable temperature, generally between 10 °C and 50 °C in a suitable solvent such as N,N-dimethylformamide.

Compounds of formula (VI) can be prepared by reacting a compound of formula (II) with an alkyl vinyl ether. The process is carried out at a suitable temperature, generally between 50 °C and 150 °C in a suitable solvent such as toluene or N,N-dimethylformamide in the presence of a transition metal catalyst such as palladium.

Compounds of formula (I) wherein R² is C(=NOR⁵²)R⁵³ can be prepared by reacting a compound of formula (I) wherein R² is C2 to 6 alkanoyl with an alkoxyamine or hydroxylamine. The process is carried out at a suitable temperature, generally between 50 °C and 150 °C in a suitable solvent such as or N,N-dimethylformamide.

Compounds of formula (I) wherein R² is CN can be prepared by reacting a compound of formula (II) with copper(I) cyanide. The process is carried out at a suitable temperature, generally between 100 °C and 150 °C in a suitable solvent in a suitable solvent such as 1-methyl-2-pyrrolidone.

Compounds of formula (I) wherein R² is NO₂ can be prepared by reacting a compound of formula (VII) with a nitrating agent such as nitric acid. The process is carried out at a suitable temperature, generally between 10 °C and 50 °C in a suitable solvent in a suitable solvent such as acetic anhydride.

Compounds of formula (II) can be prepared by reacting a compound of formula (VII) 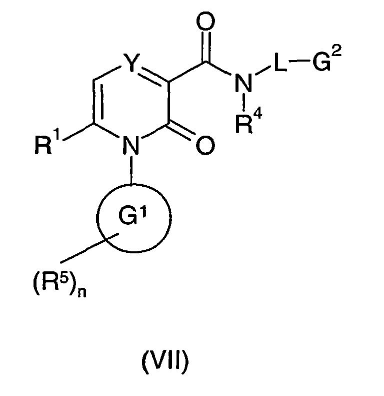 wherein R¹ R⁴, R⁵, Y, G¹, G², L and n are as defined in formula (I);
with a halogenating agent, such as N-iodosuccinimide in the presence of a strong acid such as trifluoromethanesulfonic acid.

The process is carried out at a suitable temperature, generally between 0 °C and 50 °C in a suitable solvent such as acetonitrile in the presence of an acid such as trifluoromethanesulfonic acid.

Compounds of formula (VII) can be prepared by reacting a compound of formula (VIII) 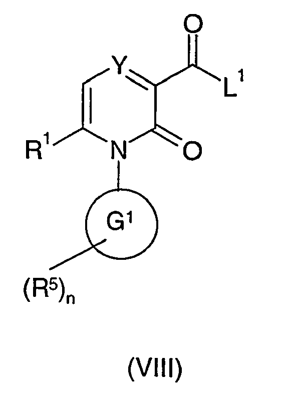 wherein R¹, R⁵, Y, G¹ and n are as defined in formula (I) and L¹ represents a leaving group, with an amine of formula (IX) or a salt thereof 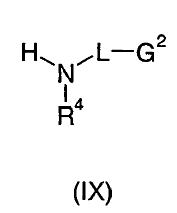 wherein R⁴, G² and L are as defined in formula (I).

The process is carried out at a suitable temperature, generally between 0 °C and the boiling point of the solvent, in a suitable solvent such as dichloromethane or N-methylpyrrolidinone. The process is optionally carried out in the presence of a base and/or a coupling reagent such as HATU, HOAT, HOBT or DIEA. Suitable leaving groups L¹ include OH and halogen.

Compounds of formula (VIII) wherein Y is CR³, L¹ is OH and R³ is hydrogen can be prepared by condensing a compound of formula (X) 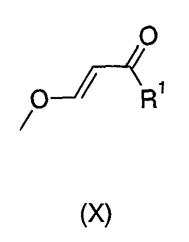 wherein R¹ is as defined in formula (I), with a compound of formula (XI) 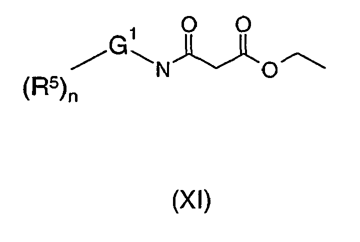 wherein G¹, R⁵ and n are as defined in formula (I), in the presence of a suitable base, such as sodium methoxide, in a suitable solvent, such as ethanol, followed by hydrolysis using a suitable base such as sodium hydroxide.

In general, compounds of formulae (X) and (XI) are either known or may be prepared using methods that will be readily apparent to the man skilled in the art. For example, compounds of formula (X) can be prepared according to the methods of S.M Brombridge et al., Synthetic Communications, 1993, 23, 487-494. And compounds of formula (XI) can be prepared according to the methods of Igor V. Ukrainets et al., Tetrahedron, 1994, 50, 10331-10338.

Compounds of formula (VIII) wherein Y is CR³, L¹ is OH and R¹ is hydrogen can be prepared by reacting a compound of formula (XII) 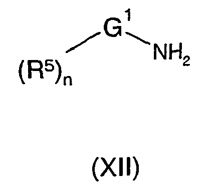 wherein G¹, R⁵ and n are as defined in formula (I), with a compound of formula (XIII) 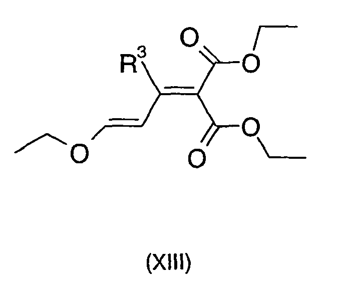 wherein R³ is as defined in formula (I), at a suitable temperature, such as 160 °C, followed by base promoted cyclisation and acid hydrolysis. Compounds of formula (XIII) can be prepared according to US 3,838,155.

Compounds of formula (VIII) wherein Y is CR³, L¹ is OH, R¹ is methyl and R³ is hydrogen can be prepared by condensing a compound of formula (XIV) 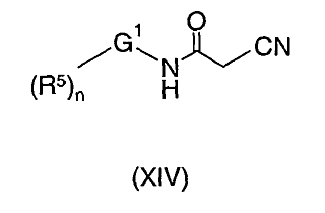 wherein G¹, R⁵ and n are as defined in formula (I), with 4-methoxy-3-buten-2-one in the presence of a suitable base, such as 1,4-diazabicyclo[2.2.2]octane, at a suitable temperature in a suitable solvent such as diethyleneglycol monomethyl ether, followed by acid hydrolysis.

Salts of compounds of formula (I) may be formed by reacting the free base or a salt, enantiomer, tautomer or protected derivative thereof, with one or more equivalents of the appropriate acid. The reaction may be carried out in a solvent or medium in which the salt is insoluble, or in a solvent in which the salt is soluble followed by subsequent removal of the solvent *in vacuo* or by freeze drying. Suitable solvents include, for example, water, dioxane, ethanol, 2-propanol, tetrahydrofuran or diethyl ether, or mixtures thereof. The reaction may be a metathetical process or it may be carried out on an ion exchange resin.

Compounds of formula (I) and intermediate compounds thereto may be prepared as such or in protected form. The protection and deprotection of functional groups is, for example, described in 'Protective Groups in Organic Chemistry', edited by J. W. F. McOmie, Plenum Press (1973), and 'Protective Groups in Organic Synthesis', 3rd edition, T. W. Greene & P. G. M. Wuts, Wiley-Interscience (1999).

The compounds of the invention and intermediates may be isolated from their reaction mixtures, and if necessary further purified, by using standard techniques.

The compounds of formula (I) may exist in enantiomeric or diastereoisomeric forms or mixtures thereof, all of which are included within the scope of the invention. The various optical isomers may be isolated by separation of a racemic mixture of the compounds using conventional techniques, for example, fractional crystallisation or HPLC. Alternatively, the individual enantiomers may be made by reaction of the appropriate optically active starting materials under reaction conditions that will not cause racemisation.

Intermediate compounds may also exist in enantiomeric forms and may be used as purified enantiomers, diastereomers, racemates or mixtures thereof.

According to a further aspect of the invention we provide a compound of formula (I) or a pharmaceutically acceptable salt thereof, for use as a medicament.

The compounds of formula (I), and their pharmaceutically acceptable salts, are useful because they possess pharmacological activity in animals. The compounds of formula (I) have activity as pharmaceuticals, in particular as modulators of human neutrophil elastase and homologous serine proteases such as proteinase 3 and pancreatic elastase, and as such are predicted to be useful in therapy. The compounds of formula (I) are particularly useful as inhibitors of human neutrophil elastase. They may thus be used in the treatment or prophylaxis of inflammatory diseases and conditions.

Examples of these conditions are: adult respiratory distress syndrome (ARDS), cystic fibrosis, pulmonary emphysema, chronic obstructive pulmonary disease (COPD) and ischaemic-reperfusion injury. The compounds of this invention may also be useful in the modulation of endogenous and/or exogenous biological irritants which cause and/or propagate atherosclerosis, diabetes, myocardial infarction; hepatic disorders including but not limited to cirrhosis, systemic lupus erythematous, inflammatory disease of lymphoid origin, including but not limited to T lymphocytes, B lymphocytes, thymocytes; autoimmune diseases, bone marrow; inflammation of the joint (especially rheumatoid arthritis, osteoarthritis and gout); inflammation of the gastro-intestinal tract (especially inflammatory bowel disease, ulcerative colitis, pancreatitis and gastritis); inflammation of the skin (especially psoriasis, eczema, dermatitis); in tumour metastasis or invasion; in disease associated with uncontrolled degradation of the extracellular matrix such as osteoarthritis; in bone resorptive disease (such as osteoporosis and Paget's disease); diseases associated with aberrant angiogenesis; the enhanced collagen remodelling associated with diabetes, periodontal disease (such as gingivitis), corneal ulceration, ulceration of the skin, post-operative conditions (such as colonic anastomosis) and dermal wound healing; demyelinating diseases of the central and peripheral nervous systems (such as multiple sclerosis); age related illness such as dementia, inflammatory diseases of cardiovascular origins; granulomatous diseases; renal diseases including but not limited to nephritis and polyarteritis; cancer; pulmonary hypertension, ingested poisons, skin contacts, stings, bites; asthma; rhinitis; HTV disease progression; for minimising the effects of organ rejection in organ transplantation including but not limited to human organs; and replacement therapy of proteinase inhibitors.

Thus, another aspect of the invention provides the use of a compound of formula (I) or a pharmaceutically acceptable salt thereof, in the manufacture of a medicament for the treatment or prophylaxis of diseases or conditions in which inhibition of neutrophil elastase activity is beneficial; and a method of treating, or reducing the risk of, diseases or conditions in which inhibition of neutrophil elastase activity is beneficial which comprises administering to a person suffering from or at risk of, said disease or condition, a therapeutically effective amount of a compound of formula (I) or a pharmaceutically acceptable salt thereof.

In another aspect, the invention provides the use of a compound of formula (I) or a pharmaceutically acceptable salt thereof, in the manufacture of a medicament for the treatment or prophylaxis of inflammatory diseases or conditions; and a method of treating, or reducing the risk of, inflammatory diseases or conditions which comprises administering to a person suffering from or at risk of, said disease or condition, a therapeutically effective amount of a compound of formula (I) or a pharmaceutically acceptable salt thereof.

In particular, the compounds of this invention may be used in the treatment of adult respiratory distress syndrome (ARDS), cystic fibrosis, pulmonary emphysema, chronic obstructive pulmonary disease (COPD), pulmonary hypertension, asthma, rhinitis, ischemia-reperfusion injury, rheumatoid arthritis, osteoarthritis, cancer, atherosclerosis and gastric mucosal injury.

Prophylaxis is expected to be particularly relevant to the treatment of persons who have suffered a previous episode of, or are otherwise considered to be at increased risk of, the disease or condition in question. Persons at risk of developing a particular disease or condition generally include those having a family history of the disease or condition, or those who have been identified by genetic testing or screening to be particularly susceptible to developing the disease or condition.

For the above mentioned therapeutic indications, the dose of the compound to be administered will depend on the compound employed, the disease being treated, the mode of administration, the age, weight and sex of the patient. Such factors may be determined by the attending physician. However, in general, satisfactory results are obtained when the compounds are administered to a human at a daily dosage of between 0.1 mg/kg to 100 mg/kg (measured as the active ingredient).

The compounds of formula (I) may be used on their own, or in the form of appropriate pharmaceutical formulations comprising the compound of the invention in combination with a pharmaceutically acceptable diluent, adjuvant or carrier. Particularly preferred are compositions not containing material capable of causing an adverse reaction, for example, an allergic reaction. Conventional procedures for the selection and preparation of suitable pharmaceutical formulations are described in, for example, "Pharmaceuticals - The Science of Dosage Form Designs", M. E. Aulton, Churchill Livingstone, 1988.

According to the invention, there is provided a pharmaceutical formulation comprising preferably less than 95% by weight and more preferably less than 50% by weight of a compound of formula (I) in admixture with a pharmaceutically acceptable diluent or carrier.

We also provide a method of preparation of such pharmaceutical formulations that comprises mixing the ingredients.

The compounds may be administered topically, for example, to the lungs and/or the airways, in the form of solutions, suspensions, HFA aerosols or dry powder formulations, for example, formulations in the inhaler device known as the Turbuhaler^{®}; or systemically, for example, by oral administration in the form of tablets, pills, capsules, syrups, powders or granules; or by parenteral administration, for example, in the form of sterile parenteral solutions or suspensions; or by rectal administration, for example, in the form of suppositories.

Dry powder formulations and pressurized HFA aerosols of the compounds of the invention may be administered by oral or nasal inhalation. For inhalation, the compound is desirably finely divided. The finely divided compound preferably has a mass median diameter of less than 10 µm, and may be suspended in a propellant mixture with the assistance of a dispersant, such as a C₈-C₂₀ fatty acid or salt thereof, (for example, oleic acid), a bile salt, a phospholipid, an alkyl saccharide, a perfluorinated or polyethoxylated surfactant, or other pharmaceutically acceptable dispersant.

The compounds of the invention may also be administered by means of a dry powder inhaler. The inhaler may be a single or a multi dose inhaler, and may be a breath actuated dry powder inhaler.

One possibility is to mix the finely divided compound with a carrier substance, for example, a mono-, di- or polysaccharide, a sugar alcohol, or an other polyol. Suitable carriers are sugars, for example, lactose, glucose, raffinose, melezitose, lactitol, maltitol, trehalose, sucrose, mannitol; and starch. Alternatively the finely divided compound may be coated by another substance. The powder mixture may also be dispensed into hard gelatine capsules, each containing the desired dose of the active compound.

Another possibility is to process the finely divided powder into spheres which break up during the inhalation procedure. This spheronized powder may be filled into the drug reservoir of a multidose inhaler, for example, that known as the Turbuhaler^{®} in which a dosing unit meters the desired dose which is then inhaled by the patient. With this system the active compound, with or without a carrier substance, is delivered to the patient.

For oral administration the active compound may be admixed with an adjuvant or a carrier, for example, lactose, saccharose, sorbitol, mannitol; a starch, for example, potato starch, corn starch or amylopectin; a cellulose derivative; a binder, for example, gelatine or polyvinylpyrrolidone; and/or a lubricant, for example, magnesium stearate, calcium stearate, polyethylene glycol, a wax, paraffin, and the like, and then compressed into tablets. If coated tablets are required, the cores, prepared as described above, may be coated with a concentrated sugar solution which may contain, for example, gum arabic, gelatine, talcum, titanium dioxide, and the like. Alternatively, the tablet may be coated with a suitable polymer dissolved in a readily volatile organic solvent.

For the preparation of soft gelatine capsules, the compound may be admixed with, for example, a vegetable oil or polyethylene glycol. Hard gelatine capsules may contain granules of the compound using either the above mentioned excipients for tablets. Also liquid or semisolid formulations of the drug may be filled into hard gelatine capsules.

Liquid preparations for oral application may be in the form of syrups or suspensions, for example, solutions containing the compound, the balance being sugar and a mixture of ethanol, water, glycerol and propylene glycol. Optionally such liquid preparations may contain colouring agents, flavouring agents, saccharine and/or carboxymethylcellulose as a thickening agent or other excipients known to those skilled in art.

The compounds of the invention may also be administered in conjunction with other compounds used for the treatment of the above conditions.

The following Examples are intended to illustrate, but in no way limit the scope of the invention.

### General Methods

¹H NMR and ¹³C NMR spectra were recorded on a Varian *Inova* 400 MHz or a Varian *Mercury*-VX 300 MHz instrument. The central peaks of chloroform-*d* (δ_{H} 7.27 ppm), dimethylsulfoxide-*d₆* (δ_{H} 2.50 ppm), acetonitrile-*d₃* (δ_{H} 1.95 ppm) or methanol-*d₄* (δ_{H} 3.31. ppm) were used as internal references. Column chromatography was carried out using silica gel (0.040-0.063 mm, Merck). Unless stated otherwise, starting materials were commercially available. All solvents and commercial reagents were of laboratory grade and were used as received.

The following abbreviations are used:
- HBTU: O-(Benzotriazol-1-yl)-N,N,N',N'-tetramethyluronium hexafluorophosphate;
- HATU: O-(7-Azabenzotriazol-1-yl)-N,N,N',N'-tetramethyluronium hexafluorophosphate;
- HOBT: 1-Hydroxybenzotriazole;
- HOAT: 1-Hydroxy-7-azabenzotriazole;
- DIEA: N,N-Diisopropylethylamine;
- NMP: 1-N-Methyl-2-pyrrolidinone;
- DME: 1,2-Dimethoxyethane;
- THF: Tetrahydrofuran;
- TFA: Trifluoroacetic acid;
- DMF: N,N-Dimethylformamide;
- DCM: Dichloromethane.

The following method was used for LC/MS analysis:
Instrument Agilent 1100; Column Waters Symmetry 2.1 x 30 mm; Mass APCI; Flow rate 0.7 ml/min; Wavelength 254 nm; Solvent A: water + 0.1% TFA; Solvent B: acetonitrile + 0.1 % TFA ; Gradient 15-95%B 8 min, 95% B 1 min.
Analytical chromatography was run on a Symmetry C₁₈-column, 2.1 x 30 mm with 3.5 µm particle size, with acetonitrile/water/0.1% trifluoroacetic acid as mobile phase in a gradient from 5% to 95% acetonitrile over 8 minutes at a flow of 0.7 ml/min.

### Example 1 5-Cyano-6-methyl-N-[4-(methylsulfonyl)benzyl]-2-oxo-1-[3-(trifluoromethyl) phenyl]-1,2-dihydropyridine-3-carboxamide

### a) Ethyl 3-oxo-3-{[3-(trifluoromethyl)phenyl]amino}propanoate

To an ice-cooled solution of 3-(trifluoromethyl)aniline (64.5 g, 0.40 mol) and triethylamine (60 ml) in acetone (700 ml) was added dropwise ethyl 3-chloro-3-oxopropanoate (63.6 g, 0.42 mol) in acetone (50 ml). After the addition (approx. 30 minutes) stirring was continued at room temperature overnight. The solvents were removed and water (1200 ml) was added. The resulting precipitate was filtered off, thoroughly washed twice with water and then dried to afford the title compound as yellow powder (109 g, 99%).
¹H NMR (CDCl₃): δ 9.52 (1H, s); 7.87 (1H, s); 7.78 (1H, d); 7.46 (1H, t); 7.39 (1H, d); 4.29 (2H, q); 3.50 (2H, s); 1.35 (3H, t).
APCI-MS m/z: 276.1 [MH⁺].

### b) 6-Methyl-2-oxo-1-[3-(trifluoromethyl)phenyl]-1,2-dihydropyridine-3-carboxylic acid

To a solution of ethyl 3-oxo-3-{ [3-(trifluoromethyl)phenyl]amino}propanoate (19.2 g, 70 mmol) and sodium methoxide (7.6 g, 140 mmol) in EtOH (250 ml) was added 4-methoxybut-3-en-2-one (90%) (7.72 g, 77 mmol). After the addition, the reaction mixture was refluxed for 2 h and then cooled. Water (50 ml) and 2M NaOH were added and the mixture was stirred at room temperature overnight. The organic solvents were removed and the reaction mixture was extracted (washed) with EtOAc. The water phases were acidified with hydrochloric acid to pH 3-4, an orange coloured precipitate appeared and was filtered off, washed with water and dried. Recrystallisation twice from heptane/EtOAc (4:1) afforded the title compound (12 g, 58%) as a white powder.
¹H NMR (CDCl₃): δ 13.68 (1H, s); 8.54 (1H, d); 7.86 (1H, d); 7.79 (1H, t); 7.55 (1H, brs); 7.48 (1H, d); 6.58 (1H, d); 2.16 (3H, s).
APCI-MS m/z: 298.1 [MH⁺].

### c) 6-Methyl-N-[4-(methylsulfonyl)benzyl]-2-oxo-1-[3-(trifluoromethyl)phenyl]-1,2-dihydropyridine-3-carboxamide

A mixture of 6-methyl-2-oxo-1-[3-(trifluoromethyl)phenyl]-1,2-dihydropyridine-3-carboxylic acid (7.43 g, 25 mmol), HATU (10.5 g, 27.5 mmol), HOAT (3.75 g, 27.5 mmol) and DIEA (14.2 ml, 82.5 mmol) in NMP (65 ml) was reacted for 1 h, then 4-methylsulphonylbenzyl amine hydrochloride (5.8 g, 26 mmol) was added. After 1 h, the reaction mixture was slowly poured into stirred ice water (1 L). A powder was formed, and the water mixture was acidified to pH 3 with citric acid (0.5 M), and stirring was continued for 1h. The precipitate was filtered off, washed with water and dried in vacuum overnight. Recrystallisation from EtOAc gave 8.1 g (70%).
¹H NMR (CDCl₃): δ 10.00 (1H, brt); 8.60 (1H, d); 7.88 (2H, d); 7.83 (1H, d); 7.76 (1H, t); 7.53 (3H, m); 7.46 (1H, d); 6.49 (1H, d); 4.68 (2H, m); 3.03 (3H, s); 2.10 (3H, s).
APCI-MS m/z: 465.1 [MH⁺].

### d) 5-Iodo-6-methyl-N-[4-(methylsulfonyl)benzyl]-2-oxo-1-[3-(trifluoromethyl)phenyl]-1,2-dihydropyridine-3-carboxamide

To a solution of 6-methyl-N-[4-(methylsulfonyl)benzyl]-2-oxo-1-[3-(trifluoromethyl)phenyl]-1,2-dihydropyridine-3-carboxamide (200 mg, 0.43 mmol) in MeCN (1.5 ml) at room temperature and under argon was added trifluoromethanesulfonic acid (1 ml) followed by N-iodosuccinimide (97 mg, 0.43 mmol). After 45 min, the reaction mixture was diluted with DCM, washed with aqueous NaHCO₃ with aqueous NaS₂O₄ and water, dried (Na₂SO₄), and evaporated to give the title compound (200 mg).
¹H NMR (CDCl₃): δ 9.85 (1H, brt); 8.90 (1H, d); 7.88 (2H, d); 7.76 (2H, m); 7.50 (2H, d); 7.48 (1H, s); 7.40 (1H, d); 4.65 (2H, m); 3.03 (3H, s); 2.32 (3H, s).
APCI-MS m/z: 591.0 [MH⁺].

### e) 5-Cyano-6-methyl-N-[4-(methylsulfonyl)benzyl]-2-oxo-1-[3-(trifluoromethy)phenyl]-1,2-dihydropyridine-3-carboxamide

A mixture of 5-iodo-6-methyl-N-[4-(methylsulfonyl)benzyl]-2-oxo-1-[3-(trifluoromethyl)phenyl]-1,2-dihydropyridine-3-carboxamide (120 mg, 0.20 mmol) and copper (I) cyanide (66.7 mg, 0.74 mmol) in NMP (2.5 ml) was stirred overnight at 140 °C. The reaction mixture was cooled and partitioned between ethyl acetate and water. The organic layer was dried over sodium sulphate, filtered and concentrated in vacuo. The residue was first purified by preparative HPLC and then by flash chromatography eluting with DCM/methanol (10:0.2) to give the title compound as a white solid (24 mg, 24 %).
¹H NMR (DMSO-d₆): δ 9.55 (1H, t, J 6.1 Hz); 8.49 (1H, s); 7.96 (1H, s); 7.93 (1H, d, J 7.8 Hz); 7.88 - 7.81 (3H, m); 7.77 (1H, d, J 8.0 Hz); 7.52 (2H, d, J 8.4 Hz); 4.56 (2H, d, J 6.2 Hz); 3.16 (3H, s); 2.22 (3H, s).
APCI-MS m/z: 490 [MH⁺].

### Example 2 6-Methyl-N-[4-(methylsulfonyl)benzyl]-5-nitro-2-oxo-1-[3-(trifluoromethyl)phenyl]-1,2-dihydropyridine-3-carboxamide

To a solution of 6-methyl-N-[4-(methylsulfonyl)benzyl]-2-oxo-1-[3-(trifluoromethyl)phenyl]-1,2-dihydropyridine-3-carboxamide (52 mg, 0.11 mmol) in acetic anhydride (2 ml) was added fuming nitric acid (0.1 ml, 2.1 mmol). The reaction mixture was stirred at room temperature for 40 min. The mixture was partitioned between ethyl acetate and aqueous sodium hydrogen carbonate. The organic layer was washed with water, dried over sodium sulfate, filtered and concentrated in vacuo. The residue was purified by preparative HPLC to give the title compound as a yellow powder (13 mg, 23%).
¹H NMR (CDCl₃): δ 9.47 (1H, t, J 5.6 Hz); 9.31 (1H, s); 7.92 - 7.86 (3H, m); 7.81 (1H, t, J 7.9 Hz); 7.54 - 7.48 (3H, m); 7.44 (1H, d, J 7.9 Hz); 4.69 (2H, dd, J 5.9, 3.9 Hz); 3.03 (3H, s); 2.52 (3H, s).
APCI-MS m/z: 510 [MH⁺].

### Example 3 5-(1-Butoxyvinyl)-6-methyl-N-[4-(methylsulfonyl)benzyl]-2-oxo-1-[3-(trifluoromethyl)phenyl]-1,2-dihydropyridine-3-carboxamide

In a Schlenk vessel equipped with a magnetic stirring bar were placed 5-iodo-6-methyl-N - [4-(methylsulfonyl)benzyl]-2-oxo-1-[3-(trifluoromethyl)phenyl]-1,2-dihydropyridine-3-carboxamide (101.5 mg, 0.17 mmol), bis[1.2-bis(diphenylphosphino)ethane]-palladium (0) (16.5 mg, 18.3 µmol), n-butyl vinyl ether (60 µl, 0.46 mmol), triethylamine (0.5 ml, 3.6 mmol) and DMF (6 ml). The vessel was purged with argon, sealed and heated at 100°C overnight. The reaction mixture was cooled and partitioned between ethyl acetate and water. The organic layer was dried over sodium sulphate, filtered and concentrated in vacuo. The residue was purified by preparative HPLC to give the title compound as a white solid (27.3 mg, 28 %).
¹H NMR (CDCl₃): δ 9.96 (1H, t, J 5.8 Hz); 8.64 (1H, s); 7.89 (2H, d, J 8.3 Hz); 7.82 (1H, d, J 8.0 Hz); 7.75 (1H, t, J 7.9 Hz); 7.56 - 7.50 (3H, m); 7.46 (1H, d, J 7.8 Hz); 4.69 (2H, ddd, J 22.1, 15.7, 6.2 Hz); 4.43 (1H, d, J 2.6 Hz); 4.26 (1H, d, J 2.6 Hz); 3.83 (2H, t, J 6.5 Hz); 3.03 (3H, s); 2.11 (3H, s); 1.74 (2H, quintet, J 9.2 Hz); 1.46 (2H, sextet, J 9.1 Hz); 0.98 (3H, t, J 7.4 Hz).
APCI-MS m/z: 563 [MH⁺].

### Example 4 5-Acetyl-6-methyl-N-[4-(methylsulfonyl)benzyl]-2-oxo-1-[3-(trifluoromethyl)phenyl]-1,2-dihydropyridine-3-carboxamide

To a solution of 5-(1-butoxyvinyl)-6-methyl-N-[4-(methylsulfonyl)benzyl]-2-oxo-1-[3-(trifluoromethyl)phenyl]-1,2-dihydropyridine-3-carboxamide (38 mg, 67.5 µmol) in DMF (0.5 ml) was added aqueous hydrochloric acid (2.0M, 50 µl). After 20 min. the solution was neutralized with aqueous sodium hydrogen carbonate. The reaction mixture was purified by preparative HPLC to give the title compound as a white solid (17.6 mg, 51%).
¹H NMR (CDCl₃): δ 9.75 (1H, t, J 5.7 Hz); 9.08 (1H, s); 7.90 (2H, d, J 8.3 Hz); 7.85 (1H, d, J 7.9 Hz); 7.78 (1H, t, J 7.9 Hz); 7.54 (2H, d, J 8.3 Hz); 7.50 (1H, s); 7.42 (1H, d, J 8.0 Hz); 4.70 (2H, t, J 6.0 Hz); 3.03 (3H, s); 2.66 (3H, s); 2.43 (3H, s).
APCI-MS m/z: 507 [MH⁺].

### Example 5 5-[(1E)-N-Methoxyethanimidoyl]-6-methyl-N-[4-(methylsulfonyl)benzyl]-2-oxo-1-[3-(trifluoromethyl)phenyl]-1,2-dihydropyridine-3-carboxamide

A mixture of 5-acetyl-6-methyl-N-[4-(methysulfonyl)benzyl]-2-oxo-1-[3-(trifluoromethyl)phenyl]-1,2-dihydropyridine-3-carboxamide (47.2 mg, 0.09 mmol), methoxylamine hydrochloride (22.9 mg, 0.27 mmol), potassium carbonate (36 mg, 0.26 mmol) and DMF (1 ml) was heated at 100°C for 1 h. After cooling the reaction mixture was neutralized with aqueous 2.0M hydrochloric acid and purified by preparative HPLC to give the title compound as a white solid (9 mg, 18 %).
¹H NMR (CDCl₃): δ 9.91 (1H, t, J 6.0 Hz); 8.58 (1H, s); 7.88 (2H, d, J 8.3 Hz); 7.82 (1H, d, J 8.3 Hz); 7.75 (1H, t, J 7.9 Hz); 7.54 - 7.49 (3H, m); 7.44 (1H, d, J 7.8 Hz); 4.67 (2H, t, J 5.9 Hz); 3.96 (3H, s); 3.02 (3H, s); 2.20 (3H, s); 2.14 (3H, s).
APCI-MS m/z: 536 [MH⁺].

### Example 6 5-[(1E)-N-Hydroxyethanimidoyl]-6-methyl-N-[4-(methylsulfonyl)benzyl]-2-oxo-1-[3-(trifluoromethyl)phenyl]-1,2-dihydropyridine-3-carboxamide

A mixture of 5-acetyl-6-methyl-N-[4-(methylsulfonyl)benzyl]-2-oxo-1-[3-(trifluoromethyl)phenyl]-1,2-dihydropyridine-3-carboxamide (34.7 mg, 68.5 µmol), hydroxylamine hydrochloride (30.3 mg, 0.44 mmol), triethylamine (0.5 ml, 3.6 mmol), tetrahydrofuran (1 ml) and methanol (0.5 ml) was heated at 70 °C for 48 h. The reaction mixture was cooled and concentrated in vacuo. The residue was purified by preparative HPLC to give the title compound as a white solid (8 mg, 22 %).
¹H NMR (CDCl₃): δ 9.92 (1H, t, J 5.6 Hz); 8.59 (1H, s); 7.88 (2H, d, J 8.1 Hz); 7.82 (1H, d, J 7.7 Hz); 7.75 (1H, t, J 7.8 Hz); 7.52 (4H, d, J 4.2 Hz); 7.44 (1H, d, J 7.5 Hz); 4.68 (2H, ddd, J 21.2, 15.3, 6.0 Hz); 3.02 (3H, s); 2.25 (3H, s); 2.12 (3H, s).
APCI-MS m/z: 522 [MH⁺].

### Example 7 6-Methyl-N-[4-(methylsulfonyl)benzyl]-2-oxo-5-(pyridin-3-ylethynyl)-1-[3-(trifluoromethyl)phenyl]-1,2-dihydropyridine-3-carboxamide

A mixture of 5-iodo-6-methyl-N-[4-(methylsulfonyl)benzyl]-2-oxo-1-[3-(trifluoromethyl)phenyl]-1,2-dihydropyridine-3-carboxamide (86.7 mg, 0.15 mmol), copper(I) iodide (3.1 mg, 0.016 mmol), 1,10-phenanthroline monohydrate (5.9 mg, 0.03 mmol), triphenylphosphine (13.8 mg, 0.05 mmol), cesium carbonate (70 mg, 0.22 mmol) tris(dibenzylideneacetone)dipalladium(0) (10 mg, 0.01 mmol), 3-(trimethylsilylethynyl)pyridine (38.1 mg, 0.22 mmol) in toluene (15 ml) was heated at 100 °C under argon for 48 h. The reaction mixture was cooled and partitioned between ethyl acetate and water. The organic layer was dried over sodium sulphate, filtered and concentrated in vacuo. The residue was first purified by preparative HPLC on a kromasil column and then by flash chromatography eluting with DCM/methanol (10:0.25) to give the title compound as a white solid (29.7 mg, 36 %).
¹H NMR (CDCl₃): δ 9.81 (1H, t, J 5.5 Hz); 8.76 (2H, d, J 11.3 Hz); 8.60 (1H, d, J 4.7 Hz); 7.94 - 7.83 (3H, m); 7.83 - 7.75 (2H, m); 7.57 - 7.50 (3H, m); 7.47 (1H, d, J 7.7 Hz); 7.33 (1H, dd, J 7.7, 5.0 Hz); 4.70 (2H, t, J 5.4 Hz); 3.03 (3H, s); 2.35 (3H, s).
APCI-MS m/z: 566 [MH⁺].

### Example 8 6-Methyl-N-[4-(methlsulfonyl)benzyl]-2-oxo-5-(2-pyndin-3-ylethyl)-1-[3-(trifluoromethyl)phenyl]-1,2-dihydropyridine-3-carboxamide

A suspension of 6-methyl-N-[4-(methylsulfonyl)benzyl]-2-oxo-5-(pyridin-3-ylethynyl)-1-[3-(trifluoromethyl)phenyl]-1,2-dihydropyridine-3-carboxamide (20 mg, 0.035 mmol), 5% palladium on carbon (10 mg) in ethanol (5 ml) and ethyl acetate (5 ml) was stirred vigorously under a hydrogen atmosphere for 48 h. The mixture was filtered through Celite, the filtrate was evaporated to dryness and the residue was purified by preparative HPLC to give the title compound as a white solid (11 mg, 55%).
¹H NMR (DMSO-d₆): δ 9.96 (1H, t, J 6.2 Hz); 8.45 (1H, d, J 1.8 Hz); 8.40 (1H, dd, J 4.8, 1.5 Hz); 8.29 (1H, s); 7.91 - 7.75 (5H, m); 7.72 - 7.60 (2H, m); 7.52 (2H, d, J 8.3 Hz); 7.34 - 7.27 (1H, m); 4.56 (2H, d, J 6.0 Hz); 3.16 (3H, s); 2.88 (4H, s); 1.87 (3H, s).
APCI-MS m/z: 570 [MH⁺].

### Example 9 6-Methyl-N-[4-(methylsulfonyl)benzyl]-oxo-1-[3-(trifluoromethyl)phenyl]-5-vinyl-1,2-dihydropyridine-3-carboxamide

A mixture of 5-iodo-6-methyl-N-[4-(methylsulfonyl)benzyl]-2-oxo-1-[3-(trifluoromethyl)phenyl]-1,2-dihydropyridine-3-carboxamide (53 mg, 0.09 mmol), tributyl(vinyl)tin (51 mg, 0.16 mmol), tetrakis(triphenylphosphine)palladium(0) (6.9 mg, 0.006 mmol) in toluene (10 ml) was heated at 100°C under argon for 12 h. After cooling, the solvent was removed in vacuo and the residue was purified by preparative HPLC to give the title compound as a white solid (18 mg, 41%).
¹H NMR (CDCl₃): δ 9.98 (1H, t, J 5.9 Hz); 8.85 (1H, s); 7.87 (2H, dt, J 8.5,1.9 Hz); 7.83 - 7.69 (2H, m); 7.54 - 7.47 (3H, m); 7.42 (1H, d, J 7.7 Hz); 6.69 (1H, dd, J 17.2, 11.0 Hz); 5.73 (1H, d, J 17.2 Hz); 5.40 (1H, d, J 11.2 Hz); 4.67 (2H, dd, J 5.9, 3.7 Hz); 3.01 (3H, s); 2.10 (3H, s).
APCI-MS m/z: 491 [MH⁺].

### Example 10 Ethyl 2-methyl-5-({[4-(methylsulfonyl)benzyl]amino}carbonyl)-6-oxo-1-[3-(trifluoromethyl)phenyl]-1,6-dihydropyridine-3-carboxylate

In a stainless-steel autoclave (100 ml) were placed 5-iodo-6-methyl-N-[4-(methylsulfonyl)benzyl]-2-oxo-1-[3-(trifluoromethyl)phenyl]-1,2-dihydropyridine-3-carboxamide (108.1 mg, 0.18 mmol), palladium(II) acetate (3.8 mg, 0.02 mmol), triphenylphosphine (10.3 mg, 0.04 mmol), triethylamine (2 ml, 14.4 mmol) and ethanol (6 ml). The reaction mixture was magnetically stirred at 100°C under a carbon monoxide pressure of 4 atmospheres overnight. After cooling, the solvent was evaporated off and the residue was purified by preparative HPLC to give the title compound as a white solid (77.6 mg, 79 %).
¹H NMR (CDCl₃): δ 9.73 (1H, t, J 5.9 Hz); 9.20 (1H, s); 7.90 (2H, d, J 8.3 Hz); 7.85 (1H, d, J 7.9 Hz); 7.78 (1H, t, J 7.8 Hz); 7.53 (2H, d, J 8.3 Hz); 7.50 (1H, s); 7.42 (1H, d, J 8.0 Hz); 4.69 (2H, t, J 5.9 Hz); 4.38 (2H, q, J 7.2 Hz); 3.03 (3H, s); 2.50 (3H, s); 1.42 (3H, t, J 7.2 Hz).
APCI-MS m/z: 537 [MH⁺].

### Example 11 5-(4-Methanesulfonyl-benzylcarbamoyl)-2-methyl-6-oxo-1-(3-trifluoromethyl-phenyl)-1,6-dihydro-pyridine-3-carboxylic acid

To a solution of ethyl 2-methyl-5-({[4-(methylsulfonyl)benzyl]amino}carbonyl)-6-oxo-1-[3-(trifluoromethyl)phenyl]-1,6-dihydropyridine-3-carboxylate (0.70 g, 1.30 mmol) in THF (10 ml) and water (10 ml) was added 1M NaOH (2 ml, 2 mmol), and the mixture was stirred for 1 h at room temperature, monitoring the progress of the reaction by LC-MS. 20% conversion was observed, and another portion of 1M NaOH (1 ml, 1 mmol) was added, and the reaction was allowed to run for another 1 h. This procedure was repeated until complete conversion of the ester was observed (normally 3-4 hours). The outcome of the reaction is two compounds with the same mass, in a 95:5 proportion. The main product is the subtitle compound, and the other is a regioisomer. The reaction mixture was evaporated to remove THF, and the residual water solution was acidified and extracted with EtOAc. The organic phase was collected and dried over Na₂SO₄. Filtration and evaporation gave a crude product 0.60 g (90%) as a yellowish solid, which can be used further without purification. A portion was purified by preparative HPLC,
¹H NMR (CDCl₃): δ 9.90 (1H, t, J 6.2 Hz); 9.31 (1H, s); 7. 9 (2H, d, J 8.2 Hz); 7.84 (1H, d, J 8.0 Hz); 7.77 (1H, t, J 8.0 Hz); 7.51 (2H, d, J 8.5 Hz); 4.5 (1H, s); 7.41 (1H, d, J 8.0 Hz); 4.92 (1H, bs); 4.78-4.63 (2H, m); 3.01 (3H, s); 2.53 (3H, s).
APCI-MS m/z: 509.2 [MH⁺].

### Example 12 6-Methyl-2-oxo-1-(3-trifluoromethyl-phenyl)-1,2-dihydro-pyridine-3,5-dicarboxylic acid 5-dimethylamide 3-(4-methanesulfonyl-benzylamide)

To 5-(4-methanesulfonyl-benzylcarbamoyl)-2-methyl-6-oxo-1-(3-trifluoromethyl-phenyl)-1,6-dihydro-pyridine-3-carboxylic acid in CH₂Cl₂ (5 ml), SOCl₂ (3 ml) was added, and the flask was sealed and stirred magnetically for 2 h. The crude mixture was evaporated in vacuo giving the intermediate acid chloride as a yellow solid. The solid was dissolved in 1,4-dioxan (5 ml, dried over molecular sieves) and dimethylamine (40% aqueous solution, 0.5 ml) was added quickly. The mixture was stirred for 5 minutes, and LC-MS showed complete formation of the title compound. The mixture was concentrated in vacuo and the residue was purified by preparative HPLC, giving 0.008 g (76%) of the title compound as a white solid after freeze-drying the pure fractions.
¹H NMR (CDCl₃): δ 9.86 (1H, t, J 5.9 Hz); 8.73 (1H, s); 7.89 (2H, d, J 8.3 Hz); 7.84 (1H, d, J 7.8 Hz); 7.77 (1H, t, J 7.8 Hz); 7.52 (2H, d, J 8.4 Hz); 7.51 (1H, s); 7.43 (1H, d, J 7.9 Hz); 6.16 (1H, bs); 4.75-4.63 (2H, m); 3.03 (3H, s); 3.02 (3H, d, J 4.3 Hz); 2.33 (3H, s).
APCI-MS m/z: 536.2 [MH⁺].

Using the general method of Example 12, the compounds of Examples 13 to 20 and 22 to 25 were prepared.

### Example 13 6-Methyl-2-oxo-1-(3-trifluoromethyl-phenyl)-1,2-dihydro-pyridine-3,5-dicarboxylic acid 5-amide 3-(4-methanesulfonyl-benzylamide)

¹H NMR (CDCl₃): δ 9.82 (1H, t, J 5.9 Hz); 8.80 (1H, s); 7.78 (2H, d, J 8.8 Hz); 7.84 (1H, d, J 8.1 Hz); 7.76 (1H, t, J 7.9 Hz); 7.51 (2H, d, J 8.9 Hz); 7.50 (1H, s); 7.42 (1H, d, J 7.8 Hz); 6.15 (1H, bs); 5.75 (1H, bs); 4.73-4.61 (2H, m); 3.02 (3H, s); 2.36 (3H, s).
APCI-MS m/z: 508.2 [MH⁺].

### Example 14 6-Methyl-2-oxo-1-(3-trifluoromethyl-phenyl)-1,2-dihydro-pyridine-3,5-dicarboxylic acid 3-(4-methanesulfonyl-benzylamide) 5-methylamide

¹H NMR (CDCl₃): δ 9.90 (1H, t, J 5.9 Hz); 8.54 (1H, s); 7.90 (2H, d, J 8.1 Hz); 7.84 (1H, d, J 7.9 Hz); 7.77 (1H, t, J 7.8 Hz); 7.53 (1H, s); 7.52 (2H, d, J 8.3 Hz); 7.45 (1H, d, J 7.9 Hz); 4.74-4.62 (2H, m); 3.15 (3H, s); 3.07 (3H, s); 3.02 (3H, s); 2.11 (3H, s).
APCI-MS m/z: 522.3 [MH⁺].

### Example 15 6-Methyl-2-oxo-1-(3-trifluoromethyl-phenyl)-1,2-dihydro-pyridine-3,5-dicarboxylic acid 5-[(2-hydroxy-ethyl)-methyl-amide] 3-(4-methanesulfonyl-benzylamide) APCI-MS m/z: 566.2 [MH⁺]. Retention time 1.82 minutes.

### Example 16 6-Methyl-2-oxo-l-(3-trifluoromethyl-phenyl)-1,2-dihydro-pyridine-3,5-dicarboxylic acid 3-(4-methanesulfonyl-benzylamide) 5-(methyl-propyl-amide)

¹H NMR (CDCl₃): δ 9.94-9.84 (1H, m); 8.50 (1H, s); 7.88 (2H, d, J 8.6 Hz); 7.83 (1H, d, J 7.9 Hz); 7.76 (1H, t, J 7.9 Hz); 7.52 (1H, s); 7.52 (2H, d, J 8.1 Hz); 7.44 (1H, d, J 7.9 Hz); 4.74-4.62 (2H, m); 3.52 (1.1H, t, J 7.5 Hz, part of rotameric system, rotamer 1); 3.29 (0.9H, t, J 7.5 Hz, part of rotameric system, rotamer 2); 3.10 (1.4H, s, part of rotameric system, rotamer 2); 3.02 (1.6H, s, part of rotameric system, rotamer 1); 3.02 (3H, s); 2.08 (3H, s); 1.75-1.60 (2H, m); 0.99 (1.6H, t, J 7.5 Hz, part of rotameric system, rotamer 1); 0.88 (1.4H, t, J 7.5 Hz, part of rotameric system, rotamer 2).
APCI-MS m/z: 564.3 [MH⁺].

### Example 17 6-Methyl-2-oxo-5-(pyrrolidine-1-carbonyl)-1-(3-trifluoromethyl-phenyl)-1,2-dihydro-pyridine-3,5-dicarboxylic acid 3-(4-methanesulfonyl-benzylamide)

¹H NMR (CDCl₃): δ 9.87 (1H, t, J 6.0 Hz); 8.59 (1H, s); 7.88 (2H, d, J 8.2 Hz); 7.82 (1H, d, J 7.9 Hz); 7.75 (1H, t, J 7.9 Hz); 7.54-7.48 (3H, m); 7.44 (1H, d, J 7.6 Hz); 4.73-4.61 (2H, m); 3.66 (2H, t, J 6.7 Hz); 3.46-3.39 (2H, m); 3.02 (3H, s); 2.14 (3H, m); 2.06-1.95 (4H, m).
APCI-MS m/z: 562.5 [MH⁺].

### Example 18 6-Methyl-2-oxo-1-(3-trifluoromethyl-phenyl)-1,2-dihydro-pyridine-3,5-dicarboxylic acid 5-[(2-dimethylamino-ethyl)-methyl-amide] 3-(4-methanesulfonyl-benzylamide) trifluoroacetic acid salt

¹H NMR (DMSO-d₆): δ 9.88 (1H, t, J 6.1 Hz); 9.40 (1H, bs); 8.43 (1H, s); 8.00 (1H, s); 7.90 (1H, d, J 7.7 Hz); 7.87 (2H, d, J 8.2 Hz); 7.83-7.76 (2H, m); 7.53 (2H, d, J 8.2 Hz); 4.58 (2H, d, J 5.9 Hz); 4.05-3.70 (2H, m); 3.43-3.31 (2H, m); 3.16 (3H, s); 2.96 (3H, s); 2.89 (3H, s); 2.88 (3H, s); 1.92 (3H, s).
APCI-MS m/z: 593.3 [MH⁺].

### Example 19 5-((2R)-2-Hydroxymethyl-pyrrolidine-1-carbonyl)-6-methyl-2-oxo-1-(3-trifluoromethyl-phenyl)-1,2-dihydro-pyridine-3-carboxylic acid 3-(4-methanesulfonyl - benzylamide)

¹H NMR (CDCl₃): δ 9.88 (1H, t, J 6.1 Hz); 8.59 (1H, d, J 3.3 Hz); 7.89 (2H, d, J 8.8 Hz); 7.84 (1H, d, J 7.7 Hz); 7.77 (1H, t, J 7.7 Hz); 7.51 (2H, d, J 8.6 Hz); 7.51 (1H, s); 7.44 (1H, d, J 7.8 Hz); 4.70-4.65 (2H, m); 4.44-4.35 (1H, m); 3.91-3.84 (1H, m); 3.74 (1H, p, J 5.7 Hz); 3.55-3.44 (2H, m); 3.02 (3H, s); 2.27-2.17 (1H, m); 2.15 (3H, s); 2.12-1.83 (3H, m); 1.82-1.71 (1H, m).
APCI-MS m/z: 592.4 [MH⁺].

### Example 20 5-(3-Hydroxy-pyrrolidine-1-carbonyl)-6-methyl-2-oxo-1-(3-trifluoromethyl-phenyl)-1,2-dihydro-pyridine-3,5-dicarboxylic acid 3-(4-methanesulfonyl-benzylamide)

¹H NMR (CDCl₃): δ 9.96-9.89 (1H, m); 8.63-8.56 (1H, m); 7.88 (2H, d, J 8.5 Hz); 7.83 (1H, d, J 8.0 Hz); 7.76 (1H, t, J 7.6 Hz); 7.52 (1H, s); 7.51 (2H, d, J 8.0 Hz); 7.44 (1H, d, J 7.6 Hz); 4.67 (2H, d, J 5.7 Hz); 4.65-4.53 (1H, m); 3.92-3.38 (4H, m); 3.02 (3H, s); 2.19-2.01 (2H, m); 2.14 (3H, s).
APCI-MS m/z: 578.3 [MH⁺]. Retention time 1.95 minutes.

### Example 21 N³-[(1,1-Dioxido-2,3-dihydro-1-benzothien-5-yl)methyl]-N⁵,N⁵,6-trimethyl-2-oxo-1-[3-(trifluoromethyl)phenyl]-1,2-dihydropyridine-3,5-dicarboxamide

In a stainless-steel autoclave (100 ml) were placed N-[(1,1-dioxido-2,3-dihydro-1-benzothien-5-yl)methyl]-5-iodo-6-methyl-2-oxo-1-[3-(trifluoromethyl)phenyl]-1,2-dihydropyridine-3-carboxamide (66.3 mg, 0.11 mmol), palladium(II)acetate (14.0 mg, 62.4 µmol), triphenylphosphine (25.1 mg, 95.7 µmol), dimethylamine (1.2 g, 27.8 mmol) and ethanol (10 ml). The reaction vessel was cooled to -50 °C, degassed by vacuum/carbon monoxide combined treatment, pressurized at 4 atmospheres with carbon monoxide, and then heated at 100 °C for 6 h. After cooling, the solution was evaporated and the residue was purified by preparative HPLC to give the title compound as a white solid (11 mg, 18%).
¹H NMR (CDCl₃): δ 9.85 (1H, t, J 5.9 Hz); 8.53 (1H, s); 7.84 (1H, d, J 7.7 Hz); 7.77 (1H, t, J 7.9 Hz); 7.68 (1H, d, J 8.0 Hz); 7.52 (1H, s); 7.44 (2H, t, J 8.6 Hz); 7.35 (1H, s); 4.65 (2H, dd, J 5.8, 4.1 Hz); 3.49 (2H, t, J 6.8 Hz); 3.36 (2H, t, J 6.9 Hz); 3.15 (3H, s); 3.07 (3H, s); 2.11 (3H, s).
APCI-MS m/z: 548 [MH⁺].

### Example 22 5-(N¹-Acetyl-hydrazinocarbonyl)-6-methyl-2-oxo-1-(3-trifluoromethylphenyl)-1,2-dihydro-pyridine-3-carboxylic acid 4-methanesulfonyl-benzylamide

¹H NMR (DMSO-d₆): δ 10.26 (1H, s); 9.95 (1H, s); 9.79 (1H, t, J 6.0 Hz); 8.50 (1H, s); 7.93 (1H, s); 7.93-7.90 (1H, m); 7.87 (2H, d, J 8.4 Hz); 7.82 (1H, d, J 7.7 Hz); 7.74 (1H, d, J 8.0 Hz); 7.55 (2H, d, J 8.3 Hz); 4.59 (2H, d, J 6.2 Hz); 3.17 (3H, s); 2.18 (3H, s); 1.91 (3H, s).
APCI-MS m/z: 565.2 [MH⁺].

### Example 23 5-[N¹-(2-Cyano-acetyl)-hydrazinocarbonyl]-6-methyl-2-oxo-1-(3-trifluoromethyl-phenyl)-1,2-dihydro-pyridine-3-carboxylic acid 4-methanesulfonyl-benzylamide

¹H NMR (DMSO-d₆): δ 10.55 (1H, s); 10.42 (1H, s); 9.78 (1H, t, J 6.2 Hz); 8.51 (1H, s); 7.94 (1H, s); 7.92 (1H, d, J 8.2 Hz); 7.87 (2H, d, J 8.2 Hz); 7.82 (1H, d, J 7.6 Hz); 7.75 (1H, d, J 7.6 Hz); 7.55 (2H, d, J 8.2 Hz); 4.59 (2H, d, J 6.0 Hz); 3.82 (2H, s); 3.17 (3H, s); 2.18 (3H, s).
APCI-MS m/z: 590.1 [MH⁺].

### Example 24 5-{[2-(Aminocarbonothioyl)hydrazino]carbonyl}-6-methyl-N-[4-(methylsulfonyl) benzyl]-2-oxo-1-[3-(trifluoromethyl)phenyl]-1,2-dihydropyridine-3-carboxamide

¹H NMR (DMSO-d₆): δ 10.31 (1H, s); 9.80 (1H, t, J 6.2 Hz); 9.32 (1H, s); 8.70 (1H, s); 7.92 (1H, d, J 7.8 Hz); 7.91 (1H, bs); 7.90 (1H, bs); 7.86 (2H, d, J 8.3 Hz); 7.83 (1H, d, J 8.3); 7.76 (1H, bs); 7.70 (1H, d, J 7.6 Hz); 7.54 (2H, d, J 8.1 Hz); 4.59 (2H, d, J 6.0 Hz); 3.17 (3H, s); 2.14 (3H, s).
APCI-MS m/z: 582.1 [MH⁺].

### Example 25 5-Hydrazinocarbonyl-6-methyl-2-oxo-1-(3-trifluoromethyl-phenyl)-1,2-dihydro-pyridine-3-carboxylic acid 4-methanesulfonyl-benzylamide

APCI-MS m/z: 523.2 [MH⁺]. Retention time 1.72 minutes.

### Example 26 5-({[2-[(Ethylamino)carbonyl]hydrazino}carbonyl)-6-methyl-N-[4-(methylsulfonyl) benzyl]-2-oxo-1-[3-(trifluoromethyl)phenyl]-1,2-dihydropyridine-3-carboxamide

To 5-hydrazinocarbonyl-6-methyl-2-oxo-1-(3-trifluoromethyl-phenyl)-1,2-dihydropyridine-3-carboxylic acid 4-methanesulfonyl-benzylamide (0.030 g, 0.057 mmol) in 1,4-dioxan (10 ml) was added ethylisocyanate (0.016 g, 0.23 mmol), and the mixture was stirred at room temperature for 1 h. The mixture was evaporated and the residue was purified on preparative HPLC giving 0.015 g (44%) of the title compound.
¹H NMR (CDCl₃): δ 9.96-9.87 (1H, m); 8.82 (1H, s); 7.88 (1H, d, J 8.2 Hz); 7.84 (2H, d, J 7.9 Hz); 7.83-7.80 (1H, m); 7.77 (1H, t, J 7.9 Hz); 7.52 (1H, s); 7.47 (2H, d, J 8.2 Hz); 7.47-7.41 (1H, m); 4.70-4.55 (2H, m); 3.23 (2H, q, J 6.9 Hz); 3.01 (3H, s); 2.31 (3H, s); 1.11 (3H, t, J 7.1 Hz).
APCI-MS m/z: 594.2 [MH⁺].

### Example 27 5({2-[(N,N-Dimethylamino)carbonyl]hydrazino}carbonyl)-6-methyl-N-[4 (methylsulfonyl) benzyl]-2-oxo-1-[3-(trifluoromethyl)phenyl]-1,2-dihydropyridine-3-carboxamide

To 5-hydrazinocarbonyl-6-methyl-2-oxo-1-(3-trifluoromethyl-phenyl)-1,2-dihydropyridine-3-carboxylic acid 4-methanesulfonyl-benzylamide (0.030 g, 0.057 mmol) in THF (10 ml) was added N,N-dimethylcarbamoyl chloride (0.0247 g, 0.23 mmol), and the mixture was stirred at 50°C for 3 h. The mixture was evaporated and the residue was purified on preparative HPLC giving 0.020 g (60%) of the title compound.
¹H NMR (DMSO-d₆): δ 9.92 (1H, bs); 9.80 (1H, t, J 6.2 Hz); 8.50 (1H, s); 8.48 (1H, s); 7.94-7.89 (2H, m); 7.87 (2H, d, J 8.5 Hz); 7.82 (1H, d, J 8.2 Hz); 7.73 (1H, d, J 7.8 Hz); 7.55 (2H, d, J 8.5 Hz); 4.59 (2H, d, J 6.0 Hz); 3.17 (3H, s); 2.85 (6H, s); 2.19 (3H, s).
APCI-MS m/z: 594.1 [MH⁺].

### Example 28 5-(3,3-Dimethyl-ureido)-6-methyl-2-oxo-1-(3-trifluoromethyl-phenyl)-1,2-dihydro-pyridine-3-carboxylic acid 4-methanesulfonyl-benzylamide

### a) 5-(4-Methanesulfonyl-benzylcarbamoyl)-2-methyl-6-oxo-1-(3-trifluoromethyl-phenyl) 1,6-dihydro-pyridine-3-carbonyl azide

To 5-(4-methanesulfonyl-benzylcarbamoyl)-2-methyl-6-oxo-1-(3-trifluoromethyl-phenyl)-1,6-dihydro-pyridine-3-carboxylic acid (0.055 g, 0.108 mmol) in CH₂Cl₂ (10 ml), triethylamine (0.020 g, 0.2 mmol) and diphenylphosphoryl azide (0.055 g, 0.2 mmol) were added, the flask was sealed, and the contents were stirred at room temperature overnight. The volatiles were removed *in vacuo* and the residue was purified on silica (heptane:EtOAc 2:1 to 1:1 to 1:2) giving 0.012 g (21 %) of the title compound.
¹H NMR (CDCl₃): δ 9.58 (1H, t, J 5.8 Hz); 9.13 (1H, s); 7.88 (2H, d, J 8.2 Hz); 7.86-7.82 (1H, m); 7.77 (1H, t, J 7.9 Hz); 7.51 (2H, d, J 8.7 Hz,); 7.49 (1H, s); 7.41 (1H, d, J 7.9 Hz); 4.73-4.61 (2H, m); 3.02 (3H, s); 2.53 (3H, s).
APCI-MS m/z: decomposes.

### b) 5-(3,3-Dimethyl-ureido)-6-methyl-2-oxo-1-(3-trifluoromethyl-phenyl)-1,2-dihydropyridine-3-carboxylic acid 4-methanesulfonyl-benzylamide

A mixture of 5-(4-methanesulfonyl-benzylcarbamoyl)-2-methyl-6-oxo-1-(3-trifluoromethyl-phenyl)-1,6-dihydro-pyridine-3-carbonyl azide (0.005 g, 0.0094 mmol) and toluene (5 ml) was heated (120 °C) with stirring for 1 h, and then allowed to cool. The toluene was removed in vacuo and the residue was dissolved in acetonitrile (5 ml), dimethylamine (0.5 ml, 40% in water) was added quickly, and the resulting mixture was stirred for 5 minutes. Evaporation of the volatiles and purification by preparative HPLC gave 0.005 g (97%) of the title compound.
¹H NMR (CDCl₃): δ 9.98 (1H, t, J 5.4 Hz); 8.47 (1H, s); 7.87 (2H, d, J 8.6 Hz); 7.80 (1H, d, J 7.7 Hz); 7.73 (1H, t, J 8.0 Hz); 7.53 (1H, s); 7.50 (2H, d, J 8.3 Hz); 7.46 (1H, d, J 8.0 Hz); 6.08 (1H, bs); 4.73-4.60 (2H, m); 3.05 (6H, s); 3.02 (3H, s); 2.02 (3H, s).
APCI-MS m/z: 551.2 [MH⁺].

Following the general method of Example 28, the compounds of Examples 29 and 30 were prepared:

### Example 29 6-Methyl-5-(3-methyl-ureido)-2-oxo-1-(3-trifluoromethyl-phenyl)-1,2-dihydro-pyridine-3-carboxylic acid 4-methanesulfonylamide

¹H NMR (CDCl₃): δ 10.13 (1H, t, J 6.1 Hz); 8.45 (1H, s); 7.87 (2H, d, J 8.1 Hz); 7.81 (1H, d, J 7.7 Hz); 7.73 (1H, t, J 7.7 Hz); 7.52 (1H, s); 7.49 (2H, d, J 8.2 Hz); 7.45 (1H, d, J 7.4 Hz); 6.82 (1H, bs); 4.71-4.58 (2H, m); 3.01 (3H, s); 2.80 (3H, s); 2.06 (3H, s).
APCI-MS m/z: 537.1 [MH⁺].

### Example 30 6-Methyl-2-oxo-1-(3-trifluoromethyl-phenyl)-5-ureido-1,2-dihydropyridine-3-carboxylic acid 4-methanesulfonyl-benzylamide

¹H NMR (DMSO-d₆): δ 9.96 (1H, t, J 6.2 Hz); 8.34 (1H, s); 7.90-7.85 (2H, m); 7.85 (2H, d, J 8.5 Hz); 7.84-7.79 (2H, m); 7.69 (1H, d, J 7.8 Hz); 7.52 (2H, d, J 8.2 Hz); 5.94 (2H,s); 4.58 (2H, d, J 6.2 Hz); 3.15 (3H, s); 1.89 (3H, s).
APCI-MS m/z: 523.1 [MH⁺].

### Example 31 5-Amino-6-methyl-2-oxo-1-(3-trifluoromethyl-phenyl)-1,2-dihydropyridine-3-carboxylic acid 4-methanesulfonyl-benzylamide

To a solution of 5-(4-methanesulfonyl-benzylcarbamoyl)-2-methyl-6-oxo-1-(3-trifluoromethyl-phenyl)-1,6-dihydro-pyridine-3-carboxylic acid (0.055 g, 0.108 mmol) in *tert*-butanol (15 ml), triethylamine (0.020 g, 0.2 mmol) and diphenylphosphoryl azide (0.055 g, 0.2 mmol) were added, the flask was equipped with a reflux condenser, and the mixture was heated with stirring at 100 °C overnight. LC-MS showed a complex mixture of products, where one was identified by mass to be the title compound. The compound was isolated by preparative HPLC, and pure fractions were freeze-dried, to give 0.006 g (12%) of an yellow solid.
¹H NMR (DMSO-d₆): δ 10.28 (1H, t, J 6.1 Hz); 8.22 (1H, s); 7.86 (2H, d, J 8.2 Hz); 7.85 (1H, s); 7.80 (1H, s); 7.78 (1H, d, J 8.0 Hz); 7.64 (1H, d, J 7.7 Hz); 7.53 (2H, d, J 8.4 Hz); 4.64 (2H, s); 4.57 (2H, d, J 6.2 Hz); 3.17 (3H, s); 1.84 (3H, s).
APCI-MS m/z: 480.1 [MH⁺].

### Example 32 6-Methyl-N-[4-(methylsulfonyl)benzyl]-2-oxo-5-propionyl-1-[3-(trifluoromethyl)phenyl]-1,2-dihydropyridine-3-carboxamide

A solution of 5-iodo-6-methyl-*N*-[4-(methylsulfonyl)benzyl]-2-oxo-1-[3-(trifluoromethyl)phenyl]-1,2-dihydropyridine-3-carboxamide (1500 mg, 2.5 mmol), bis[1,2-bis(diphenylphosphino)ethane]palladium (230 mg, 0.25 mmol), triethylamine (7.5 ml, 54 mmol) and ethylpropenyl ether (900 µl, 7.5 mmol) in DMF (45 ml) were heated at 100 °C overnight. After cooling the reaction mixture was poured into water and extracted with ethyl acetate. The extracts were separated and evaporated under reduced pressure. The crude product was dissolved in DMF (25 ml) and 2M HCl (25 ml) and then stirred for 1.5 h. The reaction mixture was then poured into aqueous NaHCO₃ and extracted with ethyl acetate The extracts were evaporated under reduced pressure and the residue was chromatographed on silica using ethyl acetate/heptane (2/1, 4/1, 10/1) as eluent. Fractions containing the product were combined and evaporated to give 1.3 g (>99%) of the title compound.
¹H NMR (CDCl₃): δ 9.76 (1H, t); 9.06 (1H, s); 7.89 (2H, d); 7.84 (1H, d); 7.76 (1H, t); 7.52 (2H, d); 7.49 (1H, s); 7.40 (1H, d); 4.68 (2H, m); 3.02 (3H, s); 3.00 (2H, q); 2.39 (3H, s); 1.22 (3H, t).

### Example 33 5-Formyl-6-methyl-N-[4-(methylsulfonyl)benzyl]-2-oxo-1-[3-(trifluoromethyl)phenyl]-1,2-dihydropyridine-3-carboxamide

The title compound was prepared using a procedure analogous to that described for Example 44.
¹H NMR (CDCl₃): δ 10.06 (1H, s); 9.57 (1H, t, J 5.9 Hz); 9.06 (1H, s); 7.89 (2H, d, J 8.2 Hz); 7.86 (1H, s); 7.79 (1H, t, J 7.9 Hz); 7.52 (3H, d, J 8.2 Hz); 7.43 (1H, d, J 8.2 Hz); 4.69 (2H, m); 3.04 (3H, s); 2.52 (3H, s).
APCI-MS m/z: 493.2 [MH⁺].

### Example 34 6-Methyl-N-[4-(methylsulfonyl)benzyl]-2-oxo-5-(3-oxobutyl)-1-[3-(trifluoromethyl)phenyl]-1,2-dihydropyridine-3-carboxamide

In a Schlenk vessel equipped with a magnetic stirring bar were placed 5-iodo-6-methyl-N - [4-(methylsulfonyl)benzyl]-2-oxo-1-[3-(trifluoromethyl)phenyl]-1,2-dihydropyridine-3-carboxamide (564.3 mg, 0.96 mmol), bis[1.2-bis(diphenylphosphino)ethane]-palladium (0) (19.9 mg, 0.02 mmol), 3-buten-2-ol (499 mg, 6.9 mmol), triethylamine (1.2 ml, 8.6 mmol) and DMF (6 ml). The vessel was purged with argon, sealed and heated at 105 °C overnight. The reaction mixture was cooled and partitioned between ethyl acetate and water. The organic layer was washed with water, brine, dried over sodium sulfate, filtered and concentrated in vacuo. The residue was purified by preparative HPLC to give the title compound as a white solid (255 mg, 50 %).
¹H NMR (CDCl₃): δ 10.05 (1H, t, J 5.8 Hz); 8.49 (1H, s); 7.87 (2H, d, J 8.4 Hz); 7.80 (1H, d, J 7.9 Hz); 7.73 (1H, t, J 7.9 Hz); 7.51 (2H, d, J 8.4 Hz); 7.48 (1H, s); 7.41 (1H, d, J 7.9 Hz); 4.69 (2H, m); 3.01 (3H, s); 2.81 (4H, s); 2.20 (3H, s); 2.10 (3H, s).
APCI-MS m/z: 535.1 [MH⁺].

### Example 35 5-Acetyl-N-[4-(isopropylsulfonyl)benzyl]-6-methyl-2-oxo-1-[3-(trifluoromethyl)phenyl]-1,2-dihydropyridine-3-carboxamide

### a) N-[4-(Isopropylsulfonyl)benzyl]-6-methyl-2-oxo-1-[3-(trifluoromethyl)phenyl]-1,2-dihydropyridine-3-carboxamide

To a mixture of 6-methyl-2-oxo-1-[3-(trifluoromethyl)phenyl]-1,2-dihydropyridine-3-carboxylic acid (16.27 g, 54.5 mmol) in DCM was added thionyl chloride (12 ml, 165 mmol) under argon. The colour of the reaction mixture turned black. After 50 minutes stirring at ambient temperature, the solvent was removed by evaporation. The last traces of thionyl chloride were removed by azeotropic evaporation with toluene. To an ice cooled solution of the residue in DCM, was added dropwise a mixture of 1-[4-(isopropylsulfonyl)phenyl] methanamine (11.8 g, 55.4 mmol) and triethylamine (30 ml, 215 mmol) in DCM under vigorous stirring. After the addition, the dark suspension was allowed to warm up to room temperature. After 30 minutes stirring at ambient temperature the reaction mixture was concentrated in vacuo and the residue was partitioned between ethyl acetate and water. The organic layer was washed with water, dried over sodium sulfate, filtered and concentrated in vacuo, giving a dark oil which crystallized on standing. The solid was triturated with ethyl acetate, filtered, washed with ethyl acetate, ether, heptane, and dried under vacuo to give the title compound as a light yellow powder (15.3 g). The filtrates were collected, concentrated and further purified by flash chromatography on silica, eluting with a gradient of tert-butyl methyl ether to 5% methanol in tert-butyl methyl ether to provide an additional 8.78 g of the crude product. The solids were combined to give (24.1 g, 89 %) of the title compound.
¹H NMR (CDCl₃): δ 9.96 (1H, t, *J* 5.5 Hz); 8.57 (1H, d, *J* 7.4 Hz); 7.78 (3H, t, *J* 4.1 Hz); 7.72 (1H, t, *J* 7.9 Hz); 7.52 - 7.45 (3H, m); 7.43 (1H, d, *J* 7.7 Hz); 6.46 (1H, d, *J* 7.6 Hz); 4.67 (2H, ddd, *J* 22.0 15.7 6.2 Hz); 3.13 (1H, septet, *J* 9.8 Hz); 2.07 (3H, s); 1.26 (6H, d, *J* 6.9 Hz).
APCI-MS m/z: 493.2 [MH⁺].

### b) 5-Iodo-N-[4-(isopropylsulfonyl)benzyl]-6-methyl-2-oxo-1-[3-(trifluoromethyl)phenyl]-1,2-dihydropyridine-3-carboxamide

To a stirred solution of N-[4-(isopropylsulfonyl)benzyl]-6-methyl-2-oxo-1-[3-(trifluoromethyl)phenyl]-1,2-dihydropyridine-3-carboxamide (23.98 g, 48.73 mmol) and TFA (90 ml) in DCM (90 ml) was added N-iodosuccinimide (11.03 g, 49.14 mmol). After 2 h the reaction was complete and the solvent was removed by evaporation. To the residue was added ethyl acetate (100 ml) and saturated aqueous sodium hydrogencarbonate solution (60 ml) under stirring. The yellow solid was collected by suction filtration, washed with water, air dried for 30 min, washed again with diethyl ether, heptane and vacuum dried to give the title compound as a light yellow powder (29.67 g, 98 %).
¹H NMR (CDCl₃): δ 9.83 (1H, t, J 6.0 Hz); 8.90 (1H, s); 7.83 - 7.76 (3H, m); 7.73 (1H, t, J 7.8 Hz); 7.47 (3H, d, J 8.0 Hz); 7.39 (1H, d, J 7.7 Hz); 4.66 (2H, ddd, J 22.3, 15.8 and 6.3 Hz); 3.13 (1H, septet, J 9.0 Hz); 2.29 (3H, s); 1.26 (6H, d, J 6.9 Hz).
APCI-MS m/z: 619.1 [MH⁺].

### c) 5-Acetyl-N-[4-(isopropylsulfonyl)benzyl]-6-methyl-2-oxo-1-[3-(trifluoromethyl)phenyl]-1,2-dihydropyridine-3-carboxamide

A mixture of 5-iodo-N-[4-(isopropylsulfonyl)benzyl]-6-methyl-2-oxo-1-[3-(trifluoromethyl)phenyl]-1,2-dihydropyridine-3-carboxamide (3.55 g, 5.7 mmol), bis[1.2-bis(diphenylphosphino)ethane]-palladium (0) (24.5 mg, 0.03 mmol), n-butyl vinyl ether (1.16 g, 11.6 mmol), triethylamine (4 ml, 28.7 mmol) in DMF (14 ml) was stirred at 100 °C under argon for 19 h. The reaction mixture was cooled and concentrated in vacuo. The residue was dissolved in methanol (20 ml) and 2M hydrochloric acid (2 ml) was added. After 1 h stirring at room temperature the mixture was partitioned between ethyl acetate/water and basified with saturated aqueous sodium bicarbonate solution. The water layer was extracted with ethyl acetate (2 x 50 ml) and DCM (1 x 30 ml). The combined organic layers were washed with water, brine, dried over sodium sulfate, filtered and concentrated in vacuo. The residue was purified by flash chromatography on silica eluting with tert-butyl methyl ether/methanol (10:0.2) to give the title compound as a light yellow solid (2.5 g, 82%).
¹H NMR (CDCl₃): δ 9.71 (1H, t, J 5.7 Hz); 9.05 (1H, s); 7.85 - 7.78 (3H, m); 7.75 (1H, t, J 7.9 Hz); 7.51 - 7.44 (3H, m); 7.39 (1H, d, J 8.2 Hz); 4.68 (2H, ddd, J 22.4, 15.8 and 6.2 Hz); 3.14 (1H, septet, J 7.7 Hz); 2.63 (3H, s); 2.40 (3H, s); 1.26 (6H, d, J 6.9 Hz).
APCI-MS m/z: 535.2 [MH⁺].

The compounds of Examples 36 to 38 were prepared using a procedure analogous to that described for Example 4.

### Example 36 5-Acetyl-1-(3-cyano-phenyl)-6-methyl-2-oxo-1,2-dihydro-pyridine-3-carboxylic acid 4-methanesulfonyl-benzylamide

¹H NMR (CDCl₃): δ 9.67 (1H, t, J5.7 Hz); 9.07 (1H, s); 7.90-7.86 (3H, m); 7.76 (1H, t, J=7.9 Hz); 7.53-7.45 (4H, m); 4.74-4.64 (2H, m); 3.02 (3H, s); 2.65 (3H, s); 2.42 (3H, s).
APCI-MS m/z: 464 [MH⁺].

### Example 37 5-Acetyl-1-(3-chloro-phenyl)-6-methyl-2-oxo-1,2-dihydro-pyridine-3-carboxylic acid 4-methanesulfonyl-benzylamide

¹H NMR (CDCl₃): δ 9.77 (1H, t, J 5.8 Hz); 9.04 (1H, s); 7.89 (2H, d, *J* 8.3 Hz); 7.56-7.51 (4H, m); 7.22 (1H, s); 7.10-7.08 (1H, m); 4.69 (2H, d, *J* 6.0 Hz); 3.02 (3H, s); 2.64 (3H, s); 2.44 (3H, s).
APCI-MS m/z: 473 [MH⁺].

### Example 38 5-Acetyl-6-methyl-2-oxo-1-m-tolyl-1,2-dihydro-pyridine-3-carboxylic acid 4-methanesulfonyl-benzylamide

¹H NMR (CDCl₃): δ 9.89 (1H, t, *J* 5.5 Hz); 9.04 (1H, s); 7.88 (2H, d, *J* 8.3 Hz); 7.53-7.47 (3H, m); 7.37-7.35 (1H, m); 6.98-6.96 (2H, m); 4.68 (2H, d, *J* 6.0 Hz); 3.02 (3H, s); 2.64 (3H, s); 2.44 (3H, s), 2.43 (3H, s).
APCI-MS m/z: 453 [MH⁺].

### Example 39 5-(1-Hydroxyethyl)-6-methyl-N-[4-(methylsulfonyl)benzyl]-2-oxo-1-[3-(trifluoromethyl)phenyl]-1,2-dihydropyridine-3-carboxamide

A mixture of 5-acetyl-6-methyl-*N*-[4-(methylsulfonyl)benzyl]-2-oxo-1-[3-(trifluoromethyl)phenyl]-1,2-dihydropyridine-3-carboxamide (Example 4, 180 mg, 0.35 mmol) and aluminum tri-sec-butoxide (0.2 mg, 0.79 mmol) in anhydrous isopropanol (30 ml) was stirred at 85 °C under a nitrogen atmosphere for 48 h. The reaction mixture was cooled to room temperature, water (0.2 ml) was added and the mixture was then concentrated in vacuo. The residue was purified by preparative HPLC to give the title compound as a white solid (134 mg, 74 %).
¹H NMR (CDCl₃): δ 10.01 (1H, t, J 5.7 Hz); 8.84 (1H, d, J 1.9 Hz); 7.87 (2H, d, J 8.3 Hz); 7.81 (1H, d, J 7.8 Hz); 7.74 (1H, t, J 8.0 Hz); 7.52 (2H, d, J 8.3 Hz); 7.50 (1H, s); 7.42 (1H, d, J 7.9 Hz); 5.03 (1H, dd, J 10.9 1.6 Hz); 4.67 (2H, q, J 6.3 Hz); 3.02 (3H, s); 2.12 (3H, s); 1.91 (1H, t, J 3.9 Hz); 1.58 (3H, dd, J 6.4 2.6 Hz).
APCI-MS m/z: 509.2 [MH⁺].

### Example 40 5-(1-Azidoethyl)-6-methyl-N-[4-(methylsulfonyl)benzyl]-2-oxo-1-[3-(trifluoromethyl)phenyl]-1,2-dihydropyridine-3-carboxamide

To a solution of 5-(1-hydroxyethyl)-6-methyl-N-[4-(methylsulfonyl)benzyl]-2-oxo-1-[3-(trifluoromethyl)phenyl]-1,2-dihydropyridine-3-carboxamide (29.1 mg, 0.06 mmol) in DCM (1.5 ml) was added thionylchloride (0.15 ml, 2.1 mmol) under an argon atmosphere. After 40 minutes stirring at ambient temperature, the solvent was removed in vacuo. The last traces of thionyl chloride were removed by azeotropic evaporation with toluene. The residue was dissolved in anhydrous DMF (1 ml) and sodium azide (20 mg, 0.3 mmol) was added. After 1 h stirring at room temperature the reaction mixture was diluted with water and purified by preparative HPLC giving the title compound as a white solid (11.5 mg, 37 %).
¹H NMR (CDCl₃): δ 9.94 (1H, t, J 5.9 Hz); 8.73 (1H, d, J 2.0 Hz); 7.88 (2H, dt, J 8.4 1.9 Hz); 7.83 (1H, d, J 8.1 Hz); 7.76 (1H, td, J 7.9 3.5 Hz); 7.55 - 7.48 (3H, m); 7.43 (1H, t, J 14.1 Hz); 4.81 - 4.59 (3H, m); 3.02 (3H, s); 2.13 (3H, s); 1.63 (3H, dd, J 6.9 1.6 Hz).
APCI-MS m/z: 534.2 [MH⁺].

### Example 41 6-Methyl-N-[4-(methylsulfonyl)benzyl]-5-(1-morpholin-4-ylethyl)-2-oxo-1-[3-(trifluoromethyl)phenyl]-1,2-dihydropyridine-3-carboxamide

To a solution of 5-(1-hydroxyethyl)-6-methyl-N-[4-(methylsulfonyl)benzyl]-2-oxo-1-[3-(trifluoromethyl)phenyl]-1,2-dihydropyridine-3-carboxamide (15 mg, 0.03 mmol) in DCM (2 ml) was added thionyl chloride (0.5 ml, 6.9 mmol) under an argon atmosphere. After 1 h stirring at ambient temperature, the solvent was removed in vacuo. The last traces of thionyl chloride were removed by azeotropic evaporation with toluene. The residue was dissolved in anhydrous DMF (1 ml) and morpholine (0.3 ml, 3.4 mmol) was added. After 35 minutes stirring at room temperature the reaction mixture was diluted with water and further purified by preparative HPLC giving the title compound as a white solid (4.3 mg, 25 %).
¹H NMR (CDCl₃): δ 10.05 (1H, t, J 5.9 Hz); 8.78 (1H, d, J 1.0 Hz); 7.87 (2H, d, J 8.3 Hz); 7.81 (1H, d, J 7.9 Hz); 7.74 (1H, t, J 8.0 Hz); 7.53 (2H, d, J 8.1 Hz); 7.50 (1H, s); 7.43 (1H, t, J 7.3 Hz); 4.67 (2H, td, J 24.2 5.9 Hz); 3.70 (4H, dd, J 20.6 11.4 Hz); 3.48 (1H, m); 3.02 (3H, s); 2.53 (2H, s); 2.43 (2H, m); 2.12 (3H, s); 1.36 (3H, dd, J 6.6 1.3 Hz).
APCI-MS m/z: 578.3 [MH⁺].

### Example 42 5-(1-Hydroxypropyl)-6-methyl-N-[4-(methylsulfonyl)benzyl]-2-oxo-1-[3-(trifluoromethyl)phenyl]-1,2-dihydropyridine-3-carboxamide

The title compound was prepared using a procedure analogous to that described for Example 39.
¹H NMR (CDCl₃): δ 10.01 (1H, t, J 5.9 Hz); 8.79 (1H, d, J 1.7 Hz); 7.87 (2H, d, J 8.3 Hz); 7.81 (2H, d, J 7.9 Hz); 7.74 (2H, t, J 7.8 Hz); 7.52 (2H, d, J 8.3 Hz); 7.49 (1H, s, ); 7.42 (1H, d, J = 7.5 Hz); 4.77 - 4.59 (3H, m); 3.01 (3H, s); 2.10 (3H, s); 2.00 (1H, t, J = 4.5 Hz); 1.98 - 1.74 (2H, m); 1.01 (3H, td, J 7.4 2.5 Hz).
APCI-MS m/z: 523.2 [MH⁺].

### Example 43 5-(1-Hydroxyethyl)-N-[4-(isopropylsulfonyl)benzyl]-6-methyl-2-oxo-1-[3-(trifluoromethyl)phenyl]-1,2-dihydropyridine-3-carboxamide

The title compound was prepared using a procedure analogous to that described for Example 39.
¹H NMR (CDCl₃): δ 10.02 (1H, t, J 5.9 Hz); 8.85 (1H, d, J 1.7 Hz); 7.81 (3H, d, J 30.8 Hz); 7.74 (1H, t, J 7.9 Hz); 7.50 (3H, d, J 19.2 Hz); 7.43 (1H, d, J 7.6 Hz); 5.03 (1H, t, J 5.4 Hz); 4.69 (2H, dd, J 29.1 9.7 Hz); 3.15 (1H, quintet, J 6.8 Hz); 2.12 (3H, s); 1.98 (1H, t, J 4.4 Hz); 1.58 (3H, dd, J 6.4 2.2 Hz); 1.28 (6H, d, J 6.9 Hz).
APCI-MS m/z: 578.3 [MH⁺].

### Example 44 N-[4-(Cyclopropylsulfonyl)benzyl]-5-formyl-6-methyl-2-oxo-1-[3-(trifluoromethyl)phenyl]-1,2-dihydropyridine-3-carboxamide

N-[4-(Cyclopropylsulfonyl)benzyl]-5-iodo-6-methyl-2-oxo-1-[3-(trifluoromethyl)phenyl]-1,2-dihydropyridine-3-carboxamide [2.6 g, 4.2 mmol; prepared by a procedure analogous to that described for Example 35 (b)], tris(dibenzylideneacetone) dipalladium (0) (81.9 mg, 0.09 mmol), triphenylphosphine (263.8 mg, 1.0 mmol) and toluene (28 ml) were added to a 100 ml single-neck round bottomed flask. The flask was fitted with a 30 ml dropping funnel with pressure-equalizer containing tributyltin hydride (2.3 g, 7.8 mmol) and toluene (20ml). The system was thoroughly flushed with carbon monoxide by repeated evacuation and filling and was then pressurized at 2.5 atmospheres carbon monoxide and heated at 95 °C with vigorous stirring. The tributyltin hydride solution was added dropwise over a period of 3 h. After the addition was complete, the reaction mixture was allowed to reach room temperature and then the carbon monoxide was flushed out with argon. The solvent was removed in vacuo and the residue was purified by flash chromatography on silica, eluting with tert-butyl methyl ether/methanol (10:0.2) to give the title compound as a white solid (1.6 g, 73 %).
¹H NMR (CDCl₃): δ 10.06 (1H, s); 9.56 (1H, t, J 5.8 Hz); 9.06 (1H, s); 7.89 - 7.78 (4H, m); 7.54 - 7.47 (3H, m); 7.44 (1H, d, J 7.8 Hz); 4.68 (2H, ddd, J 21.5 15.4 6.2 Hz); 2.51 (3H, s); 2.42 (1H, m); 1.33 (2H, m); 1.01 (2H, m).
APCI-MS m/z: 519.2 [MH⁺].

### Example 45 5-[(E)-(Methoxyimino)methyl]-6-methyl-N-[4-(methylsulfonyl)benzyl]-2-oxo-1-[3-(trifluoromethyl)phenyl]-1,2-dihydropyridine-3-carboxamide

A mixture of 5-formyl-6-methyl-N-[4-(methylsulfonyl)benzyl]-2-oxo-1-[3-(trifluoromethyl)phenyl]-1,2-dihydropyridine-3-carboxamide (Example 33,14.7 mg, 0.03 mmol), O-methylhydroxylamine hydrochloride (17.2 mg, 0.21 mmol), potassium acetate (42.7 mg, 0.44 mmol) and water (0.1 ml) in ethanol (1.5 ml) was heated at 60 °C for 2 h. The reaction mixture was cooled, concentrated in vacuo and the residue was purified by preparative HPLC to give the title compound as a white solid (6.2 mg, 39 %).
¹H NMR (DMSO-d₆): δ 9.80 (1H, t, J 6.0 Hz); 8.76 (1H, s); 8.44 (1H, s); 7.95 - 7.79 (5H, m); 7.74 (1H, d, J 8.0 Hz); 7.54 (2H, d, J 8.3 Hz); 4.58 (2H, d, J 6.1 Hz); 3.90 (3H, s); 3.17 (3H, s); 2.10 (3H, s).
APCI-MS m/z: 522.2 [MH⁺].

### Example 46 5-(Hydroxymethyl)-6-methyl-N-[4-(methylsulfonyl)benzyl]-2-oxo-1-[3-(trifluoromethyl)phenyl]-1,2-dihydropyridine-3-carboxamide

The title compound was prepared using a procedure analogous to that described for Example 39.
¹H NMR (CDCl₃): δ 9.98 (1H, t, J 28.4 Hz); 8.66 (1H, s); 7.87 (2H, d, J 8.3 Hz); 7.82 (1H, d, J 8.0 Hz); 7.75 (1H, t, J 7.9 Hz); 7.51 (3H, d, J 8.4 Hz); 7.43 (1H, d, J 8.1 Hz); 4.67 (4H, m); 3.02 (3H, s); 2.16 (3H, s); 2.01 (1H, t, J 5.3 Hz).
APCI-MS m/z: 495.1 [MH⁺].

### Example 47 5-[(Dimethylamino)methyl]-6-methyl-N-[4-(methylsulfonyl)benzyl]-2-oxo-1-[3-(trifluoromethyl)phenyl]-1,2-dihydropyridine-3-carboxamide

### a) 5-(Chloromethyl)-6-methyl-N-[4-(methylsulfonyl)benzyl]-2-oxo-1-[3-(trifluoromethyl)phenyl]-1,2-dihydropyridine-3-carboxamide

5-(Hydroxymethyl)-6-methyl-N-[4-(methylsulfonyl)benzyl]-2-oxo-1-[3-(trifluoromethyl)phenyl]-1,2-dihydropyridine-3-carboxamide (Example 46, 990 mg, 2.0 mmol) was treated with thionyl chloride (476 mg, 4.0 mmol) to give the title compound (1.0 g, 97%).
APCI-MS m/z: 513.2 [MH⁺].

### b) 5-[(Dimethylamino)methyl]-6-methyl-N-[4-(methylsulfonyl)benzyl]-2-oxo-1-[3-(trifluoromethyl)phenyl]-1,2-dihydropyridine-3-carboxamide

A solution of 5-(chloromethyl)-6-methyl-*N*-[4-(methylsulfonyl)benzyl]-2-oxo-1-[3-(trifluoromethyl)phenyl]-1,2-dihydropyridine-3-carboxamide (5 mg, 0.097 mmol) and dimethylamine in ethanol (0.5 ml, 33 %) was heated in a microwave for 10 minutes at 50 °C. The mixture was then purified by preparative HPLC to give the title compound as a white solid (4 mg, 79 %).
¹H NMR (DMSO-d₆): δ 9.96 (1H, t, J 6.0 Hz); 8.34 (1H, s); 7.90 - 7.85 (4H, m); 7.80 (1H, t, J 7.8 Hz); 7.70 (1H, d, J 8.0 Hz); 7.53 (2H, d, J 8.3 Hz); 4.58 (2H, d, J 6.2 Hz); 3.57 (2H, s); 3.17 (3H, s,); 2.18 (6H, s ); 2.02 (3H, s).
APCI-MS m/z: 522.1 [MH⁺].

### Example 48 6-Methyl-5-[(methylamino)methyl]-N-[4-(methylsulfonyl)benzyl]-2-oxo-1-[3-(trifluoromethyl)phenyl]-1,2-dihydropyridine-3-carboxamide

The title compound was prepared using a procedure analogous to that described for Example 47.
¹H NMR (DMSO-d₆) δ 9.97 (1H, t, J 6.0 Hz); 8.43 (1H, s); 7.94 - 7.85 (4H, m); 7.81 (1H, t, J 7.8 Hz); 7.67 (1H, d, J 7.9 Hz); 7.53 (2H, d, J 8.2 Hz); 4.58 (2H, d, J 6.0 Hz); 3.57 (2H, s); 3.17 (3H, s); 2.30 (3H, s); 2.02 (3H, s).
APCI-MS m/z: 508.1 [MH⁺].

### Example 49 6-Methyl-N-[4-(methylsulfonyl)benzyl]-5-(morpholin-4-ylmethyl)-2-oxo-1-[3-(trifluoromethyl)phenyl]-1,2-dihydropyridine-3-carboxamide

A solution of 5-(chloromethyl)-6-methyl-*N*-[4-(methylsulfonyl)benzyl]-2-oxo-1-[3-(trifluoromethyl)phenyl]-1,2-dihydropyridine-3-carboxamide (Example 47, 8 mg, 0.016 mmol ) and morpholine (5.5 mg, 0.062 mmol) in DMF (1 ml) was heated in a microwave oven for 10 minutes at 50°C. The mixture was then purified by preparative HPLC to give the title compound as a white solid (4 mg, 45 %).
¹H NMR (CDCl₃): δ 10.03 (1H, t, ); 8.56 (1H, s, ); 7.87 (2H, d, J=8.2 Hz); 7.81 (1H, d, J=7.8 Hz); 7.75 (2H, t, J=7.9 Hz); 7.52 (3H, d, J=8.2 Hz); 4.67 (2H, m ); 3.72 (4H, m ); 3.37 (2H, m ); 3.01 (3H, s, ); 2.50 (3H. s ); 2.13 (2H, m ); 1.57 (2H, s).
APCI-MS m/z: 564.2 [MH⁺].

The compounds of Examples 50 to 59 were prepared using a procedure analogous to that described for Example 49.

### Example 50 5-{[(2-Furylmethyl)amino]methyl}-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.64 (1H, t, J 6.1 Hz); 8.12 (1H, s); 7.58 - 7.47 (5H, m); 7.35 (1H, d, J 7.7 Hz); 7.24 - 7.20 (3H, m); 6.07 (1H, dd, J 3.1, 1.8 Hz); 5.95 (1H, d, J 3.0 Hz); 4.26 (2H, d, J 6.1 Hz); 3.40 (2H, s); 3.29 (2H, s); 2.85 (3H, s); 1.66 (3H, s).
APCI-MS m/z: 574.2 [MH⁺].

### Example 51 5-[(Cyclopropylamino)methyl]-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.97 (1H, t, J 6.0 Hz); 8.43 (1H, s); 7.90 - 7.84 (4H, m); 7.81 (1H, t, J 7.9 Hz); 7.66 (1H, d, J 7.8 Hz); 7.53 (2H, d, J 8.3 Hz); 4.58 (2H, d, J 6.0 Hz); 3.65 (2H, s); 3.17 (3H, s); 2.11 - 2.07 (1H, m); 2.02 (3H, s); 0.38 - 0.35 (2H, m, ); 0.26 - 0.23 (2H, m).
APCI-MS m/z: 534.2 [MH⁺].

### Example 52 5-{[(2-Hydroxypropyl)amino]methyl}-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.97 (1H, t, J 6.1 Hz); 8.45 (1H, s); 7.90 - 7.85 (4H, m); 7.81 (1H, t, J 7.8 Hz); 7.68 (1H, d, J 8.1 Hz); 7.53 (2H, d, J 8.4 Hz); 4.58 (2H, d, J 6.0 Hz); 4.49 (1H, d, J 4.4 Hz); 3.72 - 3.68 (1H, m, ); 3.63 (2H, s); 3.17 (3H, s); 2.49 - 2.46 (2H, m); 2.02 (3H, s); 1.05 (3H, d, J 6.2 Hz).
APCI-MS m/z: 552.2 [MH⁺].

### Example 53 5-[(Cyclopentylamino)methyl]-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.97 (1H, t, J 6.1 Hz); 8.45 (1H, s); 7.90 - 7.85 (4H, m); 7.81 (1H, t, J 7.9 Hz); 7.67 (1H, d, J 8.0 Hz); 7.53 (2H, d, J 8.3 Hz); 4.58 (2H, d, J 6.0 Hz); 3.57 (2H, s); 3.17 (3H, s); 3.05 (1H, t, J 6.1 Hz); 2.02 (3H, s); 1.77 - 1.70 (2H, m); 1.64 - 1.60 (2H, m); 1.49 - 1.45 (2H, m ); 1.39 - 1.33 (2H, m).
APCI-MS m/z: 562.2 [MH⁺].

### Example 54 5-{[(2-Hydroxyethyl)(methyl)amino]methyl}-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.97 (1H, t, J 6.0 Hz); 8.36 (1H, s); 7.89 - 7.85 (4H, m); 7.80 (1H, t, J 8.2 Hz); 7.69 (1H, d, J 8.4 Hz); 7.54 (2H, d, J 8.3 Hz); 4.58 (2H, d, J 6.0 Hz); 4.41 (1H, t, J 5.2 Hz); 3.52 (2H, q, J 5.9 Hz); 3.46 (2H, d, J 30.9 Hz); 3.17 (3H, s); 2.47 (2H, d); 2.18 (3H, s); 2.03 (3H, s).
APCI-MS m/z: 552.2 [MH⁺].

### Example 55 6-Methyl-N-[4-(methylsulfonyl)benzyl]-2-oxo-5-(pyrrolidin-1-ylmethyl)-1-[3-(trifluoromethyl)phenyl]-1,2-dihydropyridine-3-carboxamide

¹H NMR (DMSO-d₆): δ 9.97 (1H, t, J 6.1 Hz); 8.37 (1H, s); 7.90 - 7.85 (4H, m); 7.80 (1H, t, J 7.8 Hz); 7.70 (1H, d, J 7.9 Hz); 7.53 (2H, d, J 8.4 Hz); 4.57 (2H, d, J 6.1 Hz); 3.51 (2H, s); 3.17 (3H, s); 2.47 - 2.44 (4H, m); 2.03 (3H, s); 1.70 (4H, m ).
APCI-MS m/z: 548.2 [MH⁺].

### Example 56 5-{[Methoxy(methyl)amino]methyl}-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.94 (1H, t, J 6.0 Hz); 8.39 (1H, s); 7.90 - 7.85 (4H, m); 7.81 (1H, t, J 8.1 Hz); 7.69 (1H, d, J 7.9 Hz); 7.54 (2H, d, J 8.4 Hz); 4.58 (2H, d, J 6.0 Hz); 3.72 (2H, s); 3.17 (3H, s); 2.58 (3H, s, ); 2.04 (3H, s).
APCI-MS m/z: 538.2 [MH⁺].

### Example 57 5-{[(Cyanomethyl)amino]methyl}-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.78 (1H, t, J 6.2 Hz); 8.25 (1H, s ); 7.74 - 7.64 (5H, m ); 7.51 (1H, d, J 8.1 Hz); 7.37 (2H, d, J 8.3 Hz); 4.42 (2H, d, J 5.7 Hz); 3.53 (2H, d, J 5.7 Hz); 3.48 (2H, d, J 7.1 Hz); 3.01 (3H, s); 1.87 (3H, s ).
APCI-MS m/z: 533.2 [MH⁺].

### Example 58 5-{[(Cyclopropylmethyl)amino]methyl}-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.98 (1H, t, J 6.0 Hz); 8.46 (1H, s); 7.88 (4H, dd, J 10.5, 8.5 Hz); 7.81 (1H, t, J 7.9 Hz); 7.68 (1H, d, J 7.9 Hz); 7.53 (2H, d, J 8.3 Hz); 4.58 (2H, d, J 6.0 Hz); 3.62 (2H, s); 3.17 (3H, s); 2.42 (2H, d, J 6.7 Hz); 2.02 (3H, s); 0.91 (1H,s ); 0.43 - 0.38 (2H, m); 0.14 - 0.10 (2H, m).
APCI-MS m/z: 548.2 [MH⁺].

### Example 59 5-[(3-Hydroxypyrrolidin-1-yl)methyl]-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.97 (1H, t, J 6.1 Hz); 8.37 (1H, s); 7.90 - 7.85 (4H, m); 7.80 (1H, t, J 7.9 Hz); 7.70 (1H, d, J 7.9 Hz); 7.54 (2H, d, J 8.3 Hz); 4.71 (1H, d, J 4.2 Hz); 4.58 (2H, d, J 6.1 Hz); 4.20 (1H, dt, J 9.7, 3.7 Hz); 3.49 (2H, s); 3.17 (3H, s); 2.79 - 2.74 (1H, m); 2.58 (1H, t, J 7.6 Hz); 2.50 - 2.45 (1H, m); 2.35 - 2.32 (1H, m); 2.03 (3H, s); 2.00 - 1.97 (1H, m); 1.57 - 1.53 (1H, m).
APCI-MS m/z: 564.2 [MH⁺].

### Example 60 5-(2-Hydroxyethoxy)-N-[4-(isopropylsulfonyl)benzyl]-6-methyl-2-oxo-1-[3-(trifluoromethyl)phenyl]-1,2-dihydropyridine-3-carboxamide

### a) 5-Hydroxy-N-[4-(isopropylsulfonyl)benzyl]-6-methyl-2-oxo-1-[3-(trifluoromethyl)phenyl]-1,2-dihydropyridine-3-carboxamide

To ice-cooled 35% hydrogen peroxide (11.11 g, 114.4 mmol) was added concentrated sulphuric acid (8.92 g, 91.0 mmol) and 5-acetyl-N-[4-(isopropylsulfonyl)benzyl]-6-methyl-2-oxo-1-[3-(trifluoromethyl)phenyl]-1,2-dihydropyridine-3-carboxamide (Example 35 (c), 2.2 g, 4.1 mmol) in DCM (3 ml). The mixture was stirred vigorously and heated at 45 °C for 1.5 h. The reaction mixture was cooled to room temperature and then added dropwise to an ice cooled mixture of ethyl acetate (100 ml) and saturated aqueous sodium carbonate solution under stirring. The organic layer was collected and the water layer was extracted with ethyl acetate (2 x 60 ml). The combined organic layers were washed with water and brine, dried over sodium sulfate, filtered and concentrated in vacuo. The residue was purified by flash chromatography on silica eluting with tert-butyl methyl ether/methanol (10:0.2) to give the title compound as a yellow solid (1.1 g, 52 %).
¹H NMR (CDCl₃): δ 10.66 (1H, t, J 6.0 Hz); 8.97 (1H, s); 8.16 (1H, s); 7.81 (3H, m); 7.74 (1H, t, J 7.8 Hz); 7.51 (3H, t, J 4.1 Hz); 7.43 (1H, d, J 7.8 Hz); 4.68 (2H, td, J 9.5 4.5 Hz); 3.16 (1H, quintet, J 6.9 Hz); 2.04 (3H, s); 1.28 (6H, d, J 6.9 Hz).
APCI-MS m/z: 509.1 [MH⁺].

### b) 2-({5-({[4-(Isopropylsulfonyl)benzyl]amino}carbonyl)-2-methyl-6-oxo-1-[3-(trifluoromethyl)phenyl]-1,6-dihydropyridin-3-yl}oxy)ethyl acetate

A mixture of 5-hydroxy-N-[4-(isopropylsulfonyl)benzyl]-6-methyl-2-oxo-1-[3-(trifluoromethyl)phenyl]-1,2-dihydropyridine-3-carboxamide (1.05 g, 2.06 mmol), 2-bromoethyl acetate (1.03 g, 6.17 mmol), 1,8-diazabicyclo[5.4.0]undec-7-ene (0.94 g, 6.17 mmol) in anhydrous DMF (2.5 ml) was heated at 80 °C for 25 min under argon. The reaction mixture was cooled and partitioned between ethyl acetate and water. The organic layer was washed with water, dried over sodium sulfate, filtered and concentrated in vacuo. The residue was purified by flash chromatography eluting with a gradient of tert-butyl methyl ether to 2.5% methanol in tert-butyl methyl ether to provide the title compound as a light green solid (0.87 g, 71%).
¹H NMR (CDCl₃): δ 10.11 (1H, t, J 5.8 Hz); 8.58 (1H, s); 7.79 (3H, d, J 8.4 Hz); 7.73 (1H, t, J 7.9 Hz); 7.48 (3H, d, J 8.2 Hz); 7.41 (1H, d, J 7.9 Hz); 4.67 (2H, ddd, J 21.7, 15.6 and 6.1 Hz); 4.38 (2H, dd, J 5.4 4.0 Hz); 4.21 (2H, dd, J 5.2, 3.7 Hz); 3.13 (1H, septet, J 9.0 Hz); 2.10 (3H, s); 2.04 (3H, s); 1.26 (6H, d, J = 6.9 Hz).
APCI-MS m/z: 595.1 [MH⁺].

### c) 5-(2-Hydroxyethoxy)-N-[4-(isopropylsulfonyl)benzyl]-6-methyl-2-oxo-1-[3-(trifluoromethyl)phenyl]-1,2-dihydropyridine-3-carboxamide

A mixture of 2-({5-({[4-(isopropylsulfonyl)benzyl]amino}carbonyl)-2-methyl-6-oxo-1-[3-(trifluoromethyl)phenyl]-1,6-dihydropyridin-3-yl}oxy)ethyl acetate (0.87 g, 1.46 mmol), methanol (5 ml) and 2M sodium hydroxide solution (0.2 ml, 0.4 mmol) was stirred at ambient temperature for 20 min. The solution was acidified with acetic acid, diluted with water and purified by preparative HPLC to give the title compound as a light green solid (0.65 g, 82%).
¹H NMR (CDCl₃): δ 10.13 (1H, t, J 5.7 Hz); 8.59 (1H, s); 7.79 (3H, d, J 8.4 Hz); 7.73 (1H, t, J 7.9 Hz); 7.51 - 7.46 (3H, m); 7.42 (1H, d, J 7.8 Hz); 4.67 (2H, ddd, J 21.7 15.6 6.1 Hz); 4.12 (2H, t, J 4.5 Hz); 3.96 (2H, septet, J 9.6 Hz); 3.14 (1H, septet, J 9.6 Hz); 2.06 (3H, s); 1.83 (1H, t, J 6.0 Hz); 1.26 (6H, d, J 6.9 Hz).
APCI-MS m/z: 553.1 [MH⁺].

### Example 61 2-Methyl-5-({[4-(methylsulfonyl)benzyl]amino}carbonyl)-6-oxo-1-[3-(trifluoromethyl)phenyl]-1,6-dihydropyridin-3-yl acetate

The title compound was obtained as a byproduct during the synthesis of 5-hydroxy-6-methyl-*N*-[4-(methylsulfonyl)benzyl]-2-oxo-1-[3-(trifluoromethyl)phenyl]-1,2-dihydropyridine-3-carboxamide when the latter compound was prepared by a procedure analogous to that described for Example 60 (a).
¹H NMR (DMSO-d₆): δ 9.93 (1H, t, J 6.1 Hz); 8.33 (1H, s); 7.96 - 7.80 (4H, m); 7.75 (2H, d, J 8.2 H); 7.54 (2H, d, J 8.3 Hz); 4.59 (2H, d, J 6.2 Hz); 3.17 (3H, s); 2.32 (3H, s); 1.89 (3H, s).
APCI-MS m/z: 523.3 [MH⁺].

### Example 62 5-Methoxy-6-methyl-N-[4-(methylsulfonyl)benzyl]-2-oxo-1-[3-(trifluoromethyl)phenyl]-1,2-dihydropyridine-3-carboxamide

A solution of 5-hydroxy-6-methyl-*N*-[4-(methylsulfonyl)benzyl]-2-oxo-1-[3-(trifluoromethyl)phenyl]-1,2-dihydropyridine-3-carboxamide(Example 61, 15 mg, 0.031 mmol) and K₂CO₃ (13.8 mg, 0.1 mmol) in acetone (1 ml) was heated to 60°C. After 15 minutes the mixture was cooled to room temperature, iodomethane (7 mg, 0.050 mmol) was added, and the reaction mixture was heated for an additional 30 minutes at 60 °C. The mixture was then purified by preparative HPLC to give the title compound as a white solid (14 mg, 90 %).
¹H NMR (DMSO-d₆): δ 10.11 (1H, t, J=5.9 Hz); 8.39 (1H, s, ); 7.90 - 7.85 (5H, m, ); 7.81 (4H, t, J=7.9 Hz); 7.71 (1H, d, J=8.0 Hz); 7.54 (3H, d, J=8.3 Hz); 4.59 (2H, d, J=6.0 Hz); 3.81 (3H, s, ); 3.18 (4H, s, ); 1.95 (3H, s, ).
APCI-MS m/z: 495.5 [MH⁺].

### Example 63 5-(3-Methoxypropoxy)-6-methyl-N-[4-(methylsulfonyl)benzyl]-2-oxo-1-[3-(trifluoromethyl)phenyl]-1,2-dihydropyridine-3-carboxamide

To a solution of 5-hydroxy-6-methyl-*N*-[4-(methylsulfonyl)benzyl]-2-oxo-1-[3-(trifluoromethyl)phenyl]-1,2-dihydropyridine-3-carboxamide (Example 61, 10 mg, 0.02 mmol), lithium iodide (2.7 mg, 0.02 mmol) and 1,8-diazabicyclo[5.4.0]undec-7-ene (7 mg, 0.04 mmol) in DMF (1 ml), 1-bromo-3-methoxypropane (6.1 mg, 0.04 mmol) was added, and the reaction mixture was heated for 60 minutes at 40°C. The mixture was then purified by preparative HPLC to give the title compound as a white solid (5 mg, 45 %).
¹H NMR (CDCl₃): δ 8.58 (1H, s); 10.2 (1H, t); 7.87 (2H, d, J 8.3 Hz); 7.80 (1H, d); 7.74 (1H, t, ); 7.52 (3H, d, J 8.4 Hz); 7.4 (1H, d); 4.67 (2H, t, J 5.6 Hz); 4.10 (2H, t, J 6.3 Hz); 3.56 (2H, t, J 6.1 Hz); 3.37 (3H, s); 3.02 (3H, s, ); 2.05 (3H,s).
APCI-MS m/z: 553.2 [MH⁺].

### Example 64 2-Methyl-5-({[4-(methylsulfonyl)benzyl]amino}carbonyl)-6-oxo-1-[3-(trifluoromethyl)phenyl]-1,6-dihydropyridin-3-yl methanesulfonate

To a solution of 5-hydroxy-6-methyl-N-[4-(methylsulfonyl)benzyl]-2-oxo-1-[3-(trifluoromethyl)phenyl]-1,2-dihydropyridine-3-carboxamide (5 mg, 0.01 mmol) and 1,8-diazabicyclo[5.4.0]undec-7-ene (3 mg, 0.02mmol) in DCM (1 ml), 2-chloroethylamine hydrochloride (2.3 mg, 0.02 mmol) was added and the reaction mixture was heated for 60 minutes at 40°C. The mixture was then purified by preparative HPLC to give the title compound as a white solid (3 mg, 54 %).
¹H NMR (CDCl₃): δ 7.88 (2H, d, J 8.4 Hz); 7.83 (1H, s); 7.77 (1H, ); 7.55 - 7.50 (3H, m); 7.48 - 7.43 (1H, m, ); 4.67 (2H, t, J 6.3 Hz); 3.33 (3H, s); 3.02 (3H, s); 2.15 (3H, s).
APCI-MS m/z: 559.1 [MH⁺].

### Example 65 5-Ethoxy-6-methyl-N-[4-(methylsulfonyl)benzyl]-2-oxo-1-[3-(trifluoromethyl)phenyl]-1,2-dihydropyridine-3-carboxamide

A mixture of 5-hydroxy-6-methyl-N-[4-(methylsulfonyl)benzyl]-2-oxo-1-[3-(trifluoromethyl)phenyl]-1,2-dihydropyridine-3-carboxamide (Example 61, 40.7 mg, 0.08 mmol), iodoethane (0.4 ml, 5.0 mmol), 1,8-diazabicyclo[5.4.0]undec-7-ene (0.6 g, 4.1 mmol) in DMF (1.5 ml) was stirred at 85 °C for 30 minutes under argon. The reaction mixture was cooled, diluted with water and purified by preparative HPLC to give the title compound as a white solid (11.5 mg, 26 %).
¹H NMR (CDCl₃): δ 10.19 (1H, t, J 5.8 Hz); 8.57 (1H, s); 7.87 (2H, d, J 8.3 Hz); 7.81 (1H, d, J 8.0 Hz); 7.74 (1H, t, *J* 7.9 Hz); 7.52 (3H, d, *J* 8.4 Hz); 7.43 (1H, d, *J* 7.7 Hz); 4.69 (2H, m); 4.09 (2H, q, *J* 7.0 Hz); 3.01 (3H, s); 2.05 (3H, s); 1.41 (3H, t, *J* 7.0 Hz).
APCI-MS m/z: 509.0 [MH⁺].

### Example 66 5-(2-Hydroxyethoxy)-6-methyl-N-[4-(methylsulfonyl)benzyl]-2-oxo-1-[3-(trifluoromethyl)phenyl]-1,2-dihydropyridine-3-carboxamide

The title compound was obtained by a procedure analogous to that described for Example 60.
¹H NMR (CDCl₃): δ 10.15 (1H, t, J 5.9 Hz); 8.60 (1H, s); 7.87 (2H, d, J 8.3 Hz); 7.81 (1H, d, J 7.8 Hz); 7.74 (1H, t, J 7.8 Hz); 7.52 (3H, d, J 8.3 Hz); 7.43 (1H, d, J 7.8 Hz); 4.67 (2H, m); 4.13 (2H, t, J 4.5 Hz); 3.98 (2H, q, J 20.3 Hz); 3.02 (3H, s); 2.08 (3H, s); 1.86 (1H, t, J 6.0 Hz).
API-MS m/z: 525.1 [MH⁺].

### Example 67 5-(Cyanomethoxy)-6-methyl-N-[4-(methylsulfonyl)benzyl]-2-oxo-1-[3-(trifluoromethyl)phenyl]-1,2-dihydropyridine-3-carboxamide trifluoroacetate

The title compound was obtained using analogous conditions to those described in Example 65.
¹H NMR (CDCl₃): δ 10.12 (1H, t, *J* 5.6 Hz); 8.61 (1H, s); 7.89 (2H, d, *J* 8.3 Hz); 7.84 (1H, d, J 7.9 Hz); 7.76 (1H, t, *J* 7.9 Hz); 7.51 (3H, d, J 8.2 Hz); 7.45 (1H, d, *J* 8.0 Hz); 4.82 (2H, s); 4.69 (2H, ddd, *J* 21.5 15.4 6.1 Hz); 3.02 (3H, s); 2.14 (3H, s).
APCI-MS m/z: 520.0 [MH⁺].

### Example 68 2-({2-Methyl-5-({[4-(methylsulfonyl)benzyl]amino}carbonyl)-6-oxo-1-[3-(trifluoromethyl)phenyl]-1,6-dihydropvridin-3-yl}oxy)ethyl acetate

The title compound was obtained under similar conditions to those described in Example 60.
¹H NMR (CDCl₃): δ 10.13 (1H, t, J 5.7 Hz); 8.59 (1H, s); 7.88 (2H, d, J 8.4 Hz); 7.81 (1H, d, J 8.0 Hz); 7.75 (1H, t, J 7.9 Hz); 7.52 (3H, d, J 8.4 Hz); 7.43 (1H, d, J 8.0 Hz); 4.67 (2H, t, J 5.7 Hz); 4.40 (2H, dd, J 5.4 3.9 Hz); 4.22 (2H, dd, J 5.2 3.8 Hz); 3.02 (3H, s); 2.12 (3H, s); 2.05 (3H, s).
APCI-MS m/z: 567.2 [MH⁺].

### Example 69 5-[2-(Dimethylamino)-2-oxoethoxy]-6-methyl-N-[4-(methylsulfonyl)benzyl]-2-oxo-1-[3-(trifluoromethyl)phenyl]-1,2-dihydropyridine-3-carboxamide

### a) tert-Butyl ({2-methyl-5-({[4-(methylsulfonyl)benzyl]amino}carbonyl)-6-oxo-1-[3-(trifluoromethyl)phenyl]-1,6-dihydropyridin-3-ly}oxy)acetate

The title compound was obtained under similar conditions to those described in Example 60.
¹H NMR (CDCl₃): δ 10.15 (1H, t, J 5.9 Hz); 8.51 (1H, s); 7.87 (2H, d, J 8.4 Hz); 7.81 (1H, d, J 8.0 Hz); 7.74 (1H, t, J 7.9 Hz); 7.51 (3H, m); 7.43 (1H, d, J 7.8 Hz); 4.67 (2H, ddd, J 20.6, 15.2 and 5.8 Hz); 4.57 (2H, s); 3.01 (3H, s); 2.14 (3H, s); 1.51 (9H, s).
APCI-MS m/z: 595.3 [MH⁺].

### b) ({2-Methyl-5-({[4-(methylsulfonyl)benzyl]amino}carbonyl)-6-oxo-1-[3-(trifluoromethyl)phenyl]-1,6-dihydropyridin-3-yl}oxy)acetic acid

To a solution of tert-butyl ({2-methyl-5-({[4-(methylsulfonyl)benzyl]amino}carbonyl)-6-oxo-1-[3-(trifluoromethyl)phenyl]-1,6-dihydropyridin-3-yl}oxy)acetate (103 mg, 0.17 mmol) in THF (3 ml) and methanol (2 ml) was added 2M aqueous sodium hydroxide solution (0.6 ml, 1.2 mmol). After stirring for 1 h, the reaction mixture was acidified with acetic acid and concentrated in vacuo. The residue was purified by preparative HPLC to give the title compound as a white solid (87.7 mg, 95 %).
¹H NMR (CDCl₃): δ 10.48 (1H, t, J 5.9 Hz); 8.58 (1H, s); 7.87 (2H, d, J 8.4 Hz); 7.82 (1H, d, J 8.1 Hz); 7.75 (1H, t, J 7.9 Hz); 7.51 (3H, d, J 23.6 Hz); 7.44 (1H, d, J 7.9 Hz); 4.78 (2H, s); 4.67 (2H, ddd, J 21.7 15.5 6.1 Hz); 3.02 (3H, s); 2.14 (3H, s).
APCI-MS m/z: 539.1 [MH⁺].

### c) 5-[2-(Dimethylamino)-2-oxoethoxy]-6-methyl-N-[4-(methylsulfonyl)benzyl]-2-oxo-1-[3-(trifluoromethyl)phenyl]-1,2-dihydropyridine-3-carboxamide

A mixture of ({2-methyl-5-({[4-(methylsulfonyl)benzyl]amino}carbonyl)-6-oxo-1-[3-(trifluoromethyl)phenyl]-1,6-dihydropyridin-3-yl}oxy)acetic acid (87.5 mg, 0.16 mmol) and thionyl chloride (0.6 ml, 8.24 mmol) in DCM (5 ml) was stirred at 35°C for 30 minutes under an argon atmosphere. The mixture was cooled and concentrated in vacuo. The last traces of thionyl chloride were removed by azeotropic evaporation with toluene. The residue was dissolved in DCM (4 ml) and dimethylamine (0.5 ml) was added. After 30 minutes stirring at ambient temperature the reaction mixture was concentrated and purified by preparative HPLC to give the title compound as a white solid (47.7 mg, 53 %).
¹H NMR (CDCl₃): δ 10.19 (1H, t, J 5.9 Hz); 8.48 (1H, s); 7.87 (2H, d, J 8.3 Hz); 7.80 (1H, d, J 7.9 Hz); 7.73 (1H, t, J 7.8 Hz); 7.51 (3H, d, J 8.1 Hz); 7.44 (1H, d, J 8.3 Hz); 4.81 (2H, s); 4.66 (2H, dd, J 20.7 9.5 Hz); 3.02 (6H, d, J 4.8 Hz); 2.99 (3H, s); 2.16 (3H, s).
APCI-MS m/z: 566.2 [MH⁺].

### Example 70 5-(2-Aminoethoxy)-N-[4-(isopropylsulfonyl)benzyl]-6-methyl-2-oxo-1-[3-(trifluoromethyl)phenyl]-1,2-dihydropyridine-3-carboxamide

To a solution of 5-hydroxy-6-methyl-N-[4-(isopropylsulfonyl)benzyl]-2-oxo-1-[3-(trifluoromethyl)phenyl]-1,2-dihydropyridine-3-carboxamide (Example 60 (a), 25 mg, 0.049 mmol) and 1,8-diazabicyclo[5.4.0]undec-7-ene (15 mg, 0.099 mmol) in NMP (1.5 ml), 2-chloroethylamine hydrochloride (11.5 mg, 0.099 mmol) was added and the reaction mixture was heated in a microwave oven for 10 minutes at 70°C. The mixture was then purified by preparative HPLC to give the title compound as a white solid (8 mg, 25 %).
¹H NMR (DMSO-d₆): δ 10.11 (1H, t, J=6.1 Hz); 8.38 (1H, s, ); 7.90 (2H, d, J=7.8 Hz); 7.83 - 7.78 (3H, m, ); 7.72 (1H, d, J=8.0 Hz); 7.54 (2H, d, J=8.3 Hz); 4.61 (2H, d, J=6.2 Hz); 3.93 (2H, t, J=5.6 Hz); 3.42 - 3.31 (2H, m, ); 2.86 (2H, t, J=5.6 Hz); 1.97 (3H, s, J=4.6 Hz); 1.14 (3H, s, ); 1.12 (3H, s, ).
APCI-MS m/z: 552.2 [MH⁺].

### Example 71 5-(Acetylamino)-6-methyl-N-[4-(methylsulfonyl)benzyl]-2-oxo-1-[3-(trifluoromethyl)phenyl]-1,2-dihydropyridine-3-carboxamide

A mixture of 6-methyl-N-[4-(methylsulfonyl)benzyl]-5-nitro-2-oxo-1-[3-(trifluoromethyl)phenyl]-1,2-dihydropyridine-3-carboxamide (Example 2, 15.3 mg, 0.03 mmol) and iron powder (20 mg, 0.36 mmol) in acetic acid (1.5 ml) was stirred at ambient temperature for 1 h. The iron powder was filtered off and the filtrate was concentrated in vacuo. Acetic anhydride (0.25 ml, 2.6 mmol), DMF (1 ml) and saturated aqueous sodium hydrogencarbonate solution were added to the residue. After stirring the reaction mixture at room temperature for 30 minutes, it was neutralized with aqueous sodium hydroxide solution and purified by preparative HPLC to give the title compound as a white solid (3.5 mg, 22 %).
¹H NMR (CDCl₃): δ 9.94 (1H, t, *J* 6.0 Hz); 8.47 (1H, s); 7.87 (2H, d, *J* 8.4 Hz); 7.81 (1H, d, *J* 7.9 Hz); 7.74 (1H, t, *J* 7.9 Hz); 7.53 (1H, s); 7.50 (2H, d, J 8.4 Hz); 7.46 (1H, d, *J* 7.9 Hz); 7.13 (1H, s); 4.68 (2H, m); 3.02 (3H, s); 2.20 (3H, s); 1.99 (3H, s).
APCI-MS m/z: 522.1 [MH⁺].

### Example 72 N-[4-(Isoropylsulfonyl)benzyl]-6-methyl-5-[3-(methylamino)propoxy]-2-oxo-1-[3-(trifluoromethyl)phenyl]-1,2-dihydropyridine-3-carboxamide.

### a) 5-(3-Bromopropoxy)-N-[4-(isopropylsulfonyl)benzyl]-6-methyl-2-oxo-1-[3-(trifluoromethyl)phenyl]-1,2-dihydropyridine-3-carboxamide

A solution of 5-hydroxy-N-[4-(isopropylsulfonyl)benzyl]-6-methyl-2-oxo-1-[3-(trifluoromethyl)phenyl]-1,2-dihydropyridine-3-carboxamide (500 mg, 0.98 mmol), CsCO₃ (1.28 mg, 3.94 mmol) and 1,3 dibromopropane (795 mg, 3.94mmol) in DMF (6 ml) was heated to 70 °C for 30 minutes. After cooling the mixture was purified by preparative HPLC to give the title compound as a white solid (100 mg, 16 %).
APCI-MS m/z: 629.2 [MH⁺].

### b) N-[4-(Isopropylsulfonyl)benzyl]-6-methyl-5-[3-(methylamino)propbxy]-2-oxo-1-[3-(trifluoromethyl)phenyl]-1,2-dihydropyridine-3-carboxamide

A solution of 5-(3-bromopropoxy)-*N*-[4-(isopropylsulfonyl)benzyl]-6-methyl-2-oxo-1-[3-(trifluoromethyl)phenyl]-1,2-dihydropyridine-3-carboxamide (30 mg, 0.048 mmol) and methanamine (0.5 ml, 2M in THF) was heated in a microwave oven for 60 minutes at 50 °C. The mixture was then purified by preparative HPLC to give the title compound as a white solid (13 mg, 46%).
¹H NMR (CDCl₃): δ 10.06 (1H, t, J 6.0 Hz); 8.43 (1H, 3); 7.68 - 7.66 (3H, m); 7.61 (1H, t, J 7.8 ); 7.37 (3H, d, J 8.4 Hz); 7.31 (1H, d, J 7.6 Hz); 4.55 (2H, t, J 5.8 Hz); 3.98 (2H, t, J 6.1 Hz); 3.06 - 3.00 (1H, m); 2.75 (2H, t, J 7.0 Hz); 2.40 (3H, s); 1.95 (2H, t); 1.91 (3H, s); 1.15 (3H, s); 1.14 (3H, s).
APCI-MS m/z: 580.2 [MH⁺].

### Example 73 5-(1-Methoxyethyl)-6-methyl-N-[4-(methylsulfonyl)benzyl]-2-oxo-1-[3-(trifluoromethyl)phenyl]-1,2-dihydropyridine-3-carboxamide

To a solution of 6-methyl-N-[4-(methylsulfonyl)benzyl]-2-oxo-1-[3-(trifluoromethyl)phenyl]-5-vinyl-1,2-dihydropyridine-3-carboxamide (Example 9, 20.7 mg, 0.04 mmol) in methanol (2 ml) was added concentrated sulphuric acid (0.05 ml). The mixture was stirred at 65 °C overnight and then neutralized with aqueous sodium hydrogencarbonate solution. The reaction mixture was purified by preparative HPLC to give the title compound as white solid (5.9 mg, 27 %).
¹H NMR (CDCl₃): δ 10.01 (1H, t, J 5.8 Hz); 8.72 (1H, s); 7.87 (2H, d, J 8.3 Hz); 7.81 (1H, d, J 7.8 Hz); 7.75 (1H, t, J 7.8 Hz); 7.52 (3H, d, J 28.4 Hz); 7.44 (1H, t, J 6.6 Hz); 4.67 (2H, m); 4.44 (1H, dq, J 0.1 6.3 Hz); 3.29 (3H, d, J 1.0 Hz); 3.01 (3H, s); 2.09 (3H, s); 1.48 (3H, dd, J 6.5 2.1 Hz).
APCI-MS m/z: 523.0 [MH⁺].

### Example 74 5-(2-Bromo-1-methoxyethyl)-6-methyl-N-[4-(methylsulfonyl)benzyl]-2-oxo-1-[3-(trifluoromethyl)phenyl]-1,2-dihydropyridine-3-carboxamide

A mixture of 6-methyl-N-[4-(methylsulfonyl)benzyl]-2-oxo-1-[3-(trifluoromethyl)phenyl]-5-vinyl-1,2-dihydropyridine-3-carboxamide (Example 9, 66.9 mg, 0.14 mmol), N-bromosuccinimide (37.7 mg, 0.21 mmol) and dibenzoyl peroxide (7.1 mg, 0.03 mmol) in methanol (2 ml) was stirred at ambient temperature for 1.5 h. The solvent was removed in vacuo and the residue was purified by preparative HPLC to give the title compound as white solid (44 mg, 52 %).
¹H NMR (CDCl₃): δ 9.94 (1H, t, J 5.9 Hz); 8.69 (1H, d, J 0.8 Hz); 7.88 (2H, d, J 8.3 Hz); 7.83 (1H, d, J 8.0 Hz); 7.76 (1H, t, J 7.9 Hz); 7.52 (2H, d, J 8.2 Hz); 7.50 (1H, s); 7.44 (1H, t, J 7.2 Hz); 4.68 (2H, m); 4.54 (1H, t, J 6.7 Hz); 3.67 (1H, m); 3.46 (1H, m); 3.37 (3H, d, J 1.1 Hz); 3.02 (3H, s); 2.13 (3H, s).
APCI-MS m/z: 600.1, 601.1, 602.1, 603.2 [MH⁺].

### Example 75 5-(1-Isopropoxyethyl)-6-methyl-N-[4-(methylsulfonyl)benzyl]-2-oxo-1-[3-(trifluoromethyl)phenyl]-1,2-dihydropyridine-3-carboxamide

The title compound was prepared by a procedure analogous to that described for Example 73.
¹H NMR (CDCl₃): δ 10.02 (1H, t, J 5.8 Hz); 8.76 (1H, s); 7.87 (2H, d, J 8.3 Hz); 7.81 (1H, d, J 7.8 Hz); 7.74 (1H, t, J 7.9 Hz); 7.52 (3H, d, J 25.3 Hz); 7.43 (1H, t, J 6.7 Hz); 4.68 (3H, m); 3.56 (1H, td, J 6.1 3.7 Hz); 3.01 (3H, s); 2.10 (3H, s); 1.44 (3H, dd, J 6.5 and 2.2 Hz); 1.18 (6H, dd, J 10.8 and 6.1 Hz).
APCI-MS m/z: 551.2 [MH⁺].

### Example 76 5-(N¹-Isobutyryl-hydrazinocarbonyl)-6-methyl-2-oxo-1-(3-trifluoromethylphenyl)-1,2-dihydro-pyridine-3-carboxylic acid 4-methanesulfonyl-benzylamide

### a) 5-Hydrazinocarbonyl-6-methyl-2-oxo-1-(3-trifluoromethyl-phenyl)-1,2-dihydropyridine-3-carboxylic acid 4-methanesulfonyl-benzylamide

The compound obtained in Example 11 (0.051 g, 0.14 mmol) in DCM (5 ml) was treated with SOCl₂ (5 ml) and the flask was sealed and stirred magnetically for 2 h, when LC-MS showed that the reaction was complete. The crude mixture was evaporated in vacuo, giving the intermediate acid chloride as a yellow solid. The solid was dissolved in 1,4-dioxane (5 ml, dried over molecular sieves) and hydrazine hydrate (0.05 g, 1.0 mmol) was added. The mixture was stirred for 10 minutes, and LC-MS showed complete formation of the title compound. The mixture was concentrated in vacuo and the residue was purified by preparative HPLC giving the title compound (0.036 g, 70%) as a white solid after freeze-drying the pure fractions.
APCI-MS m/z: 523.2 [MH⁺].

### b) 5-(N¹-Isobutyryl-hydrazinocarbonyl)-6-methyl-2-oxo-1-(3-trifluoromethyl-phenyl)-1,2-dihydro-pyridine-3-carboxylic acid 4-methanesulfonyl-benzylamide

The compound obtained in step (a) (0.025 g, 0.047 mmol) in dry THF (10 ml) was stirred and isobutyric anhydride (0.040 g, 0.25 mmol) was added. The obtained mixture was stirred for 15 minutes, and LC-MS showed complete conversion of the starting material to the desired amide. The solvent was evaporated and the residue was purified by preparative HPLC giving the subtitle compound (0.024 g, 85%) as a white powder after freeze-drying the pure fractions.
¹H NMR (DMSO-d₆): δ 10.25 (1H, bs); 9.89 (1H, bs); 9.79 (1H, t, *J* 6.2 Hz); 8.50 (1H, s); 7.93 (1H, s); 7.94-7.90 (1H, m); 7.87 (2H, d, *J* 8.5 Hz); 7.84 (1H, t, *J* 7.7 Hz); 7.74 (1H, d, *J* 7.7 Hz); 7.55 (2H, d, *J* 8.3 Hz); 4.63-4.56 (2H, m); 3.18 (3H, s); 2.55-2.49 (1H, p, *J* 6.8 Hz); 2.18 (3H, s); 1.08 (6H, d, *J* 6.8 Hz).
APCI-MS m/z: 593.2 [MH⁺].

The compounds of Examples 77 to 87 and 91 to 97 were prepared using a procedure analogous to that described for Example 12.

### Example 77 N⁵-Methoxy-6-methyl-N³-[4-(methylsulfonyl)benzyl]-2-oxo-1-[3-(trifluoromethyl)phenyl]-1,2-dihydropyridine-3,5-dicarboxamide

¹H NMR (DMSO-d₆): δ 11.74 (1H, bs); 9.79 (1H, t); 8.41 (1H, s); 7.95-7.70 (6H, m); 7.54 (2H, d); 4.58 (2H, d); 3.70 (3H, s); 3.18 (3H, s); 2.13 (3H, s).
APCI-MS m/z: 538.1 [MH⁺].

### Example 78 N⁵-Methoxy-N⁵,6-dimethyl-N³-[4-(methylsulfonyl)benzyl]-2-oxo-1-[3-(trifluoromethyl)phenyl]-1,2-dihydropyridine-3,5-dicarboxamide

¹H NMR (DMSO-d₆): δ 9.83 (1H, t); 8.36 (1H, s); 8.01 (1H, bs); 7.94-7.76 (5H, m); 7.54 (2H, d); 4.58 (2H, d); 3.59 (3H, s); 3.28 (3H, s); 3.18 (3H, s); 2.00 (3H, s).
APCI-MS m/z: 552.2 [MH⁺].

### Example 79 5-[(2,5-Dimethyl-2,5-dihydro-1H-pyrrol-1-yl)carbonyl]-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.86 (1H, q); 8.29 (0.5H, d); 8.26 (0.5H, s); 8.06 (0.5H, bs); 8.01 (0.5H, bs); 7.95-7.74 (5H, m); 7.55 (2H, d); 5.83 (2H, dt); 4.80 (1H, bt); 4.58 (2H, d); 4.49 (1H, bd); 3.17 (3H, s); 2.03 (1H, d); 1.92 (1H, d);1.34 (3H, d); 1.08 (2H, d); 1.05 (1H, m).
APCI-MS m/z: 588.2 [MH⁺].

### Example 80 6-Methyl-N³-[4-(methylsulfonyl)benzyl]-2-oxo-N⁵-pyrrolidin-1-yl-1-[3-(trifluoromethyl)phenyl]-1,2-dihydropyridine-3,5-dicarboxamide

¹H NMR (DMSO-d₆): δ 9.84 (2H, bt); 8.43 (1H, s); 7.99-7.78 (5H, m); 7.72 (1H, d); 7.54 (2H, d); 4.58 (2H, d); 3.18 (3H, s); 3.02 (4H, bs); 2.13 (3H, s); 1.80 (4H, bs).
APCI-MS m/z: 577.2 [MH⁺].

### Example 81 6-Methyl-N-[4-(methylsulfonyl)benzyl]-2-oxo-5-(piperidin-1-ylcarbonyl)-1-[3-(trifluoromethyl)phenyl]-1,2-dihydropyridine-3-carboxamide

¹H NMR (DMSO-d₆): δ 9.88 (1H, t); 8.20 (1H, s); 8.03 (1H, d); 7.94-7.74 (5H, m); 7.54 (2H, d); 4.58 (2H, bs); 3.79-3.22 (4H, m); 3.18 (3H, s); 1.93 (3H, s); 1.69-1.36 (6H, m).
APCI-MS m/z: 576.2 [MH⁺].

### Example 82 6-Methyl-N³-[4-(methylsulfonyl)benzyl]-N⁵-morpholin-4-yl-2-oxo-1-[3-(trifluoromethyl)phenyl]-1,2-dihydropyridine-3,5-dicarboxamide

¹H NMR (DMSO-d₆): δ 9.82 (1H, t); 9.57 (0.8H, s); 9.22 (0.2H, s); 8.41 (0.8H, s); 8.36 (0.2H, s); 8.00-7.76 (5H, m); 7.72 (1H, d); 7.54 (2H, d); 4.58 (2H, d); 3.66 (4H, m); 3.17 (3H, s); 2.84 (3.2H, m); 2.73 (0.8H, bs); 2.11 (0.8H, s); 2.02 (0.2H, s).
APCI-MS m/z: 593.2 [MH⁺].

### Example 83 6-Methyl-5-[(4-methylpiperidin-1-yl)carbonyl]-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.88 (1H, t); 8.19 (1H, bd); 8.08-7.73 (6H, m); 7.72 (1H, d); 7.54 (2H, d); 4.58 (2H, bs); 4.43 (1H, bs); 3.72 (1H, bs); 3.17 (3H, s); 3.05 (1H, m); 2.76 (1H, m); 1.93 (3H, d); 1.77-1.55 (3H, m); 1.19-0.80 (5H, m).
APCI-MS m/z: 590.2 [MH⁺].

### Example 84 6-Methyl-N³-[4-(methylsulfonyl)benzyl]-2-oxo-N⁵-piperidin-1-yl-1-[3-(trifluoromethyl)phenyl]-1,2-dihydropyridine-3,5-dicarboxamide

¹H NMR (DMSO-d₆): δ 9.82 (1H, t); 9.64 (0.8H, s); 9.12 (0.2H, s); 8.39 (0.8H, s); 8.35 (0.2H, s); 8.00-7.77 (5H, m); 7.72 (1H, d); 7.54 (2H, d); 4.58 (2H, d); 3.19 (3H, s); 2.84 (3H, m); 2.11 (0.8H, bs); 2.02 (0.2H, s); 1.66-1.53 (4H, m); 1.53-1.32 (3H, m).
APCI-MS m/z: 591.2 [MH⁺].

### Example 85 N⁵-(tert-Butyl)-N⁵,6-dimethyl-N³-[4-(methylsulfonyl)benzyl]-2-oxo-1-[3-(trifluoromethyl)phenyl]-1,2-dihydropyridine-3,5-dicarboxamide

¹H NMR (DMSO-d₆): δ 9.88 (1H, t); 8.19 (1H, s); 7.99 (1H, d); 7.91-7.73 (5H, m); 7.54 (2H, d); 4.58 (2H, bs); 3.18 (3H, s); 2.91 (3H, s); 1.95 (3H, s); 1.45 (9H, s).
APCI-MS m/z: 578.2 [MH⁺]**.**

### Example 86 N⁵-Butyl-N⁵,6-dimethyl-N³-[4-(methylsulfonyl)benzyl]-2-oxo-1-[3-(trifluoromethyl)phenyl]-1,2-dihydropyridine-3,5-dicarboxamide

¹H NMR (DMSO-d₆): δ 9.88 (1H, t); 8.19 (1H, d); 8.01 (1H, s); 7.95-7.74 (5H, m); 7.54 (2H, d); 4.58 (2H, bs); 3.18 (3H, s); 2.95 (1.2H, s); 2.94 (1.8H, s); 1.92 (1.8H, s); 1.91 (1.2H, s); 1.63-1.40 (2H, m); 1.40-1.10 (2H, m); 0.92 (1.8H, t); 0.83 (1.2H t).
APCI-MS m/z: 578.2 [MH⁺].

### Example 87 N⁵-Ethyl-N⁵-isopropyl-6-methyl-N³-[4-(methylsulfonyl)benzyl]-2-oxo-1-[3-(trifluoromethyl)phenyl]-1,2-dihydropyridine-3,5-dicarboxamide

¹H NMR (DMSO-d₆): δ 9.90 (1H, t); 8.20 (0.3H, s); 8.15 (0.7H, s); 8.10-7.95 (1H, m); 7.95-7.75 (5H, m); 7.54 (2H, d); 4.58 (2H, bs); 4.02 (1H, bs); 3.36 (2H, bs); 3.18 (3H, s); 1.91 (3H, s); 1.32-0.96 (9H, m).
APCI-MS m/z: 578.2 [MH⁺].

The compounds of Examples 88 to 90 were prepared using a procedure analogous to that described for Example 76.

### Example 88 5-[N¹-(Formyl-hydrazinocarbonyl]-6-methyl-2-oxo-1-(3-trifluoromethylphenyl)-1,2-dihydro-pyridine-3-carboxylic acid 4-methanesulfonyl-benzylamide

¹H NMR (DMSO-d*₆*): δ 10.43 (1H, s); 10.13 (1H, s); 9.79 (1H, t, *J* 6.2 Hz); 8.52 (1H, s); 8.11 (1H, s); 7.93 (1H, s); 7.94-7.89 (1H, s); 7.87 (2H, d, *J* 8.65 Hz); 7.87 (1H, d, *J* 8.21); 7.74 (1H, d, *J* 8.21 Hz); 7.55 (2H, d, *J* 8.21 Hz); 4.63-4.54 (2H, m); 3.17 (3H, s); 2.18 (3H, s).
APCI-MS m/z: 551.2 [MH⁺].

### Example 89 N¹-[5-(4-Methanesulfonyl-benzylcarbamoyl)-2-methyl-6-oxo-1-(3-trifluoromethyl-phenyl)-1,6-dihydro-pyridine-3-carbonyl]-hydrazinecarboxylic acid methyl ester

¹H NMR (DMSO-d₆): δ 10.23 (1H, s); 9.79 (1H, t, *J* 6.1 Hz); 9.23 (1H, s); 8.47 (1H, s); 7.94 (1H, s); 7.94-7.89 (1H, d, *J* 8.2 Hz); 7.87 (2H, d, *J* 8.4 Hz); 7.82 (1H, d, *J* 7.7 Hz); 7.74 (1H, d, *J* 7.8 Hz); 7.54 (2H, d, *J* 8.4 Hz); 4.65-4.55 (2H, m); 4.14-4.01 (2H, m); 3.17 (3H, s); 2.16 (3H, s); 1.25-1.15 (3H, m).
APCI-MS m/z: 595.2 [MH⁺].

### Example 90 5-({2-[(Ethylamino)carbonothioyl]hydrazino}carbonyl)-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 10.26 (1H, s); 9.81 (1H, t, *J* 6.0 Hz); 9.23 (1H, s); 8.71 (1H, s); 8.14 (1H, bs); 7.95-7.81 (4H, m); 7.71 (1H, d, *J* 8.0 Hz); 7.55 (2H, d, *J* 8.3 Hz); 4.60 (2H, d, *J* 6.1 Hz); 3.54-3.44 (2H, m); 3.18 (3H, s); 2.16 (3H, s); 1.08 (3H, t, *J* 7.1 Hz)
APCI-MS m/z: 610.2 [MH⁺].

### Example 91 5-(Isoxazolidin-2-ylcarbonyl)-6-methyl-N-[4-(methylsulfonyl)benzyl]-2-oxo-1-[3-(trifluoromethyl)phenyl]-1,2-dihydropyridine-3-carboxamide

¹H NMR (DMSO-d₆): δ 9.79 (1H, t); 8.41 (1H, s); 7.99 (1H, s); 7.93-7.74 (5H, m); 7.54 (2H, d); 4.58 (2H, d); 3.96 (2H, t); 3.76 (2H, m); 3.17 (3H, s); 2.31 (2H, p); 2.05 (3H, s).
APCI-MS m/z: 564.3 [MH⁺].

### Example 92 6-Methyl-2-oxo-1-(3-trifluoromethyl-phenyl)-1,2-dihydro-pyridine-3,5-dicarboxylic acid 5-(methoxy-methyl-amide) 3-[4-(propane-2-sulfonyl)-benzylamide]

¹H NMR (CDCl₃): δ 9.87 (1H, bt); 8.67 (1H, s); 7.84-7.74 (4H, m); 7.52-7.49 (3H, bd); 7.45-7.43 (1H, bd); 4.74-4.64 (2H, m); 3.62 (3H, s); 3.38 (3H, s); 3.19-3.13 (1H, m); 2.13 (3H, s); 1.28 (6H, d, *J* 6.8 Hz).
APCI-MS m/z: 580 [MH⁺].

### Example 93 6-Methyl-2-oxo-1-(3-trifluoromethyl-phenyl)-1,2-dihydro-pyridine-3,5-dicarboxylic acid 3-(4-ethanesulfonyl-benzylamide) 5-(methoxy-methyl-amide)

¹H NMR (CDCl₃): δ 9.87 (1H, bt); 8.67 (1H, s); 7.86-7.82 (3H, m); 7.78-7.74 (1H, bt); 7.53-7.50 (3H, m); 7.45-7.43 (1H, bd); 4.74-4.64 (2H, m); 3.66 (3H, s); 3.38 (3H, s); 3.09 (2H, q, *J* 7.5 Hz); 2.13 (3H, s); 1.26 (3H, t, *J* 7.5 Hz).
APCI-MS m/z: 566 [MH⁺].

### Example 94 6-Methyl-2-oxo-1-(3-trifluoromethyl-phenyl)-1,2-dihydro-pyridine-3,5-dicarboxylicacid 3-(4-cyclopropanesulfonyl-benzylamide) 5-(methoxy-methyl-amide)

¹H NMR (CDCl₃): δ 9.86 (1H, bt); 8.67 (1H, s); 7.84-7.82 (3H, bd); 7.78-7.74 (1H, bt); 7.51-7.49 (3H, bd); 7.45-7.43 (1H, bd); 4.73-4.63 (2H, m); 3.66 (3H, s); 3.38 (3H, s); 2.46-2.39 (1H, m); 1.35-1-31 (2H, m); 1.04-0.99 (2H, m).
APCI-MS m/z: 578 [MH⁺].

### Example 95 6-Methyl-2-oxo-1-(3-trifluoromethyl-phenyl)-1,2-dihydro-pyridine-3,5-dicarboxylic acid 5-[(2-hydroxy-ethyl)-amide] 3-(4-methanesulfonyl-benzylamide

¹H NMR (DMSO-d*₆*): δ 9.82 (1H, t, *J* 6.0 Hz); 8.48 (1H, s); 8.48 (1H, t, *J* 5.6 Hz); 7.93-7.89 (1H, m); 7.90 (1H, s); 7.87 (2H, d, *J* 8.2 Hz); 7.83 (1H, t, *J* 7.6 Hz); 7.72 (1H, d, *J* 7.9 Hz); 7.54 (2H, d, *J* 8.2 Hz); 4.74 (1H, bs); 4.64-4.54 (2H, m); 3.50 (2H, t, *J* 6.2 Hz); 3.32-3.25 (2H, m); 3.17 (3H, s); 2.14 (3H, s).
APCI-MS m/z: 552.2 [MH⁺].

### Example 96 5-(Isoxazolidine-2-carbonyl)-6-methyl-2-oxo-1-(3-trifluoromethylphenyl)1,2dihydro-pyridine-3-carboxylic acid 4-ethanesulfonyl-benzylamide

¹H NMR (CDCl₃): δ 9.83 (1H, bt); 8.70 (1H, s); 7.85-7.81 (3H, m); 7.77-7.73 (1H, bt); 7.53-7.50 (3H, m); 7.45-7.43 (1H, bd); 4.73-4.63 (2H, m); 4.06 (2H, t, *J* 7.2 Hz); 3.91 (2H, t, *J* 7.2 Hz); 3.08 (2H, q, *J* 7.7 Hz); 2.48-2.41 (2H, m); 2.19 (3H, s); 1.26 (3H, t, *J* 7.7 Hz).
APCI-MS m/z: 579 [MH⁺].

### Example 97 5-(Isoxazolidine-2-carbonyl)-6-methyl-2-oxo-1-(3-trifluoromethylphenyl) 1,2dihydropyridine-3-carboxylic acid 4-cyclopropanesulfonylbenzylamide

¹H NMR (CDCl₃): δ 9.81 (1H, bt); 8.70 (1H, s); 7.84-7.81 (3H, m); 7.77-7.73 (1H, bt); 7.52-7.48 (3H, m); 7.45-7.43 (1H, bd); 4.73-4.62 (2H, m); 4.06 (2H, t, *J* 6.8 Hz); 3.91 (2H, t, *J* 7.2 Hz); 2.48-2.39 (3H, m); 2.19 (3H, s); 1.35-1.31 (2H, m); 1.04-0.99 (2H, m).
APCI-MS m/z: 590[MH⁺].

### Example 98 5-(N-Hydroxycarbamimidoyl)-6-methyl-2-oxo-1-(3-trifluoromethylphenyl)-1,2-dihydro-pyridine-3-carboxylic acid 4-methanesulfonyl-benzylamide

5-Cyano-6-methyl-2-oxo-1-(3-trifluoromethyl-phenyl)-1,2-dihydro-pyridine-3-carboxylic acid 4-methanesulfonyl-benzylamide Example 1, 0.040 g, 0.082 mmol), hydroxylamine hydrochloride (0.015 g, 0.209 mmol), NaOAc (0.017 g, 0.209 mmol), ethanol (3 ml), water (0.1 ml) and a magnetic stirrer were placed in a vial. The mixture was heated at 90 °C overnight. LC-MS showed a 50:50 mixture of two components, one of which had the expected MW. The product was isolated by preparative HPLC giving 0.012 g (28%) of the title compound.
¹H NMR (DMSO-d*₆*): δ 9.85 (1H, t, *J* 6.2 Hz); 9.53 (1H, s); 8.33 (1H, s); 7.91 (1H, d, *J* 7.6 Hz); 7.86 (2H, d, *J* 8.2 Hz); 7.85 (1H, s); 7.83 (1H, t, *J* 7.8 Hz); 7.69 (1H, d, *J* 7.8 Hz); 7.54 (2H, d, *J* 8.3 Hz); 5.88 (2H, bs); 4.64-4.55 (2H, m); 3.17 (3H, s); 2.07 (3H, s).
APCI-MS m/z: 523.2 [MH⁺].

### Example 99 N³-(Cyclohexylmethyl)-N⁵,N⁵,6-trimethyl-2-oxo-1-[3-(trifluoromethyl)phenyl]-1,2-dihydropyridine-3,5-dicarboxamide

### a) Ethyl 6-methyl-2-oxo-1-[3-(trifluoromethyl)phenyl]-1,2-dihydropyridine-3-carboxylate

A suspension of 6-methyl-2-oxo-1-[3-(trifluoromethyl)phenyl]-1,2-dihydropyridine-3-carboxylic acid (Example 1 (b), 13.1 g, 43.9 mmol), sodium carbonate (5.2 g, 48.3 mmol) and iodoethane (10.6 g, 67.7 mmol) in NMP (60 ml) was stirred at ambient temperature for 19 h under a nitrogen atmosphere. The reaction mixture was partitioned between ethyl acetate and water. The organic phase was collected, washed with water and brine, dried over sodium sulphate, filtered and concentrated in vacuo. The residue was purified by flash chromatography on silica eluting with tert-butyl methyl ether/methanol (10:0.4) to give the title compound as a light brown solid (12.5 g, 87%).
¹H NMR (CDCl₃): δ 8.21 (1H, d, *J* 7.4 Hz); 7.75 (1H, d, *J* 7.8 Hz); 7.68 (1H, t, *J* 7.8 Hz); 7.49 (1H, s); 7.42 (1H, d, *J* 7.8 Hz); 6.25 (1H, d, *J* 7.4 Hz); 4.36 (2H, q, *J* 7.2 Hz); 2.03 (3H, s); 1.37 (3H, t, *J* 7.2 Hz).
APCI-MS m/z: 326.1 [MH⁺].

### b) Ethyl 5-iodo-6-methyl-2-oxo-1-[3-(trifluoromethyl)phenyl]-1,2-dihydropyridine-3-carboxylate

To a solution ethyl 6-methyl-2-oxo-1-[3-(trifluoromethyl)phenyl]-1,2-dihydropyridine-3-carboxylate (9.9 g, 30.5 mmol) in CH₂Cl₂ (45 ml) and TFA (38 ml) was added N-iodosuccinimide (6.89 g, 30.6 mmol) under a nitrogen atmosphere. After 19 h stirring at ambient temperature the solvent was concentrated in vacuo. To the residue were added ethyl acetate and saturated aqueous sodium hydrogencarbonate to neutralize the remaining TFA. The organic phase was collected, washed with water and brine, dried over sodium sulfate, filtered and concentrated in vacuo. The residue was purified by flash chromatography on silica eluting with DCM/methanol (10:0.2) to give the title compound as a yellow solid (11.4 g, 83%).
¹H NMR (CDCl₃): δ 8.52 (1H, s); 7.76 (1H, d, *J* 7.8 Hz); 7.69 (1H, t, *J* 7.9 Hz); 7.46 (1H, s); 7.38 (1H, d, *J* 7.7 Hz); 4.36 (2H, q, *J* 7.1 Hz); 2.26 (3H, s); 1.37 (3H, t, *J* 7.2 Hz).
APCI-MS m/z: 452.0 [MH⁺].

### c) Ethyl 5-[(dimethylamino)carbonyl]-6-methyl-2-oxo-1-[3-(trifluoromethyl)phenyl]-1,2-dihydropyridine-3-carboxylate

Ethyl 5-iodo-6-methyl-2-oxo-1-[3-(trifluoromethyl)phenyl]-1,2-dihydropyridine-3-carboxylate (2.6 g, 5.76 mmol), palladium(II) acetate (16.4 mg, 0.07 mmol), triphenylphosphine (38.7 mg, 0.15 mmol), anhydrous dimethylamine (5 ml) and anhydrous ethanol (20 ml) were placed in a stainless-steel autoclave (100 ml). The reaction mixture was stirred at 85 °C under a carbon monoxide pressure of 5.5 atmospheres for 2 h. After cooling, excess carbon monoxide was released into a fume hood and the solvent was concentrated in vacuo. The residue was purified by preparative HPLC to give the title compound as a white solid (1.3 g, 58 %).
¹H NMR (CDCl₃): δ 8.16 (1H, s); 7.77 (1H, d, *J* 7.9 Hz); 7.70 (1H, t, *J* 7.8 Hz); 7.49 (1H, s); 7.41 (1H, d, *J* 7.9 Hz); 4.36 (2H, q, *J* 7.1 Hz); 3.09 (6H, d, *J* 30.2 Hz); 2.04 (3H, s); 1.37 (3H, t, *J* 7.2 Hz).
APCI-MS m/z: 397.2 [MH⁺].

### d) 5-[(Dimethylamino)carbonyl]-6-methyl-2-oxo-1-[3-(trifluoromethyl)phenyl]-1,2-dihydropyridine-3-carboxylic acid

To a solution of ethyl 5-[(dimethylamino)carbonyl]-6-methyl-2-oxo-1-[3-(trifluoromethyl)phenyl]-1,2-dihydropyridine-3-carboxylate (1.28 g, 3.2 mmol) in THF (5 ml) and water (1 ml) was added dropwise 2M sodium hydroxide (1.7 ml, 3.4 mmol) over 1 h. After an additional 2 h stirring at ambient temperature, the solution was acidified to pH 1 and concentrated in vacuo. The residue was purified by preparative HPLC to give the title compound as a white solid (0.92 g, 77 %).
¹H NMR (CDCl₃): δ 8.47 (1H, s); 7.88 (1H, d, *J* 7.8 Hz); 7.80 (1H, t, *J* 7.9 Hz); 7.56 (1H, s); 7.48 (1H, d, *J* 7.9 Hz); 3.12 (6H, d, *J* 36.0 Hz); 2.15 (3H, s).
APCI-MS m/z: 369.1 [MH⁺].

### e) N³-(Cyclohexylmethyl)-N⁵,N⁵,6-trimethyl-2-oxo-1-[3-(trifluoromethyl)phenyl]-1,2-dihydropyridine-3,5-dicarboxamide

A mixture of 5-[(dimethylamino)carbonyl]-6-methyl-2-oxo-1-[3-(trifluromethyl)-phenyl]-1,2-dihydropyridine-3-carboxylic acid (12 mg, 0.03 mmol), HATU (15 mg, 0.04 mmol), HOAT (7 mg, 0.04 mmol) and DIEA (13 mg, 0.1 mmol) in NMP (160 µl) was added to (cyclohexylmethyl)amine in NMP (135 µl, 0.3M, 0.04 mmol). The reaction mixture was stirred for 17 h at room temperature. The solvent was removed in vacuo, and the residue was dissolved in acetonitrile/water, 50/50, to a total volume of 1.6 ml, and purified using preparative HPLC to give the title compound (8 mg, 58%).
RT (C₁₈, UV 220 nm): 7.3 min.
APCI-MS m/z: 464.2 [MH⁺].

The compounds of Examples 100 to 146 were prepared using a procedure analogous to that described for Example 99.

### Example 100 N⁵,N⁵,6-Trimethyl-2-oxo-N³-(pyridin-3-ylmethyl)-1-[3-(trifluoromethyl)-phenyl]-1,2-dihydropyridine-3,5-dicarboxamide

RT (C₁₈, UV 220 nm): 5.0 min.
APCI-MS m/z: 459.2 [MH⁺].

### Example 101 N⁵,N⁵,6-Trimethyl-N³-(2-morpholin-4-ylethyl)-2-oxo-1-[3-(trifluoromethyl)-phenyl]-1,2-dihydropyridine-3,5-dicarboxamide

RT (C₁₈, UV 220 nm): 4.9 min.
APCI-MS m/z: 481.1 [MH⁺].

### Example 102 N⁵,N⁵,6-Trimethyl-N³-(3-morpholin-4-ylpropyl)-2-oxo-1-[3-(trifluoromethyl)-phenyl]-1,2-dihydropyridine-3,5-dicarboxamide

RT (C₁₈, UV 220 nm): 5.0 min.
APCI-MS m/z: 495.2 [MH⁺].

### Example 103 N³-Benzyl-N⁵,N⁵,6-trimethyl-2-oxo-1-[3-(trifluoromethyl)phenyl]-1,2-dihydro-pyridine-3,5-dicarboxamide

RT (C₁₈, UV 220 nm): 6.8 min.
APCI-MS m/z: 458.2 [MH⁺].

### Example 104 N³-[2-(1H-Indol-3-yl)ethyl]-N⁵,N⁵,6-trimethyl-2-oxo-1-[3-(trifluoromethyl)-phenyl]-1,2-dihydropyridine-3,5-dicarboxamide

RT (C₁₈, UV 220 nm): 6.9 min.
APCI-MS m/z: 511.1 [MH⁺].

### Example 105 N⁵,N⁵,6-Trimethyl-2-oxo-N³-(1-phenylethyl)-1-[3-(trifluoromethyl)phenyl]-1,2-dihydropyridine-3,5-dicarboxamide

RT (C₁₈, UV 220 nm): 7.0 min.
APCI-MS m/z: 472.3 [MH⁺].

### Example 106 N⁵,N⁵,6-Trimethyl-2-oxo-N³-(2-phenylethyl)-1-[3-(trifluoromethyl)phenyl]-1,2-dihydropyridine-3,5-dicarboxamide

RT (C₁₈, UV 220 nm): 5.3 min.
APCI-MS m/z: 472.1 [MH⁺].

### Example 107 N⁵,N⁵,6-Trimethyl-2-oxo-N³-[(2R)-2-phenylcyclopropyl]-1-[3-(trifluoromethyl)-phenyl]-1,2-dihydropyridine-3,5-dicarboxamide

RT (C₁₈, UV 220 nm): 5.5 min.
APCI-MS m/z: 484.1 [MH⁺].

### Example 108 N³-(2,3-Dihydro-1H-inden-2-yl)-N⁵,N⁵,6-trimethyl-2-oxo-1-[3-(trifluoromethyl)-phenyl]-1,2-dihydropyridine-3,5-dicarboxamide

RT (C₁₈, UV 220 nm): 5.4 min.
APCI-MS m/z: 484.1 [MH⁺].

### Example 109 N³-[2-(1,3-Benzodioxol-5-yl)ethyl]-N⁵,N⁵,6-trimethyl-2-oxo-1-[3-(trifluoromethyl)phenyl]-1,2-dihydropyridine-3,5-dicarboxamide

RT (C₁₈, UV 220 nm): 5.2 min.
APCI-MS m/z: 516.2 [MH⁺].

### Example 110 5-{[4-(2-Hydroxyethyl)piperazin-1-yl]carbonyl}-N,N,2-trimethyl-6-oxo-1-[3-(trifluoromethyl)phenyl]-1,6-dihydropyridine-3-carboxamide

RT (C₁₈, UV 220 nm): 3.1 min.
APCI-MS m/z: 481.3 [MH⁺].

### Example 111 N³-[(1-Ethylpyrrolidin-2-yl)methyl]-N⁵,N⁵,6-trimethyl-2-oxo-1-[3-(trifluoro-methyl)phenyl]-1,2-dihydropyridine-3,5-dicarboxamide

RT (C₁₈, UV 220 nm): 3.5 min.
APCI-MS m/z: 479.2 [MH⁺].

### Example 112 N⁵,N⁵,6-Trimethyl-N³-[3-(2-methylpiperidin-1-yl)propyl]-2-oxo-1-[3-(trifluoro-methyl)phenyl]-1,2-dihydropyridine-3,5-dicarboxamide

RT (C₁₈, UV 220 nm): 3.7 min.
APCI-MS m/z: 507.2 [MH⁺].

### Example 113 N⁵,N⁵,6-Trimethyl-N³-(1-naphthylmethyl)-2-oxo-1-[3-(trifluoromethyl)phenyl]-1,2-dihydropyridine-3,5-dicarboxamide

RT (C₁₈, UV 220 nm): 5.7 min.
APCI-MS m/z: 508.1 [MH⁺].

### Example 114 N³-(1,3-Benzodioxol-5-ylmethyl)-N⁵,N⁵,6-trimethyl-2-oxo-1-[3-(trifluoromethyl)phenyl]-1,2-dihydropyridine-3,5-dicarboxamide

RT (C₁₈, UV 220 nm): 5.1 min.
APCI-MS m/z: 502.1 [MH⁺].

### Example 115 N³-(3,4-Difluorobenzyl)-N⁵,N⁵,6-trimethyl-2-oxo-1-[3-(trifluoromethyl)phenyl]-1,2-dihydropyridine-3,5-dicarboxamide

RT (C₁₈, UV 220 nm): 5.4 min.
APCI-MS m/z: 494.2 [MH⁺].

### Example 116 N³-(2-Chloro-4-fluorobenzyl)-N⁵,N⁵,6-trimethyl-2-oxo-1-[3-(trifluoromethyl)-phenyl]-1,2-dihydropyridine-3,5-dicarboxamide

RT (C₁₈, UV 220 nm): 5.6 min.
APCI-MS m/z: 510.1 [MH⁺].

### Example 117 N⁵,N⁵,6-Trimethyl-2-oxo-N³-(2-thienylmethyl)-1-[3-(trifluoromethyl)phenyl]-1,2-dihydropyridine-3,5-dicarboxamide

RT (C₁₈, UV 220 nm): 5.0 min.
APCI-MS m/z: 464.2 [MH⁺].

### Example 118 N³-(3,4-Dichlorobenzyl)-N⁵,N⁵,6-trimethyl-2-oxo-1-[3-(trifluoromethyl)phenyl]-1,2-dihydropyridine-3,5-dicarboxamide

RT (C₁₈, UV 220 nm): 5.9 min.
APCI-MS m/z: 526.1 [MH⁺].

### Example 119 N³-[2-(2,4-Dichlorophenyl)ethyl]-N⁵,N⁵,6-trimethyl-2-oxo-1-[3-(trifluoromethyl)-phenyl]-1,2-dihydropyridine-3,5-dicarboxamide

RT (C₁₈, UV 220 nm): 6.0 min.
APCI-MS m/z: 540.1 [MH⁺].

### Example 120 N³-(2-Cyclohex-1-en-1-ylethyl)-N⁵,N⁵,6-trimethyl-2-oxo-1-[3-(trifluoromethyl)-phenyl]-1,2-dihydropyridine-3,5-dicarboxamide

RT (C₁₈, UV 220 nm): 5.7 min.
APCI-MS m/z: 476.2 [MH⁺].

### Example 121 N³-[1-(4-Chlorophenyl)ethyl]-N⁵,N⁵,6-trimethyl-2-oxo-1-[3-(trifluoromethyl)-phenyl]-1,2-dihydropyridine-3,5-dicarboxamide

RT (C₁₈, UV 220 nm): 5.8 min.
APCI-MS m/z: 506.2 [MH⁺].

### Example 122 N⁵,N⁵,6-Trimethyl-2-oxo-N³-[3-(2-oxopyrrolidin-1-yl)propyl]-1-[3-(trifluoro-methyl)phenyl]-1,2-dihydropyridine-3,5-dicarboxamide

RT (C₁₈, UV 220 nm): 4.0 min.
APCI-MS m/z: 493.3 [MH⁺].

### Example 123 N⁵,N⁵,6-Trimethyl-2-oxo-N³-(pyridin-4-ylmethyl)-1-[3-(trifluoromethyl)phenyl]-1,2-dihydropyridine-3,5-dicarboxamide

RT (C₁₈, UV 220 nm): 3.3 min.
APCI-MS m/z: 459.2 [MH⁺].

### Example 124 N,N,2-Trimethyl-6-oxo-5-[(4-phenylpiperazin-1-yl)carbonyl]-1-[3-(trifluoro-methyl)phenyl]-1,6-dihydropyridine-3-carboxamide

RT (C₁₈, UV 220 nm): 4.1 min.
APCI-MS m/z: 513.2 [MH⁺].

### Example 125 N,N,2-Trimethyl-6-oxo-5-[(4-pyridin-2-ylpiperazin-1-yl)carbonyl]-1-[3-(trifluoro-methyl)phenyl]-1,6-dihydropyridine-3-carboxamide

RT (C₁₈, UV 220 nm): 3.2 min.
APCI-MS m/z: 514.3 [MH⁺].

### Example 126 N³-(2,3-Dihydro-1-benzofuran-5-ylmethyl)-N⁵,N⁵,6-trimethyl-2-oxo-1-[3-(trifluoromethyl)phenyl]-1,2-dihydropyridine-3,5-dicarboxamide

RT (C₁₈, UV 220 nm): 5.1 min.
APCI-MS m/z: 500.2 [MH⁺].

### Example 127 Methyl 4-{[({5-[(dimethylamino)carbonyl]-6-methyl-2-oxo-1-[3-(trifluoromethyl)phenyl]-1,2-dihydropyridin-3-yl}carbonyl)amino]methyl}benzoate

RT (C₁₈, UV 220 nm): 5.1 min.
APCI-MS m/z: 516.2 [MH⁺].

### Example 128 5-{[3-(Dimethylamino)pyrrolidin-1-yl]carbonyl}-N,N,2-trimethyl-6-oxo-1-[3-(trifluoromethyl)phenyl]-1,6-dihydropyridine-3-carboxamide

RT (C₁₈, UV 220 nm): 3.1 min.
APCI-MS m/z: 465.2 [MH⁺].

### Example 129 N⁵,N⁵,6-Trimethyl-2-oxo-N³-[2-(2-thienyl)ethyl]-1-[3-(trifluoromethyl)phenyl]-1,2-dihydropyridine-3,5-dicarboxamide

RT (C₁₈, UV 220 nm): 5.2 min.
APCI-MS m/z: 478.1 [MH⁺].

### Example 130 N⁵,N⁵,6-Trimethyl-2-oxo-N³-(4-phenoxybenzyl)-1-[3-(trifluoromethyl)phenyl]-1,2-dihydropyridine-3,5-dicarboxamide

RT (C₁₈, UV 220 nm): 6.0 min.
APCI-MS m/z: 550.3 [MH⁺].

### Example 131 N⁵,N⁵,6-Trimethyl-2-oxo-N³-(3-thienylmethyl)-1-[3-(trifluoromethyl)phenyl]-1,2-dihydropyridine-3,5-dicarboxamide

RT (C₁₈, UV 220 nm): 5.0 min.
APCI-MS m/z: 464.2 [MH⁺].

### Example 132 N³-[2-(4-tert-Butylphenyl)ethyl]-N⁵,N⁵,6-trimethyl-2-oxo-1-[3-(trifluoromethyl)-phenyl]-1,2-dihydropyridine-3,5-dicarboxamide

RT (C₁₈, UV 220 nm): 6.3 min.
APCI-MS m/z: 528.2 [MH⁺].

### Example 133 N³-{2-[4-(Aminosulfonyl)phenyl]ethyl}-N⁵,N⁵,6-trimethyl-2-oxo-1-[3-(trifluoro-methyl)phenyl]-1,2-dihydropyridine-3,5-dicarboxamide

RT (C₁₈, UV 220 nm): 4.4 min.
APCI-MS m/z: 551.2 [MH⁺].

### Example 134 N⁵,N⁵,6-Trimethyl-2-oxo-N³-[4-(1H-pyrazol-1-yl)benzyl]-1-[3-(trifluoromethyl)-phenyl]-1,2-dihydropyridine-3,5-dicarboxamide

RT (C₁₈, UV 220 nm): 5.0 min.
APCI-MS m/z: 524.2 [MH⁺].

### Example 135 N⁵,N⁵,6-Trimethyl-2-oxo-N³-phenoxy-1-[3-(trifluoromethyl)phenyl]-1,2-dihydro-pyridine-3,5-dicarboxamide

RT (C₁₈, UV 220 nm): 5.1 min.
APCI-MS m/z: 460.1 [MH⁺].

### Example 136 N³-(2,3-Dihydro-1,4-benzodioxin-2-ylmethyl)-N⁵,N⁵,6-trimethyl-2-oxo-1-[3-(trifluoromethyl)phenyl]-1,2-dihydropyridine-3,5-dicarboxamide

RT (C₁₈, UV 220 nm): 5.4 min.
APCI-MS m/z: 516.2 [MH⁺].

### Example 137 N³-[(6-Fluoro-4H-1,3-benzodioxin-8-yl)methyl]-N⁵,N⁵,6-trimethyl-2-oxo-1-[3-(trifluoromethyl)phenyl]-1,2-dihydropyridine-3,5-dicarboxamide

RT (C₁₈, UV 220 nm): 5.2 min.
APCI-MS m/z: 534.2 [MH⁺].

### Example 138 N³-(1-Benzothien-3-ylmethyl)-N⁵,N⁵,6-trimethyl-2-oxo-1-[3-(trifluoromethyl)-phenyl]-1,2-dihydropyridine-3,5-dicarboxamide

RT (C₁₈, UV 220 nm): 5.6 min.
APCI-MS m/z: 514.1 [MH⁺].

### Example 139 N⁵,N⁵,6-Trimethyl-2-oxo-N³-[2-(tetrahydro-2H-pyran-4-yl)ethyl]-1-[3-(trifluoro-methyl)phenyl]-1,2-dihydropyridine-3,5-dicarboxamide

RT (C₁₈, UV 220 nm): 4.5 min.
APCI-MS m/z: 480.2 [MH⁺].

### Example 140 N⁵,N⁵,6-Trimethyl-N³-[(1-methyl-1H-pyrazol-4-yl)methyl]-2-oxo-1-[3-(trifluoro-methyl)phenyl]-1,2-dihydropyridine-3,5-dicarboxamide

RT (C₁₈, UV 220 nm): 4.0 min.
APCI-MS m/z: 462.2 [MH⁺].

### Example 141 N⁵,N⁵,6-Trimethyl-2-oxo-N³-[(1-phenyl-1H-pyrazol-4-yl)methyl]-1-[3-(trifluoro-methyl)phenyl]-1,2-dihydropyridine-3,5-dicarboxamide

RT (C₁₈, UV 220 nm): 5.1 min.
APCI-MS m/z: 524.2 [MH⁺].

### Example 142 N³-[(5-Methoxy-4-oxo-4H-pyran-2-yl)methyl]-N⁵,N⁵,6-trimethyl-2-oxo-1-[3-(trifluoromethyl)phenyl]-1,2-dihydropyridine-3,5-dicarboxamide

RT (C₁₈, UV 220 nm): 3.9 min.
APCI-MS m/z: 506.2 [MH⁺].

### Example 143 N³-(3-Azepan-1-ylpropyl)-N⁵,N⁵,6-trimethyl-2-oxo-1-[3-(trifluoromethyl)phenyl]-1,2-dihydropyridine-3,5-dicarboxamide

RT (C₁₈, UV 220 nm): 3.7 min.
APCI-MS m/z: 507.2 [MH⁺].

### Example 144 N³-(4-Cyanobenzyl)-N⁵,N⁵,6-trimethyl-2-oxo-1-[3-(trifluoromethyl)phenyl]-1,2-dihydropyridine-3,5-dicarboxamide

RT (C₁₈, UV 220 nm): 5.0 min.
APCI-MS m/z: 483.2 [MH⁺].

### Example 145 N⁵,N⁵,6-Trimethyl-2-oxo-N³-[3-(5-oxo-4,5-dihydro-1H-pyrazol-4-yl)propyl]-1-[3-(trifluoromethyl)phenyl]-1,2-dihydropyridine-3,5-dicarboxamide

RT (C₁₈, UV 220 nm): 3.8 min.
APCI-MS m/z: 492.2 [MH⁺].

### Example 146 N³-{[(2R)-1-Ethylpyrrolidin-2-yl]methyl}-N⁵,N⁵,6-trimethyl-2-oxo-1-[3-(trifluoro-methyl)phenyl]-1,2-dihydropyridine-3,5-dicarboxamide

RT (C₁₈, UV 220 nm): 3.4 min.
APCI-MS m/z: 479.2 [MH⁺].

### Example 147 5-Cyclopropyl-6-methyl-N-[4-(methylsulfonyl)benzyl]-2-oxo-1-[3-(trifluoromethyl)phenyl]-1,2-dihydropyridine-3-carboxamide

A mixture of 5-iodo-6-methyl-N-[4-(methylsulfonyl)benzyl]-2-oxo-1-[3-(trifluoromethyl)phenyl]-1,2-dihydropyridine-3-carboxamide (Example 1 (d), 120.4 mg, 0.20 mmol), cyclopropylboronic acid (48.7 mg, 0.57 mmol), potassium phosphate (195.3 mg, 0.85 mmol), tricyclohexylphosphine (19.9 mg, 0.07 mmol) and palladium acetate (8.5 mg, 0.03 mmol) in toluene (4 ml) and water (0.08 ml) was stirred at 100 °C under an argon atmosphere for 2 h. The reaction mixture was cooled and concentrated in vacuo. The residue was purified by preparative HPLC to give the title compound as a white solid (36 mg, 35%).
¹H NMR (CDCl₃): δ 10.06 (1H, t, J 5.9 Hz); 8.48 (1H, s); 7.87 (2H, d, J 8.4 Hz); 7.81 (1H, d, J 7.8 Hz); 7.74 (1H, t, J 7.8 Hz); 7.51 (3H, d, J 8.4 Hz); 7.42 (1H, d, J 7.7 Hz); 4.67 (2H, m); 3.01 (3H, s); 2.20 (3H, s); 1.76 (1H, dd, J 13.6 3.0 Hz); 0.99 (2H, m); 0.70 (2H, q, J 5.1 Hz).
APCI-MS m/z: 505.1 [MH⁺].

### Example 148 6-Methyl-5-(2-methyl-1,3-dioxolan-2-yl)-N-[4-(methylsulfonyl)benzyl]-2-oxo-1-[3-(trifluoromethyl)phenyl]-1,2-dihydropyridine-3-carboxamide

5-Acetyl-6-methyl-*N*-[4-(methylsulfonyl)benzyl]-2-oxo-1-[3-(trifluoromethyl)phenyl]-1,2-dihydropyridine-3-carboxamide (Example 4, 55 mg, 0.11 mmol), ethane-1,2-diol (482 mg, 8.7 mmol), toluene-4-sulfonic acid monohydrate (21 mg, 0.11 mmol) and toluene (50 ml) were placed in a round-bottomed flask (100 ml) equipped with a Dean and Stark water separator, reflux condenser and magnetic stirrer. The reaction mixture was heated at reflux for 48 h. After cooling, the mixture was neutralized with aqueous sodium hydrogen carbonate solution and the solvent was removed in vacuo. The residue was purified by preparative HPLC to give the title compound as a white solid (31.5 mg, 52 %).
¹H NMR (CDCl₃): δ 9.99 (1H, t); 8.90 (1H, s); 7.87 (2H, d); 7.81 (1H, d); 7.74 (1H, t); 7.52 (2H, d); 7.50 (1H, s); 7.42 (1H, d); 4.67 (2H, m); 4.07 (2H, m); 3.85 (2H, m); 3.01 (3H, s); 2.23 (3H, s); 1.67 (3H, s).
APCI-MS m/z: 551.0 [MH⁺].

### Example 149 5-(4,5-Dihydro-oxazol-2-yl)-6-methyl-2-oxo-1-(3-trifluoromethyl-phenyl)-1,2-dihydro-pyridine-3-carboxylic acid 4-methanesulfonyl-benzylamide

The compound obtained in Example 95 (0.022 g, 0.04 mmol) was dissolved in dry DCM (2 ml). To this solution was added one drop of SOCl₂, and the mixture was allowed to stand for 1 h. LC-MS showed complete consumption of the starting material. Evaporation and subsequent purification on preparative HPLC afforded the title compound 0.004 g (20%) as a white solid after freeze-drying the pure fractions.
¹H NMR (DMSO-d*₆*): δ 9.74 (1H, t, *J* 6.1 Hz); 8.78 (1H, s); 7.96 (1H, s); 7.91 (1H, d, *J* 7.6 Hz); 7.86 (2H, d, *J* 8.2 Hz); 7.83 (1H, t, *J* 7.8 Hz); 7.76 (1H, d, *J* 7.8 Hz); 7.54 (2H, d, *J* 8.2 Hz); 4.65-4.55 (2H, m); 4.38 (2H, t, *J* 9.2 Hz); 3.99 (2H, t, *J* 9.2 Hz); 3.17 (3H, s); 2.40 (3H, s).
APCI-MS m/z: 534.1 [MH⁺].

### Example 150 5-Cyclopropyl-6-methyl-N-{[5-(methylsulfonyl)pyridin-2-yl]methyl}-2-oxo-1-[3-(trifluoromethyl)phenyl]-1,2-dihydropyridine-3-carboxamide

### a) 5-Cyclopropyl-6-methyl-2-oxo-1-[3-(trifluoromethyl)phenyl]-1,2-dihydropyridine-3-carboxylic acid

Ethyl 5-iodo-6-methyl-2-oxo-1-[3-(trifluoromethyl)phenyl]-1,2-dihydropyridine-3-carboxylate (Example 99 (b), 0.77 g, 1.6 mmol), toluene (35 ml), cyclopropyl-boronic acid (0.257 g, 3 mmol), Pd(OAc)₂ (0.072 g, 0.35 mmol), P(c-Hex)₃ (0.169 g, 0.6 mmol), K₃PO₄ monohydrate (1.6 g, 6.9 mmol), water (0.7 ml) and a magnetic stirrer bar were placed in a pressure safe glass vessel. The vessel was sealed and heated (130 °C) with stirring overnight. LC-MS showed complete formation of the intermediate ethyl ester. The mixture was allowed to cool and the phases were diluted with EtOAc (50 ml) and water (50 ml) and the phases were allowed to separate. The organic phase was washed with water and brine, and further dried with Na₂SO₄. Filtration and evaporation gave a crude intermediate. This material was dissolved in THF (10 ml) and water (5 ml). To this solution was added NaOH (1M, 3 ml, 3 mmol) and the mixture was stirred for 2 h at 50 °C. The THF was evaporated and the residual aqueous phase was acidified and extracted with EtOAc (2 x 20 ml). The extracts were washed with water and brine and finally dried over Na₂SO₄. Filtration and evaporation gave the required carboxylic acid (0.19 g, 33%).
APCI-MS m/z: 338.1 [MH⁺]. Retention time 2.35 minutes.

### b) 5-(Methylthio)pyridine-2-carbonitrile

5-Bromo-pyridine-2-carbonitrile (2.63 g, 13.7mmol), sodium methanethiolate (1.44 g, 20.5 mmol), potassium carbonate (3.79 g, 27.4 mmol) in NMP (60 ml) were stirred in a sealed flask overnight. The mixture was partitioned between ethyl acetate and water. The organic phase was washed with water several times, brine and dried over sodium sulphate. The solvent was removed in vacuo to afford the title compound as a yellow solid (2.0 g, 99%).
¹H NMR (CD₃OD): δ 8.54 (1H, d, *J* 2.3 Hz); 7.83 - 7.71 (2H, m); 2.60 (3H, s).

### c) 5-(Methylsulfonyl)pyridine-2-carbonitrile

5-(Methylthio)pyridine-2-carbonitrile (2.0 g, 13.3 mmol) was dissolved in DCM (20 ml) and cooled to -15 °C and 3-chloroperoxybenzoic acid (6.75 g, 27.4 mmol) was added in portions while the temperature was kept between -15 °C to -10 °C. When the addition was complete, the cooling bath was removed and the mixture was stirred at room temperature for 2 h. 2M KOH and DCM were added. The organic phase was separated, washed twice with 2M KOH, water and brine, dried over sodium sulphate and evaporated to afford the title compound as a white solid (2.15 g, 89%).
¹H NMR (CD₃OD): δ 9.22 (1H, d, J 2.3 Hz); 8.54 (1H, dd, J 8.1, 2.3 Hz); 8.13 (1H, d, J 8.3 Hz); 3.27 (3H, s).

### d) {[5-(Methylsulfonyl)pyridin-2-yl]methyl}amine hydrochloride

5-(Methylsulfonyl)pyridine-2-carbonitrile (2.15 g, 11.8 mmol) was dissolved in methanol (230 ml). 6M HCl (1 ml) and 10% palladium on carbon (234 mg) were added and the mixture was stirred under an atmospheric pressure of hydrogen overnight. The catalyst was removed by filtration through celite and the solvent was evaporated, water was added and the solution was freeze-dried to afford the title compound as a yellow powder (2.34 g, 89%).
¹H NMR (CD₃OD): δ 9.10 (1H, d, J 2.2 Hz); 8.36 (1H, dd, J 8.2, 2.4 Hz); 7.68 (1H, d, J 8.8 Hz); 4.29 (2H, s); 3.22 (3H, s).

### e) 5-Cyclopropyl-6-methyl-N-{[5-(methylsulfonyl)pyridin-2-yl]methyl}-2-oxo-1-[3-(trifluoromethyl)phenyl]-1,2-dihydropyridine-3-carboxamide

5-Cyclopropyl-6-methyl-2-oxo-1-[3-(trifluoromethyl)phenyl]-1,2-dihydropyridine-3-carboxylic acid, {[5-(methylsulfonyl)pyridin-2-yl]methyl}amine hydrochloride, HBTU and DIEA in NMP were reacted together overnight. Purification by HPLC gave the title compound.
¹H NMR (DMSO): δ 10.14 (1H, t, J 5.8 Hz); 8.98 (1H, d, J 2.3 Hz); 8.26 (1H, dd, J 8.2, 2.4 Hz); 8.18 (1H, s); 7.94 - 7.79 (3H, m); 7.74 - 7.68 (1H, m); 7.55 (1H, d, J 8.4 Hz); 4.69 (2H, d, J 5.9 Hz); 3.29 (3H, s); 2.13 (3H, s); 1.90 - 1.78 (1H, m); 0.99 - 0.90 (2H, m); 0.64 - 0.56 (2H, m).
APCI-MS m/z: 506.1 [MH⁺].

### Screen

### Human Neutrophil Elastase Quenched-FRET Assay

The assay uses Human Neutrophil Elastase (HNE) purified from serum (Calbiochem art. 324681; Ref. Baugh, R.J. et al., 1976, Biochemistry. 15, 836-841). HNE was stored in 50 mM NaOAc, 200 mM NaCl, pH 5.5 with added 30% glycerol at -20 °C. The protease substrate used was Elastase Substrate V Fluorogenic, MeOSuc-AAPV-AMC (Calbiochem art. 324740; Ref. Castillo, M.J. et al., 1979, Anal. Biochem. 99, 53-64). The substrate was stored in DMSO at -20 °C. The assay additions were as follows: Test compounds and controls were added to black 96-well flat-bottom plates (Greiner 655076), 1 µL in 100% DMSO, followed by 30 µL HNE in assay buffer with 0.01% TritonX-100. The assay buffer constitution was: 100 mM Tris (pH 7.5) and 500 mM NaCl. The enzyme and the compounds were incubated at room temperature for 15 minutes. Then 30 µl substrate in assay buffer was added. The assay was stopped after 30 minutes incubation at room temperature by adding 60 µl stop solution (140 mM acetic acid, 200 mM sodium monochloroacetate, 60 mM sodium acetate, pH 4.3). Fluorescence was measured on a Wallac 1420 Victor 2 instrument at settings: Excitation 380 nm, Emission 460 nm. IC₅₀ values were determined using Xlfit curve fitting using model 205.

When tested in the above screen, the compounds of the Examples gave IC₅₀ values for inhibition of human neutrophil elastase activity of less than 30 µM, indicating that the compounds of the invention are expected to possess useful therapeutic properties. Specimen results are shown in the following Table:

| Compound | Inhibition of Human Neutrophil Elastase IC₅₀ (nM) |
|---|---|
| Example 8 | 83 |
| Example 16 | 48 |
| Example 19 | 99 |
| Example 58 | 51 |
| Example 66 | 22 |

## Claims

1. A compound of formula (I) 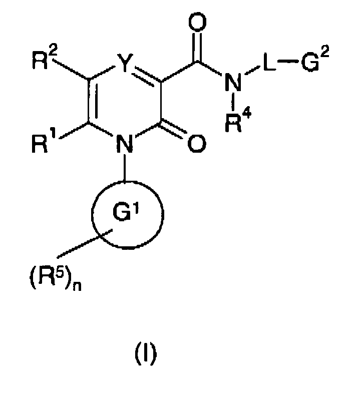 wherein:
**Y** represents CR³or N;
**R¹** represents H or C1 to 6 alkyl;
**R²** represents:
i) CN, NO₂, OH, OSO₂R⁴⁷, O-C2 to 6 alkanoyl, CO₂R⁴⁷, CHO or C2 to 6 alkanoyl; or
ii) C1 to 6 alkoxy optionally substituted by OH, C1 to 6 alkoxy, CN, NR⁵⁴R⁵⁵, CONR⁵⁴R⁵⁵, OCOR⁴⁷ or one or more F atoms; or
iii) C3 to 6 saturated or partially unsaturated cycloalkyl optionally further substituted by C1 to 6 alkyl; or
iv) C4 to 7 saturated or partially unsaturated heterocyclic ring containing 1 to 3 heteroatoms independently selected from O, S(O)ₘ and NR⁶² optionally further substituted by C 1 to 6 alkyl; or
v) CONR⁴⁸R⁴⁹, CONR⁵⁰NR⁴⁸R⁴⁹, C(=NOR⁵²)R⁵³, C(=NH)NHOR⁵² or NR⁴⁸R⁴⁹; or
vi) C2 to 6 alkenyl or C2 to 6 alkynyl; said alkenyl or alkynyl group being optionally further substituted by C1 to 6 alkoxy or phenyl or a five- or six-membered heteroaromatic ring containing 1 to 3 heteroatoms independently selected from O, S and N; said phenyl or heteroaromatic ring being optionally further substituted by halogen, CN, C1 to 6 alkyl or C1 to 6 alkoxy; or
vii)C1 to 6 alkyl substituted by one or more F atoms; or
viii) C1 to 6 alkyl substituted by one or more groups selected from halogen, OH, oxo, azido, NR⁴⁸R⁴⁹, C1 to 6 alkoxy and C1 to 6 alkoxy substituted by one or more F atoms; or
ix) C1 to 6 alkyl substituted by phenyl or a five- or six-membered heteroaromatic ring containing 1 to 3 heteroatoms independently selected from O, S and N; said phenyl or heteroaromatic ring being optionally further substituted by halogen, CN, C1 to 6 alkyl or C 1 to 6 alkoxy;
**R⁴⁸** and **R⁴⁹** independently represent H, OH, C1 to 6 alkoxy, C3 to 6 cycloalkyl, CHO, C2 to 6 alkanoyl, CO₂R⁵⁰, C(X)NR⁶³R⁶⁴ or C1 to 6 alkyl; said alkyl being optionally further substituted by OH, C1 to 4 alkoxy, C3 to 6 cycloalkyl, CN or phenyl or a five- or six-membered heteroaromatic ring containing 1 to 3 heteroatoms independently selected from O, S and N; said alkanoyl being optionally further substituted by CN;
**X** represent O or S;
or the group **NR⁴⁸R⁴⁹** together represents a saturated or partially unsaturated 5 to 7 membered azacyclic ring optionally incorporating one further heteroatom selected from O, S and NR⁵⁶; said azacyclic ring being optionally further substituted by one or more substituents selected from OR⁵⁷ and C1 to 4 alkyl; said alkyl being optionally further substituted by OR⁵⁷;
**R³** represents H or F;
**G¹** represents phenyl or a five- or six-membered heteroaromatic ring containing 1 to 3 heteroatoms independently selected from O, S and N;
**R⁵** represents H, halogen, C1 to 6 alkyl, CN, C1 to 6 alkoxy, NO₂, NR¹⁴R¹⁵, C1 to 3 alkyl substituted by one or more F atoms or C1 to 3 alkoxy substituted by one or more F atoms;
**R¹⁴** and **R¹⁵** independently represent H or C1 to 3 alkyl; said alkyl being optionally further substituted by one or more F atoms;
n represents an integer 1, 2 or 3 and when n represents 2 or 3, each R⁵ group is selected independently;
**R⁴** represents H or C1 to 6 alkyl: said alkyl being optionally further substituted by OH or C 1 to 6 alkoxy;
or **R⁴** and **L** are joined together such that the group **-NR⁴L** represents a 5 to 7 membered azacyclic ring optionally incorporating one further heteroatom selected from O, S and NR¹⁶; said ring being optionally further substituted by C1 to 6 alkyl or NR⁶⁰R⁶¹; said alkyl being optionally further substituted by OH;
**L** represents a bond, O, NR²⁹ or C1 to 6 alkyl; said alkyl optionally incorporating a heteroatom selected from O, S and NR¹⁶; and said alkyl being optionally further substituted by OH or OMe;
**G²** represents a monocyclic ring system selected from:
i) phenyl or phenoxy,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group; or
**G²** represents a bicyclic ring system in which each of the two rings is independently selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group;
and the two rings are either fused together, or are bonded directly together or are separated by a linker group selected from O, S(O)_{q} or CH₂,
said monocyclic or bicyclic ring system being optionally further substituted by one to three substituents independently selected from CN, OH, C 1 to 6 alkyl, C1 to 6 alkoxy, halogen, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C(=NH)NH₂, S(O)ₛR²⁵, SO₂NR²⁶R²⁷, C1 to 3 alkoxy substituted by one or more F atoms and C1 to 3 alkyl substituted by SO₂R³⁹ or by one or more F atoms; or
when L does not represent an bond, **G²** may also represent H;
at each occurrence, **m, p, q, s** and **t** independently represent an integer 0, 1 or 2;
**R¹⁸** and **R¹⁹** independently represent H, C1 to 6 alkyl, formyl, C2 to 6 alkanoyl, S(O)ₜR³² or SO₂NR³³R³⁴; said alkyl group being optionally further substituted by halogen, CN, C1 to 4 alkoxy or CONR⁴¹R⁴²;
**R²⁵** represents H, C1 to 6 alkyl or C3 to 6 cycloalkyl; said alkyl group being optionally further substituted by one or more substituents selected independently from OH, CN, CONR³⁵R³⁶, CO₂R³⁷, OCOR⁴⁰, C3 to 6 cycloalkyl, a C4 to 7 saturated heterocyclic ring containing one or two heteroatoms independently selected from O, S(O)ₚ and NR⁴³ and phenyl or a 5 or 6 membered heteroaromatic ring containing one to three heteroatoms independently selected from O, S and N; said aromatic ring being optionally further substituted by one or more substituents selected independently from halogen, CN, C 1 to 4 alkyl, C1 to 4 alkoxy, OH, CONR⁴⁴R⁴⁵, CO₂R⁴⁶, S(O)ₛR⁶⁵ and NHCOCH₃;
**R³²** represents H, C1 to 6 alkyl or C3 to 6 cycloalkyl;
**R¹⁶, R¹⁷, R²⁰, R²¹, R²², R²³, R²⁴, R²⁶, R²⁷, R²⁹, R³¹, R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁵⁰, R⁵², R⁵³, R⁵⁴, R⁵⁵, R⁵⁶, R⁵⁷, R⁶⁰, R⁶¹, R⁶², R⁶³, R⁶⁴ and R⁶⁵** independently represent H or C1 to 6 alkyl;
and pharmaceutically acceptable salts thereof.

2. A compound of formula (I), according to Claim 1, wherein Y represents CR³.

3. A compound of formula (I), according to Claim 1 or Claim 2, wherein G¹ represents phenyl.

4. A compound of formula (I), according to any one of Claims 1 to 3, wherein R⁵ represents Cl, CH₃, CN or CF₃.

5. A compound of formula (I), according to any one of Claims 1 to 4, or a pharmaceutically acceptable salt thereof, for use as a medicament.

6. A pharmaceutical formulation comprising a compound of formula (I), as defined in any one of Claims 1 to 4, or a pharmaceutically acceptable salt thereof, optionally in admixture with a pharmaceutically acceptable diluent or carrier.

7. The use of a compound of formula (I) as defined in any one of Claims 1 to 4, or a pharmaceutically acceptable salt thereof, in the manufacture of a medicament for the treatment or prophylaxis of adult respiratory distress syndrome (ARDS), cystic fibrosis, pulmonary emphysema, chronic obstructive pulmonary disease (COPD), pulmonary hypertension, asthma, rhinitis, ischemia-reperfusion injury, rheumatoid arthritis, osteoarthritis, cancer, atherosclerosis or gastric mucosal injury.

8. The use of a compound of formula (I) as defined in any one of Claims 1 to 4, or a pharmaceutically acceptable salt thereof, in the manufacture of a medicament for the treatment or prophylaxis of inflammatory diseases or conditions.

9. A process for the preparation of a compound of formula (I), as defined in any one of Claims 1 to 4, and optical isomers, racemates and tautomers thereof and pharmaceutically acceptable salts thereof, which comprises:
a) reacting a compound of formula (II) 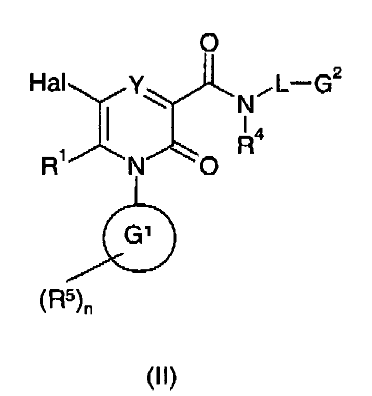 with a nucleophilic equivalent of R², such as Cu(I)CN, an alkyl vinyl ether, an organo-tin compound, an organo boronic acid, a terminal alkyne or an alcohol and carbon monoxide; wherein R¹, R², R⁴, R⁵, Y, G¹, G², L and n are as defined in formula (I) and Hal represents a halogen atom, preferably bromo or iodo; or
b) reacting a compound of formula (XV) 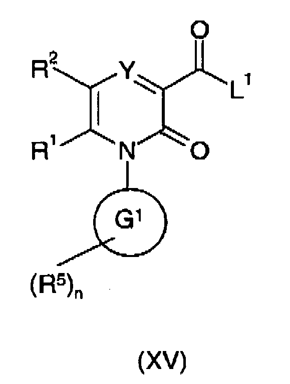
wherein R¹, R², R⁵, n, G¹ and Y arc as defined in formula (I) and L¹ represents a leaving group,
with a compound of formula (IX) or a salt thereof 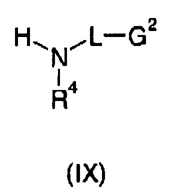 wherein R⁴, G² and L are as defined in formula (I);
and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

## Patentansprüche

1. Verbindungen der Formel (I) 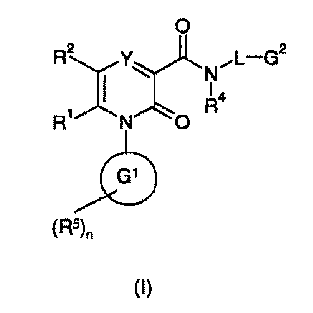 wobei:
**Y** für CR³ oder N steht;
**R¹** für H oder C1-6-Alkyl steht;
**R²** für:
i) CN, NO₂, OH, OSO₂R⁴⁷, O-C2-6-Alkanoyl, CO₂R⁴⁷, CHO oder C2-6-Alkanoyl; oder
ii) gegebenenfalls durch OH, C1-6-Alkoxy, CN, NR⁵⁴R⁵⁵, CONR⁵⁴R⁵⁵, OCOR⁴⁷ oder einen oder mehrere F-Atome substituiertes C1-6-Alkoxy; oder
iii) gegebenenfalls weiter durch C1-6-Alkyl substituiertes, gesättigtes oder teilweise ungesättigtes C3-6-Cycloalkyl; oder
iv) einen gegebenenfalls weiter durch C1-6-Aklyl substituierten, gesättigten oder teilweise ungesättigten heterocyclischen C4-7-Ring mit 1 bis 3 Heteroatomen unabhängig voneinander ausgewählt aus O, S(O)ₘ und NR⁶²; oder
v) CONR⁴⁸R⁴⁹, CONR⁵⁰NR⁴⁸R⁴⁹, C(=NOR⁵²)R⁵³, C(=NH)NHOR⁵² oder NR⁴⁸R⁴⁹; oder
vi) C2-6-Alkenyl oder C2-6-Alkinyl; wobei die Alkenyl- bzw. Alkinylgruppe gegebenenfalls weiter substituiert ist durch C1-6-Alkoxy oder Phenyl oder einen fünf- oder sechsgliedrigen heteroaromatischen Ring mit 1 bis 3 unabhängig voneinander aus O, S und N ausgewählten Heteroatomen; wobei dieses Phenyl bzw. dieser heteroaromatische Ring gegebenenfalls weiter substituiert ist durch Halogen, CN, C1-6-Alkyl oder C1-6-Alkoxy; oder
vii) durch einen oder mehrere F-Atome substituiertes C1-6-Alkyl; oder
viii) durch einen oder mehrere Gruppen ausgewählt aus Halogen, OH, Oxo, Azido, NR⁴⁸R⁴⁹, C1-6-Alkoxy und durch ein oder mehrere F-Atome substituiertes C1-6-Alkoxy substituiertes C1-6-Alkyl; oder
ix) durch Phenyl oder durch einen fünf- oder sechsgliedrigen heteroaromatischen Ring mit 1 bis 3 Heteroatomen unabhängig ausgewählt aus O, S und N substituiertes C1-6-Alkyl; wobei das Phenyl bzw. der heteroaromatische Ring gegebenenfalls weiter substituiert ist durch Halogen, CN, C1-6-Alkyl oder C1-6-Alkoxy, steht;
**R⁴⁸** und **R⁴⁹**unabhängig voneinander für H, OH, C1-6-Alkoxy, C3-6-Cycloalkyl, CHO, C2-6-Alkanoyl, CO₂R⁵⁰, C(X)NR⁶³R⁶⁴ oder C1-6-Alkyl stehen; wobei dieses Alkyl gegebenenfalls weiter substituiert ist durch OH, C1-4-Alkoxy, C3-6-Cycloalkyl, CN oder Phenyl oder einen fünf- oder sechsgliedrigen heteroaromatischen Ring mit 1 bis 3 Heteroatomen unabhängig voneinander ausgewählt aus O, S und N; wobei Alkanoyl gegebenenfalls weiter substituiert sein kann durch CN;
**X** für O oder S steht;
oder die Gruppe **NR⁴⁸R⁴⁹** zusammen für einen gesättigten oder teilweise ungesättigten fünf- bis siebengliedrigen azacyclischen Ring steht, der gegebenenfalls ein weiteres Heteroatom ausgewählt aus O, S und NR⁵⁶ enthält; wobei der azacyclische Ring gegebenenfalls weiter substituiert ist durch ein oder mehrere Substituenten ausgewählt aus OR⁵⁷ und C1-4-Alkyl; wobei das Alkyl gegebenenfalls weiter substituiert ist durch OR⁵⁷;
**R³** für H oder F steht;
**G¹** für Phenyl oder einen fünf- oder sechsgliedrigen heteroaromatischen Ring mit 1 bis 3 Heteroatomen unabhängig voneinander ausgewählt aus O, S und N steht;
**R⁵** für H, Halogen, C1-6-Alkyl, CN, C1-6-Alkoxy, NO₂, NR¹⁴R¹⁵, durch ein oder mehrere F-Atome substituiertes C1-3-Alkyl oder durch ein oder mehrere F-Atome substituiertes C1-3-Alkoxy steht;
**R¹⁴** und **R¹⁵** unabhängig voneinander für H oder C1-3-Alkyl stehen; wobei dieses Alkyl gegebenenfalls weiter durch ein oder mehrere F-Atome substituiert ist;
**n** für eine ganze Zahl 1, 2 oder 3 steht und, wenn n für 2 oder 3 steht, die R⁵-Gruppen jeweils unabhängig voneinander ausgewählt sind;
**R⁴** für H oder C1-6-Alkyl steht; wobei dieses Alkyl gegebenenfalls weiter durch OH oder C1-6-Alkoxy substituiert ist;
Oder **R⁴** und **L** miteinander verbunden sind, so daß die Gruppe **-NR⁴L** für einen fünf- bis siebengliedrigen azacyclischen Ring steht, der gegebenenfalls ein weiteres Heteroatom ausgewählt aus O, S und NR¹⁶ enthält; wobei dieser Ring gegebenenfalls weiter substituiert ist durch C1-6-Alkyl oder NR⁶⁰R⁶¹; wobei Alkyl gegebenenfalls weiter substituiert ist durch OH;
**L** für eine Bindung, O, NR²⁹ oder C1-6-Alkyl steht; wobei dieses Alkyl gegebenenfalls ein Heteroatom ausgewählt aus O, S und NR¹⁶ enthält; und wobei dieses Alkyl gegebenenfalls weiter substituiert ist durch OH oder OMe;
**G²** für ein monocyclisches Ringsystem ausgewählt aus:
i) Phenyl oder Phenoxy,
ii) einem fünf- oder sechsgliedrigen heteroaromatischen Ring mit einem bis drei Heteroatomen unabhängig voneinander ausgewählt aus O, S und N;
iii) gesättigtes oder teilweise ungesättigtes C3-6-Cycloalkyl, oder
iv) einen gesättigten oder teilweise ungesättigten heterocyclischen C4-7-Ring mit einem oder zwei Heteroatomen unabhängig voneinander ausgewählt aus O, S(O)ₚ oder NR¹⁷, der gegebenenfalls weiter eine Carbonylgruppe enthält, steht; oder
**G²** für ein bicyclisches Ringsystem steht, in welchem die beiden Ringe jeweils unabhängig voneinander ausgewählt sind aus:
i) Phenyl,
ii) einem fünf- oder sechsgliedrigen heteroaromatischen Ring mit einem bis drei Heteroatomen unabhängig voneinander ausgewählt aus O, S und N;
iii) gesättigtes oder teilweise ungesättigtes C3-6-Cycloalkyl, oder
iv) einen gesättigten oder teilweise ungesättigten heterocyclischen C4-7-Ring mit einem oder zwei Heteroatomen unabhängig voneinander ausgewählt aus O, S(O)ₚ oder NR¹⁷, der gegebenenfalls weiter eine Carbonylgruppe enthält;
und die beiden Ringe entweder miteinander kondensiert sind oder direkt aneinander gebunden sind oder über eine Linker-Gruppe, ausgewählt aus O, S(O)_{q} oder CH₂ voneinander getrennt sind;
wobei das monocyclische bzw. bicyclische Ringsystem gegebenenfalls weiter substituiert ist durch einen bis drei Substituenten unabhängig voneinander ausgewählt aus CN, OH, C1-6-Alkyl, C1-6-Alkoxy, Halogen, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C(=NH)NH₂, S(O)₅R²⁵, SO₂NR²⁶R²⁷, durch ein oder mehrere F-Atome substituiertes C1-3-Alkoxy und durch SO₂R³⁹ oder durch ein oder mehrere F-Atome substituiertes C1-3-Alkyl; oder
wenn L nicht für eine Bindung steht, **G²** auch für H stehen kann;
**m, p, q, s** und **t** jedesmal, wenn sie vorkommen, unabhängig voneinander für eine ganze Zahl 0, 1 oder 2 stehen;
**R¹⁸** und **R¹⁹** unabhängig voneinander für H, C1-6-Alkyl, Formyl, C2-6-Alkanoyl, S(O)ₜR³² oder SO₂NR³³R³⁴ stehen, wobei die Alkylgruppe gegebenenfalls weiter substituiert ist durch Halogen, CN, C1-4-Alkoxy oder CONR⁴¹R⁴²;
**R²⁵** für H, C1-6-Alkyl oder C3-6-Cycloalkyl steht; wobei die Alkylgruppe gegebenenfalls weiter substituiert ist durch einen oder mehrere Substituenten unabhängig voneinander ausgewählt aus OH, CN, CONR³⁵R³⁶, CO₂R³⁷, OCOR⁴⁰, C3-6-Cycloalkyl, einen gesättigten heterocyclischen C4-7-Ring mit einem oder zwei Heteroatomen unabhängig voneinander aus O, S(O)ₚ und NR⁴³ und Phenyl oder einem fünf- oder sechsgliedrigen heteroaromatischen Ring mit ein bis drei Heteroatomen unabhängig voneinander ausgewählt aus O, S und N; wobei der aromatische Ring gegebenenfalls weiter substituiert ist durch einen oder mehrere Substituenten unabhängig voneinander ausgewählt aus Halogen, CN, C1-4-Alkyl, C1-4-Alkoxy, OH, CONR⁴⁴R⁴⁵, CO₂R⁴⁶, S(O)_{S}R⁶⁵ und NHCOCH₃;
**R³²** für H, C1-6-Alkyl oder C3-6-Cycloalkyl steht;
**R¹⁶, R¹⁷, R²⁰, R²¹, R²², R²³, R²⁴, R²⁶, R²⁷, R²⁹, R³¹, R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁵⁰, R⁵², R⁵³, R⁵⁴, R⁵⁵, R⁵⁶, R⁵⁷, R⁶⁰, R⁶¹, R⁶², R⁶³, R⁶⁴ und R⁶⁵** unabhängig voneinander für H oder C1-6-Alkyl stehen;
und deren pharmazeutisch annehmbare Salze.

2. Verbindung der Formel (I) nach Anspruch 1, wobei Y für CR³ steht.

3. Verbindung der Formel (I) nach Anspruch 1 oder 2, wobei G¹ für Phenyl steht.

4. Verbindung der Formel (I) nach einem der Ansprüche 1 bis 3, wobei R⁵ für Cl, CH₃, CN oder CF₃ steht.

5. Verbindung der Formel (I) nach einem der Ansprüche 1 bis 4 und deren pharmazeutisch annehmbare Salze zur Verwendung als Medikament.

6. Pharmazeutische Formulierung, enthaltend eine wie in einem der Ansprüche 1 bis 4 definierte Verbindung der Formel (I) oder ein pharmazeutisch annehmbares Salz davon, gegebenenfalls als Mischung mit einem pharmazeutisch annehmbaren Verdünnungsmittel oder Träger.

7. Verwendung einer wie in einem der Ansprüche 1 bis 4 definierten Verbindung der Formel (I) oder eines pharmazeutisch annehmbaren Salzes davon bei der Herstellung eines Medikaments zur Behandlung oder Prophylaxe von akutem Atemnotsyndrom (Adult Respiratory Distress Syndrom, ARDS), cystischer Fibrose, Lungenemphysem, chronisch-obstruktiver Lungenerkrankung (Chronic Obstructive Pulmonary Disease, COPD), pulmonaler Hypertonie, Asthma, Rhinitis, ischämischer Reperfusionsverletzung, rheumatoider Arthritis, Osteoarthritis, Krebs, Atherosklerose oder Verletzung der Magenschleimhaut.

8. Verwendung einer wie in einem der Ansprüche 1 bis 4 definierten Verbindung der Formel (I) oder eines pharmazeutisch annehmbaren Salzes davon bei der Herstellung eines Medikaments zur Behandlung oder Prophylaxe von entzündlichen Krankheiten bzw. Leiden.

9. Verfahren zur Herstellung einer wie in einem der Ansprüche 1 bis 4 definierten Verbindung der Formel (I) und optischen Isomeren, Racematen und Tautomeren davon und pharmazeutisch annehmbaren Salzen davon, bei dem man:
a) eine Verbindung der Formel (II) 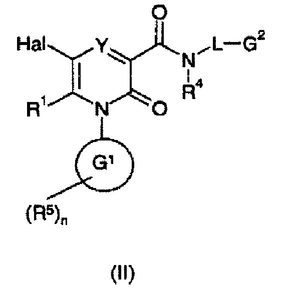 mit einem nucleophilen Äquivalent von R² wie Cu(I)CN, einem Alkylvinylether, einer zinnorganischen Verbindung, einer Organoboronsäure, einem terminalen Alkin oder einem Alkohol und Kohlenmonoxid umsetzt; wobei R¹, R², R⁴, R⁵, Y, G¹, G², L und n wie in Formel (I) definiert sind und Hal für ein Halogenatom, vorzugsweise Brom oder Iod, steht; oder
b) eine Verbindung der Formel (XV) 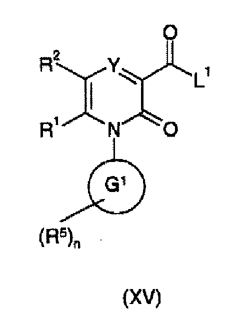
wobei R¹, R², R⁵, n, G¹ und Y wie in Formel (I) definiert sind und L¹ für eine Abgangsgruppe steht,
mit einer Verbindung der Formel (IX) oder einem Salz davon 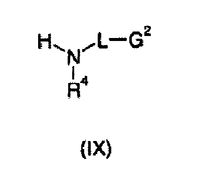 wobei R⁴, G² und L wie in Formel (I) definiert sind, umsetzt;
und, falls gewünscht oder erforderlich, die erhaltene Verbindung der Formel (I) oder ein anderes Salz davon in ein pharmazeutisch annehmbares Salz davon umwandelt; oder eine Verbindung der Formel (I) in eine andere Verbindung der Formel (I) umwandelt; und, falls gewünscht, die erhaltene Verbindung der Formel (I) in ein optisches Isomer davon umwandelt.

## Revendications

1. Composé de formule (I) 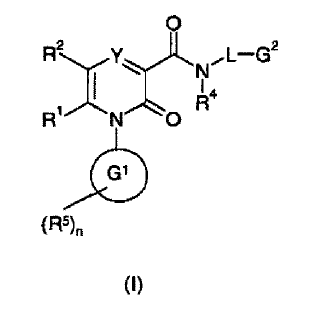 dans laquelle :
**Y** représente CR³ ou N ;
**R¹** représente H ou alkyle en C1 à 6 ;
**R²** représente :
i) CN, NO₂, OH, OSO₂R⁴⁷, O-alcanoyle en C2 à 6, CO₂R⁴⁷, CHO ou alcanoyle en C2 à 6 ;
ou
ii) alcoxy en Cl à 6 éventuellement substitué par OH, alcoxy en Cl à 6, CN, NR⁵⁴R⁵⁵, CONR⁵⁴R⁵⁵, OCOR⁴⁷ ou un ou plusieurs atomes de F ; ou
iii) cycloalkyle en C3 à 6 saturé ou partiellement insaturé éventuellement encore substitué par alkyle en Cl à 6 ; ou
iv) un cycle hétérocyclique en C4 à 7 saturé ou partiellement insaturé contenant de 1 à 3 hétéroatomes choisis indépendamment parmi O, S(O)ₘ et NR⁶² éventuellement encore substitué par alkyle en C1 à 6 ; ou
v) CONR⁴⁸R⁴⁹, CONR⁵⁰NR⁴⁸R⁴⁹, C(=NOR⁵²)R⁵³, C(=NH)NHOR⁵² ou NR⁴⁸R⁴⁹ ;
ou
vi) alcényle en C2 à 6 ou alcynyle en C2 à 6 ; ledit groupement alcényle ou alcynyle étant éventuellement encore substitué par alcoxy en C1 à 6 ou phényle ou un cycle hétéroaromatique à 5 ou 6 chaînons contenant de 1 à 3 hétéroatomes choisis indépendamment parmi O, S et N ; ledit phényle ou cycle hétéro aromatique étant éventuellement encore substitué par halogène, CN, alkyle en Cl à 6 ou alcoxy en C1 à 6 ; ou
vii) alkyle en C1 à 6 substitué par un ou plusieurs atomes de F ; ou
viii) alkyle en C1 à 6 substitué par un ou plusieurs groupements choisis parmi halogène, OH, oxo, azido, NR⁴⁸R⁴⁹, alcoxy en C1 à 6 et alcoxy en C1 à 6 substitué par un ou plusieurs atomes de F ; ou
ix) alkyle en C1 à 6 substitué par phényle ou un cycle hétéroaromatique à 5 ou 6 chaînons contenant de 1 à 3 hétéroatomes choisis indépendamment parmi O, S et N ; ledit phényle ou cycle hétéroaromatique étant éventuellement encore substitué par halogène, CN, alkyle en C1 à 6 ou alcoxy en C1 à 6 ;
**R⁴⁸** et **R⁴⁹** représentent indépendamment H, OH, alcoxy en C1 à 6, cycloalkyle en C3 à 6, CHO, alcanoyle en C2 à 6, CO₂R⁵⁰, C(X)NR⁶³R⁶⁴ ou alkyle en C1 à 6 ; ledit alkyle étant éventuellement encore substitué par OH, alcoxy en C1 à 4, cycloalkyle en C3 à 6, CN ou phényle ou un cycle hétéroaromatique à cinq ou six chaînons contenant de 1 à 3 hétéroatomes choisis indépendamment parmi O, S et N ; ledit alcanoyle étant éventuellement encore substitué par CN ;
**X** représente O ou S ;
ou le groupement **NR⁴⁸R⁴⁹** représente ensemble un cycle azacyclique saturé ou partiellement insaturé à 5 à 7 chaînons comportant éventuellement un autre hétéroatome choisi parmi O, S et NR⁵⁶ ; ledit cycle azacyclique étant éventuellement encore substitué par un ou plusieurs substituants choisis parmi OR⁵⁷ et alkyle en C1 à 4 ; ledit alkyle étant éventuellement encore substitué par OR⁵⁷ ;
**R³** représente H ou F ;
**G¹** représente phényle ou un cycle hétéroaromatique à cinq ou six chaînons contenant de 1 à 3 hétéroatomes choisis indépendamment parmi O, S et N ;
**R⁵** représente H, halogène, alkyle en C1 à 6, CN, alcoxy en C1 à 6, NO₂, NR¹⁴R¹⁵, alkyle en C1 à 3 substitué par un ou plusieurs atomes de F ou alcoxy en C1 à 3 substitué par un ou plusieurs atomes de F ;
**R¹⁴** et **R¹⁵** représentent indépendamment H ou alkyle en C1 à 3 ; ledit alkyle étant éventuellement encore substitué par un ou plusieurs atomes de F ;
**n** représente un entier 1, 2 ou 3 et lorsque n représente 2 ou 3, chaque groupement **R⁵** est choisi indépendamment ;
**R⁴** représente H ou alkyle en C1 à 6, ledit alkyle étant éventuellement encore substitué par OH ou alcoxy en C1 à 6 ;
ou **R⁴** et **L** sont liés ensemble de sorte que le groupement **-NR⁴L** représente un cycle azacyclique à 5 à 7 chaînons comportant éventuellement un autre hétéroatome choisi parmi O, S et NR¹⁶ ; ledit cycle étant éventuellement encore substitué par alkyle en C1 à 6 ou NR⁶⁰R⁶¹ ; ledit alkyle étant éventuellement encore substitué par OH ;
**L** représente une liaison, O, NR²⁹ ou alkyle en C1 à 6 ; ledit alkyle comportant éventuellement un hétéroatome choisi parmi O, S et NR¹⁶ ; et ledit alkyle étant éventuellement encore substitué par OH ou OMe ;
**G²** représente un système de cycle monocyclique choisi parmi :
i) phényle ou phénoxy,
ii) un cycle hétéroaromatique à 5 ou 6 chaînons contenant de un à trois hétéroatomes choisis indépendamment parmi O, S et N,
iii) un cycloalkyle en C3 à 6 saturé ou partiellement insaturé, ou
iv) un cycle hétérocyclique en C4 à 7 saturé ou partiellement insaturé contenant un ou deux hétéroatomes choisis indépendamment parmi O, S(O)ₚ et NR¹⁷ et comportant encore éventuellement un groupement carbonyle ; ou
**G²** représente un système de cycle bicyclique dans lequel chacun des deux cycles est choisi indépendamment parmi :
i) phényle,
ii) un cycle hétéroaromatique à 5 ou 6 chaînons contenant de un à trois hétéroatomes choisis indépendamment parmi O, S et N,
iii) un cycloalkyle en C3 à 6 saturé ou partiellement insaturé, ou
iv) un cycle hétérocyclique en C4 à 7 saturé ou partiellement insaturé contenant un ou deux hétéroatomes choisis indépendamment parmi O, S(O)ₚ et NR¹⁷ et comportant éventuellement encore un groupement carbonyle ;
et les deux cycles sont condensés ensemble, liés directement ensemble ou séparés par un groupement lieur choisi parmi O, S(O)_{q} ou CH₂,
ledit système de cycle monocyclique ou bicyclique étant éventuellement encore substitué par de un à trois substituants choisis indépendamment parmi CN, OH, alkyle en C1 à 6, alcoxy en Cl à 6, halogène, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C(=NH)NH₂, S(O)ₛR²⁵, SO₂NR²⁶R²⁷, alcoxy en C1 à 3 substitué par un ou plusieurs atomes de F et alkyle en Cl à 3 substitué par SO₂R³⁹ ou par un ou plusieurs atomes de F ;
ou
lorsque L ne représente pas une liaison, **G²** peut également représenter H ;
à chaque apparition, **m, p, q, s** et **t** représentent indépendamment un entier 0, 1 ou 2 ;
**R¹⁸** et **R¹⁹** représentent indépendamment H, alkyle en C1 à 6, formyle, alcanoyle en C2 à 6, S(O)ₜR³² ou SO₂NR³³R³⁴ ; ledit groupement alkyle étant éventuellement encore substitué par halogène, CN, alcoxy en C1 à 4 ou CONR⁴¹R⁴² ;
**R²⁵** représente H, alkyle en Cl à 6 ou cycloalkyle en C3 à 6 ; ledit groupement alkyle étant éventuellement encore substitué par un ou plusieurs substituants choisis indépendamment parmi OH, CN, CONR³⁵R³⁶, CO₂R³⁷, OCOR⁴⁰, cycloalkyle en C3 à 6, un cycle hétérocyclique en C4 à 7 saturé contenant un ou deux hétéroatomes choisis indépendamment parmi O, S(O)ₚ et NR⁴³ et phényle ou un cycle hétéroaromatique à 5 ou 6 chaînons contenant de un à trois hétéroatomes choisis indépendamment parmi O, S et N ; ledit cycle aromatique étant éventuellement encore substitué par un ou plusieurs substituants choisis indépendamment parmi halogène, CN, alkyle en C1 à 4, alcoxy en C1 à 4, OH, CONR⁴⁴R⁴⁵, CO₂R⁴⁶, S(O)ₛR⁶⁵ et NHCOCH₃ ;
**R³²** représente H, alkyle en C1 à 6 ou cycloalkyle en C3 à 6 ;
**R¹⁶, R¹⁷, R²⁰, R²¹, R²², R²³, R²⁴, R²⁶, R²⁷, R²⁹, R³¹, R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁵⁰, R⁵², R⁵³, R⁵⁴, R⁵⁵, R⁵⁶, R⁵⁷, R⁶⁰, R⁶¹, R⁶², R⁶³, R⁶⁴ et R⁶⁵** représentent indépendamment H ou alkyle en Cl à 6 ;
et les sels pharmaceutiquement acceptables de celui-ci.

2. Composé de formule (I), selon la revendication 1, dans laquelle Y représente CR³.

3. Composé de formule (I), selon la revendication 1 ou la revendication 2, dans laquelle G¹ représente phényle.

4. Composé de formule (I), selon l'une quelconque des revendications 1 à 3, dans laquelle R⁵ représente Cl, CH₃, CN ou CF₃.

5. Composé de formule (I), selon l'une quelconque des revendications 1 à 4, ou un sel pharmaceutiquement acceptable de celui-ci, destiné à être utilisé comme médicament.

6. Formulation pharmaceutique comprenant un composé de formule (I), telle que définie dans l'une quelconque des revendications 1 à 4, ou un sel pharmaceutiquement acceptable de celui-ci, éventuellement en mélange avec un diluant ou un support pharmaceutiquement acceptable.

7. Utilisation d'un composé de formule (I) telle que définie dans l'une quelconque des revendications 1 à 4, ou d'un sel pharmaceutiquement acceptable de celui-ci, dans la fabrication d'un médicament destiné au traitement ou à la prophylaxie du syndrome de détresse respiratoire chez l'adulte (SDRA), de la mucoviscidose, de l'emphysème pulmonaire, de la bronchopneumopathie chronique obstructive (BPCO), de l'hypertension pulmonaire, de l'asthme, de la rhinite, de la lésion d'ischémie-reperfusion, de la polyarthrite rhumatoïde, de l'ostéoarthrite, du cancer, de l'athérosclérose ou de la lésion de la muqueuse gastrique.

8. Utilisation d'un composé de formule (I) telle que définie dans l'une quelconque des revendications 1 à 4, ou d'un sel pharmaceutiquement acceptable de celui-ci, dans la fabrication d'un médicament destiné au traitement ou à la prophylaxie de maladies ou d'affections inflammatoires.

9. Procédé de préparation d'un composé de formule (I), tel que défini dans l'une quelconque des revendications 1 à 4, et des isomères optiques, des racémates ou des tautomères de celui-ci et des sels pharmaceutiquement acceptables de celui-ci, qui comprend :
a) la mise en réaction d'un composé de formule (II) 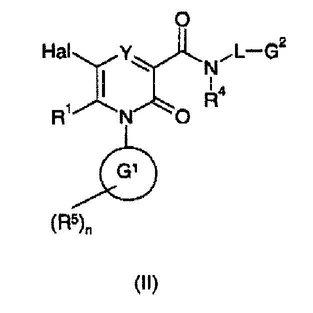 avec un équivalent nucléophile de R², tel que Cu(I)CN, un alkylvinyléther, un composé organostannique, un acide organoboronique, un alcyne terminal ou un alcool et le monoxyde de carbone ;
dans laquelle R¹, R², R⁴, R⁵, Y, G¹, G², L et n sont tels que définis dans la formule (I) et Hal représente un atome d'halogène, de préférence bromo ou iodo ; ou
b) la mise en réaction d'un composé de formule (XV) 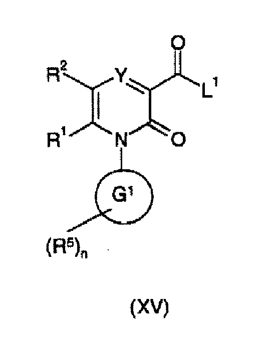
dans laquelle R¹, R², R⁵, n, G¹ et Y sont tels que définis dans la formule (I) et L¹ représente un groupement partant,
avec un composé de formule (IX) ou un sel de celui-ci 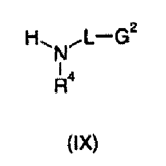 dans laquelle R⁴, G² et L sont tels que définis dans la formule (I) ;
et lorsqu'on le souhaite ou lorsque cela est nécessaire, la transformation du composé résultant de formule (I), ou d'un autre sel de celui-ci, en un sel pharmaceutiquement acceptable de celui-ci ; ou la transformation d'un composé de formule (I) en un autre composé de formule (I) ; et lorsqu'on le souhaite, la transformation du composé résultant de formule (I) en un isomère optique de celui-ci.
